# EUROPEAN PATENT APPLICATION

(11) **EP 4 640 210 A1**
(43) Date of publication of application: **29.10.2025**
(21) Application number: 23907858.7
(22) Date of filing: 22.12.2023
(51) Int. Cl.: A61K 9/08, A61K 47/68, A61K 47/22, A61K 47/12, A61K 47/02, A61K 47/20, A61K 47/26, A61K 47/18, A61K 9/19, C07K 14/55

(54) **FORMULATION CONTAINING IMMUNOACTIVE INTERLEUKIN 2 ANALOG CONJUGATE**

(30) Priority: 23.12.2022 KR 20220182979
(71) Applicant: Hanmi Pharm. Co., Ltd., Hwaseong-si, Gyeonggi-do 18536 (KR)
(72) Inventor: KIM, Sang Yun, Hwaseong-si, Gyeonggi-do 18469 (KR); KIM, Jeong Woo, Hwaseong-si, Gyeonggi-do 18469 (KR); YOO, Nyeong Sang, Hwaseong-si, Gyeonggi-do 18469 (KR); LEE, Jong Min, Hwaseong-si, Gyeonggi-do 18469 (KR); CHOI, Min Young, Hwaseong-si, Gyeonggi-do 18469 (KR); HONG, Sung Hee, Hwaseong-si, Gyeonggi-do 18469 (KR)
(74) Representative: Mewburn Ellis LLP
(86) International application number: PCT/KR2023/021397
(87) International publication number: WO 2024/136575

(57) **Abstract**

The present invention relates to a formulation comprising a long-acting conjugate of an interleukin 2 analog.

## Description

### [Technical Field]

The present invention relates to a formulation comprising an interleukin 2 analog conjugate.

### [Background Art]

Interleukin 2 (IL-2) is an important immunostimulant that is composed of a total of 133 amino acid residues and has a molecular weight of about 15 kDa, which activates various cells of the immune system including T cells and B cells. The high efficacy of interleukin 2 as an immunostimulant can be used to treat various immune-related diseases including cancer and AIDS (Korean Patent Publication No. 10-2017-0070091). Currently, interleukin 2 (brand name Proleukin) is an FDA-approved drug for the treatment of metastatic renal cell carcinoma and metastatic melanoma. However, because of the serious toxicity associated with high-dose interleukin 2 therapy, the applicable patients are limited, and in reality, this therapy is performed only for a small number of suitable patients. Toxicity associated with interleukin 2 includes severe fever, nausea, vomiting, vascular leak, severe hypotension, pulmonary edema, and liver damage.

The interleukin 2 receptor has three subunit receptors. The subunits consist of an alpha chain (IL-2Rα, CD25), a beta chain (IL-2Rβ or CD122), and a gamma chain (IL-2Rγ or CD132), and interleukin 2 can exert various functions by binding to various combinations of receptor subunits. The single interleukin 2 alpha receptor is called the low-affinity interleukin 2 receptor and is not involved in signaling. The complex of interleukin 2 beta and gamma receptors binds to interleukin 2 with intermediate affinity. The complex of interleukin 2 alpha, beta, and gamma receptors binds to interleukin 2 with high affinity. The complex of interleukin 2 beta and gamma receptors is required for effective signal transduction through kinase activation of multiple signaling pathways. In particular, interleukin 2 beta and gamma coupled receptors are prominent on CD8+ cells and natural killer (NK) cells. The high-affinity complex of interleukin 2 alpha, beta, and gamma receptors is commonly found on CD4+ T regulatory cells (Tregs) as well as recently activated T cells. Interleukin 2 beta receptors are distributed on CD8+ T cells or natural killer cells (NK cells) and are involved in the body's immune response. Therefore, studies have been conducted to develop therapeutic agents by increasing the activity of beta receptors for immune activation.

### [Disclosure]

### [Technical Problem]

The present inventors have developed a novel interleukin 2 analog conjugate that can be used as a therapeutic agent for various immune-related diseases while reducing toxicity and side effects (WO2022-211537 A1). It is required to develop a formulation that can maintain the activity even after long-term storage by stabilizing this.

### [Technical Solution]

An object of the present invention is to provide a formulation comprising a long-acting conjugate comprising an interleukin 2 analog.

Another object of the present invention is to provide a liquid formulation comprising the long-acting conjugate.

Still another object of the present invention is to provide a lyophilized formulation comprising the long-acting conjugate.

Still another object of the present invention is to provide a method for preparing the lyophilized formulation.

Still another object of the present invention is to provide a method for reconstructing the lyophilized formulation.

### [Advantageous Effects]

The formulation comprising a long-acting conjugate including an interleukin 2 analog according to the present invention exhibits excellent stability and has the advantage of being economically available.

### [Best Mode for Carrying Out the Invention]

An aspect of the present invention is a formulation comprising a long-acting conjugate including an interleukin 2 analog (or IL-2 analog). The interleukin 2 analog may include a sequence in which one or more amino acids in native interleukin 2 are mutated.

As a specific example, the formulation is a liquid formulation.

As another specific example, the formulation is a lyophilized formulation comprising a mixture obtained by lyophilizing an aqueous solution containing an interleukin 2 analog long-acting conjugate and a buffer.

The formulation according to any one of the preceding specific examples, in which the formulation comprises a long-acting conjugate represented by the following Chemical Formula 1; and a buffer having a pH of 6 to 8:

[Chem. 1] X - Lₐ - F

where, X is an interleukin 2 analog;
L is a polyethylene glycol linker;
a is 0 or a natural number, provided that the respective Ls are independent of each other when a is 2 or more;
F is an immunoglobulin Fc region in a dimeric form; and
   - indicates linkage between X and L and between L and F by a covalent bond,
in which the interleukin 2 analogue includes a sequence in which one or more of amino acids corresponding to positions 1, 12, 18, 19, 20, 22, 32, 35, 38, 42, 43, 45, 48, 49, 61, 68, 69, 74, 76, 80, 81, 82, 84, 85, 86, 87, 88, 89, 91, 92, 94, 95, 96, 125, 126 and 133 in native interleukin 2 are mutated.

The formulation according to any one of the preceding specific examples, in which the formulation comprises the long-acting conjugate represented by Chemical Formula 1 at 10 to 3000 nmol/mL.

The formulation according to any one of the preceding specific examples, in which the buffer contains a buffering substance selected from the group consisting of histidine and its salts, citric acid and its salts, acetic acid and its salts, phosphoric acid and its salts, and a combination thereof.

The formulation according to any one of the preceding specific examples, in which a concentration of the buffering substance is 5 to 100 mM to maintain a pH of the liquid formulation in a range of 6 to 8.

The formulation according to any one of the preceding specific examples, in which the buffer contains 1 to 50 mM histidine.

The formulation according to any one of the preceding specific examples, in which the buffer contains 5 to 25 mM histidine.

The formulation according to any one of the preceding specific examples, in which the formulation additionally comprises an antioxidant.

The formulation according to any one of the preceding specific examples, in which the antioxidant is methionine.

The formulation according to any one of the preceding specific examples, in which the formulation comprises methionine at 0.05 to 0.5 mg/mL.

**The** formulation according to any one of the preceding specific examples, in which the formulation additionally comprises a surfactant, sugar, an amino acid, a tonicity agent, or a combination thereof.

**The** formulation according to any one of the preceding specific examples, in which the surfactant is selected from the group consisting of poloxamer 188, polysorbate 20, polysorbate 40, polysorbate 60, polysorbate 80, sodium lauryl sulfate, and a combination thereof.

**The** formulation according to any one of the preceding specific examples, in which the formulation comprises a surfactant at a concentration of 0.005% to 1.5% *(w*/*v).*

The formulation according to any one of the preceding specific examples, in which the formulation comprises poloxamer 188 at a concentration of 0.005% to 0.1% *(w*/*v).*

The formulation according to any one of the preceding specific examples, in which the sugar is glucose, fructose, galactose, lactose, maltose, sucrose, or a combination thereof.

The formulation according to any one of the preceding specific examples, in which the formulation comprises sugar at a concentration of 1% to 20% *(w*/*v).*

The formulation according to any one of the preceding specific examples, in which the formulation comprises sugar at a concentration of 8% to 20% *(w*/*v).*

The formulation according to any one of the preceding specific examples, in which the formulation comprises sugar at a concentration of 10% to 20% *(w*/*v).*

The formulation according to any one of the preceding specific examples, in which the amino acid is selected from the group consisting of arginine, serine, alanine, glutamic acid, proline and a combination thereof.

The formulation according to any one of the preceding specific examples, in which the formulation comprises an amino acid at a concentration of 0.5% to 5% (w/v).

The formulation according to any one of the preceding specific examples, in which the formulation comprises arginine at 2% to 5% (w/v).

The formulation according to any one of the preceding specific examples, in which the tonicity agent is sodium chloride.

The formulation according to any one of the preceding specific examples, in which the formulation comprises 50 to 100 mM sodium chloride.

The formulation according to any one of the preceding specific examples, in which the formulation comprises 30 to 70 mM sodium chloride.

The formulation according to any one of the preceding specific examples, in which the aqueous solution of the lyophilized formulation additionally contains a surfactant, sugar, an amino acid, an antioxidant, or a combination thereof.

The formulation according to any one of the preceding specific examples, in which the aqueous solution of the lyophilized formulation is an aqueous solution with a pH of 6.5 to 7.5 containing 5 to 25 mM histidine.

The formulation according to any one of the preceding specific examples, in which the aqueous solution of the lyophilized formulation contains a surfactant at a concentration of 0.01% to 0.1% *(w*/*v).*

The formulation according to any one of the preceding specific examples, in which the aqueous solution of the lyophilized formulation contains polysorbate 20 at a concentration of 0.01% to 0.1% *(w*/*v).*

The formulation according to any one of the preceding specific examples, in which the aqueous solution of the lyophilized formulation contains sugar at a concentration of 8% to 20% *(w*/*v).*

The formulation according to any one of the preceding specific examples, in which the aqueous solution of the lyophilized formulation contains an amino acid at a concentration of 0.5% to 5% (w/v).

The formulation according to any one of the preceding specific examples, in which the aqueous solution of the lyophilized formulation contains arginine at 2% to 5% (w/v).

The formulation according to any one of the preceding specific examples, in which the long-acting conjugate includes, as a part of the conjugate, an interleukin 2 analogue having changed interleukin 2 alpha receptor binding affinity and enhanced interleukin 2 beta receptor binding affinity compared to native interleukin 2 or aldesleukin.

The formulation according to any one of the preceding specific examples, in which the long-acting conjugate includes, as a part of the conjugate, an interleukin 2 analogue having one or more amino acids added to an amino acid corresponding to position 133.

The formulation according to any one of the preceding specific examples, in which the long-acting conjugate includes, as a part of the conjugate, an interleukin 2 analogue including a sequence in which amino acid at position 1 is deleted and amino acid at position 125 is substituted with a different amino acid in native interleukin 2.

The formulation according to any one of the preceding specific examples, in which the long-acting conjugate includes, as a part of the conjugate, an interleukin 2 analogue additionally including 1 to 10 amino acid substitutions.

The formulation according to any one of the preceding specific examples, in which the long-acting conjugate includes an interleukin 2 analog in which one or more of amino acids corresponding to positions 18, 19, 20, 22, 38, 42, 43, 45, 61, 68, 69, 74, 80, 81, 84, 85, 86, 88, 89, 91, 92, 94, and 96 of the interleukin 2 analog moiety are additionally substituted with a different amino acid.

The formulation according to any one of the preceding specific examples, in which the long-acting conjugate includes, as a part of the conjugate, an interleukin 2 analogue in which one or more of amino acids corresponding to positions 18, 22, 38, 42, 61, 68, 80, 81, 85, 86, and 92 are additionally substituted with a different amino acid.

The formulation according to any one of the preceding specific examples, in which the long-acting conjugate includes, as a part of the conjugate, any one of the following interleukin 2 analogues:
(a) an interleukin 2 analog in which the amino acid at position 1 is removed and the amino acids at positions 125 and 32 are substituted with different amino acids in native interleukin 2;
(b) an interleukin 2 analog in which the amino acid at position 1 is removed and the amino acids at positions 125 and 35 are substituted with different amino acids in native interleukin 2;
(c) an interleukin 2 analog in which the amino acid at position 1 is removed and the amino acids at positions 125 and 38 are substituted with different amino acids in native interleukin 2;
(d) an interleukin 2 analog in which the amino acid at position 1 is removed and the amino acids at positions 125 and 42 are substituted with different amino acids in native interleukin 2;
(e) an interleukin 2 analog in which the amino acid at position 1 is removed and the amino acids at positions 125 and 43 are substituted with different amino acids in native interleukin 2;
(f) an interleukin 2 analog in which the amino acid at position 1 is removed and the amino acids at positions 125 and 48 are substituted with different amino acids in native interleukin 2;
(g) an interleukin 2 analog in which the amino acid at position 1 is removed and the amino acids at positions 125 and 49 are substituted with different amino acids in native interleukin 2;
(h) an interleukin 2 analog in which the amino acid at position 1 is removed and the amino acids at positions 125 and 76 are substituted with different amino acids in native interleukin 2;
(i) an interleukin 2 analog in which the amino acid at position 1 is removed and the amino acids at positions 125, 38, 42, 81, 92, 94, and 96 are substituted with different amino acids in native interleukin 2;
(j) an interleukin 2 analog in which the amino acid at position 1 is removed and the amino acids at positions 125 and 87 are substituted with different amino acids in native interleukin 2;
(k) an interleukin 2 analog in which the amino acid at position 1 is removed and the amino acids at positions 125, 38, and 42 are substituted with different amino acids in native interleukin 2;
(l) an interleukin 2 analog in which the amino acid at position 1 is removed and the amino acids at positions 125, 38, and 80 are substituted with different amino acids in native interleukin 2;
(m) an interleukin 2 analog in which the amino acid at position 1 is removed and the amino acids at positions 125, 38, and 84 are substituted with different amino acids in native interleukin 2;
(n) an interleukin 2 analog in which the amino acid at position 1 is removed and the amino acids at positions 125, 19, 38, and 42 are substituted with different amino acids in native interleukin 2;
(o) an interleukin 2 analog in which the amino acid at position 1 is removed and the amino acids at positions 125, 12, 38, and 42 are substituted with different amino acids in native interleukin 2;
(p) an interleukin 2 analog in which the amino acid at position 1 is removed and the amino acids at positions 125, 38, 42, and 61 are substituted with different amino acids in native interleukin 2;
(q) an interleukin 2 analog in which the amino acid at position 1 is removed and the amino acids at positions 125, 38, 42, and 84 are substituted with different amino acids in native interleukin 2;
(r) an interleukin 2 analog in which the amino acid at position 1 is removed and the amino acids at positions 125, 38, 42, and 88 are substituted with different amino acids in native interleukin 2;
(s) an interleukin 2 analog in which the amino acid at position 1 is removed and the amino acids at positions 125, 38, 42, and 89 are substituted with different amino acids in native interleukin 2;
(t) an interleukin 2 analog in which the amino acid at position 1 is removed and the amino acids at positions 125, 38, 42, and 91 are substituted with different amino acids in native interleukin 2;
(u) an interleukin 2 analog in which the amino acid at position 1 is removed and the amino acids at positions 125, 38, 42, and 94 are substituted with different amino acids in native interleukin 2;
(v) an interleukin 2 analog in which the amino acid at position 1 is removed and the amino acids at positions 125, 38, 42, and 126 are substituted with different amino acids in native interleukin 2;
(w) an interleukin 2 analog in which the amino acid at position 1 is removed and the amino acids at positions 125, 38, 80, and 84 are substituted with different amino acids in native interleukin 2;
(x) an interleukin 2 analog in which the amino acid at position 1 is removed and the amino acids at positions 125, 38, 94, and 96 are substituted with different amino acids in native interleukin 2;
(y) an interleukin 2 analog in which the amino acid at position 1 is removed and the amino acids at positions 125, 38, 81, and 92 are substituted with different amino acids in native interleukin 2;
(z) an interleukin 2 analog in which the amino acid at position 1 is removed and the amino acids at positions 125, 61, 81, and 92 are substituted with different amino acids in native interleukin 2;
(aa) an interleukin 2 analog in which the amino acid at position 1 is removed and the amino acids at positions 125, 38, 42, 81, and 92 are substituted with different amino acids in native interleukin 2;
(ab) an interleukin 2 analog in which the amino acid at position 1 is removed and the amino acids at positions 125, 38, 80, 81, and 92 are substituted with different amino acids in native interleukin 2;
(ac) an interleukin 2 analog in which the amino acid at position 1 is removed and the amino acids at positions 125, 38, 81, 84, and 92 are substituted with different amino acids in native interleukin 2;
(ad) an interleukin 2 analog in which the amino acid at position 1 is removed and the amino acids at positions 125, 20, 38, 42, 81, and 92 are substituted with different amino acids in native interleukin 2;
(ae) an interleukin 2 analog in which the amino acid at position 1 is removed and the amino acids at positions 125, 38, 42, 80, 81, and 92 are substituted with different amino acids in native interleukin 2;
(af) an interleukin 2 analog in which the amino acid at position 1 is removed and the amino acids at positions 125, 38, 42, 74, 81, and 92 are substituted with different amino acids in native interleukin 2;
(ag) an interleukin 2 analog in which the amino acid at position 1 is removed and the amino acids at positions 125, 38, 42, 81, 84, and 92 are substituted with different amino acids in native interleukin 2;
(ah) an interleukin 2 analog in which the amino acid at position 1 is removed and the amino acids at positions 125, 38, 42, 81, 88, and 92 are substituted with different amino acids in native interleukin 2;
(ai) an interleukin 2 analog in which the amino acid at position 1 is removed and the amino acids at positions 125, 38, 42, 85, 86, and 92 are substituted with different amino acids in native interleukin 2;
(aj) an interleukin 2 analog in which the amino acid at position 1 is removed and the amino acids at positions 125, 38, 80, 81, 85, and 92 are substituted with different amino acids in native interleukin 2;
(ak) an interleukin 2 analog in which the amino acid at position 1 is removed and the amino acids at positions 125, 38, 80, 81, 86, and 92 are substituted with different amino acids in native interleukin 2;
(al) an interleukin 2 analog in which the amino acid at position 1 is removed and the amino acids at positions 125, 80, 81, 85, 86, and 92 are substituted with different amino acids in native interleukin 2;
(am) an interleukin 2 analog in which the amino acid at position 1 is removed and the amino acids at positions 125, 38, 42, 45, 74, 81, and 92 are substituted with different amino acids in native interleukin 2;
(an) an interleukin 2 analog in which the amino acid at position 1 is removed and the amino acids at positions 125, 38, 42, 74, 80, 81, and 92 are substituted with different amino acids in native interleukin 2;
(ao) an interleukin 2 analog in which the amino acid at position 1 is removed and the amino acids at positions 125, 38, 42, 80, 81, 84, and 92 are substituted with different amino acids in native interleukin 2;
(ap) an interleukin 2 analog in which the amino acid at position 1 is removed and the amino acids at positions 125, 38, 42, 45, 80, 85, 86, and 92 are substituted with different amino acids in native interleukin 2;
(aq) an interleukin 2 analog in which the amino acid at position 1 is removed and the amino acids at positions 125, 38, 80, 81, 85, 86, and 92 are substituted with different amino acids in native interleukin 2;
(ar) an interleukin 2 analog in which the amino acid at position 1 is removed and the amino acids at positions 125, 42, 80, 81, 85, 86, and 92 are substituted with different amino acids in native interleukin 2;
(as) an interleukin 2 analog in which the amino acid at position 1 is removed and the amino acids at positions 125, 61, 80, 81, 85, 86, and 92 are substituted with different amino acids in native interleukin 2;
(at) an interleukin 2 analog in which the amino acid at position 1 is removed and the amino acids at positions 125, 69, 80, 81, 85, 86, and 92 are substituted with different amino acids in native interleukin 2;
(au) an interleukin 2 analog in which the amino acid at position 1 is removed and the amino acids at positions 125, 80, 81, 85, 86, 91, and 92 are substituted with different amino acids in native interleukin 2;
(av) an interleukin 2 analog in which the amino acid at position 1 is removed and the amino acids at positions 125, 18, 22, 80, 81, 85, 86, and 92 are substituted with different amino acids in native interleukin 2;
(aw) an interleukin 2 analog in which the amino acid at position 1 is removed and the amino acids at positions 125, 38, 42, 80, 81, 85, 86, and 92 are substituted with different amino acids in native interleukin 2;
(ax) an interleukin 2 analog in which the amino acid at position 1 is removed and the amino acids at positions 125, 38, 42, 45, 74, 80, 81, and 92 are substituted with different amino acids in native interleukin 2;
(ay) an interleukin 2 analog in which the amino acid at position 1 is removed and the amino acids at positions 125, 38, 68, 80, 81, 85, 86, and 92 are substituted with different amino acids in native interleukin 2;
(az) an interleukin 2 analog in which the amino acid at position 1 is removed and the amino acids at positions 125, 69, 74, 80, 81, 85, 86, and 92 are substituted with different amino acids in native interleukin 2;
(ba) an interleukin 2 analog in which the amino acid at position 1 is removed and the amino acids at positions 125, 80, 81, 84, 85, 86, 91, and 92 are substituted with different amino acids in native interleukin 2;
(bb) an interleukin 2 analog in which the amino acid at position 1 is removed and the amino acids at positions 125, 80, 81, 85, 86, 92, 94, and 96 are substituted with different amino acids in native interleukin 2;
(bc) an interleukin 2 analog in which the amino acid at position 1 is removed and the amino acids at positions 125, 18, 19, 22, 80, 81, 85, 86, and 92 are substituted with different amino acids in native interleukin 2;
(bd) an interleukin 2 analog in which the amino acid at position 1 is removed and the amino acids at positions 125, 18, 22, 38, 80, 81, 85, 86, and 92 are substituted with different amino acids in native interleukin 2;
(be) an interleukin 2 analog in which the amino acid at position 1 is removed and the amino acids at positions 125, 18, 22, 61, 80, 81, 85, 86, and 92 are substituted with different amino acids in native interleukin 2;
(bf) an interleukin 2 analog in which the amino acid at position 1 is removed and the amino acids at positions 125, 18, 22, 68, 80, 81, 85, 86, and 92 are substituted with different amino acids in native interleukin 2;
(bg) an interleukin 2 analog in which the amino acid at position 1 is removed and the amino acids at positions 125, 35, 38, 42, 80, 81, 85, 86, and 92 are substituted with different amino acids in native interleukin 2;
(bh) an interleukin 2 analog in which the amino acid at position 1 is removed and the amino acids at positions 125, 38, 42, 45, 80, 81, 85, 86, and 92 are substituted with different amino acids in native interleukin 2;
(bi) an interleukin 2 analog in which the amino acid at position 1 is removed and the amino acids at positions 125, 38, 42, 80, 81, 85, 86, 91, and 92 are substituted with different amino acids in native interleukin 2;
(bj) an interleukin 2 analog in which the amino acid at position 1 is removed and the amino acids at positions 125, 38, 42, 80, 81, 85, 86, 92, and 95 are substituted with different amino acids in native interleukin 2;
(bk) an interleukin 2 analog in which the amino acid at position 1 is removed and the amino acids at positions 125, 35, 38, 42, 74, 80, 81, 85, 86, and 92 are substituted with different amino acids in native interleukin 2;
(bl) an interleukin 2 analog in which the amino acid at position 1 is removed and the amino acids at positions 125, 38, 42, 43, 61, 80, 81, 85, 86, and 92 are substituted with different amino acids in native interleukin 2;
(bm) an interleukin 2 analog in which the amino acid at position 1 is removed and the amino acids at positions 125, 38, 42, 80, 81, 85, 86, 91, 92, and 95 are substituted with different amino acids in native interleukin 2;
(bn) an interleukin 2 analog in which the amino acid at position 1 is removed and the amino acids at positions 125, 35, 38, 42, 74, 80, 81, 82, 85, 86, and 92 are substituted with different amino acids in native interleukin 2;
(bo) an interleukin 2 analog in which the amino acid at position 1 is removed and the amino acids at positions 125, 80, 81, 86, and 92 are substituted with different amino acids in native interleukin 2; and
(bp) an interleukin 2 analog in which the amino acid at position 1 is removed and the amino acids at positions 125, 80, 81, 85, and 86 are substituted with different amino acids in native interleukin 2.

The formulation according to any one of the preceding specific examples, in which the long-acting conjugate includes an interleukin 2 analog including any one or more substitutions selected from the group consisting of the following amino acid substitutions:
(a) a substitution in which the amino acid at position 12 is substituted with valine or phenylalanine;
(b) a substitution in which the amino acid at position 18 is substituted with arginine;
(c) a substitution in which the amino acid at position 19 is substituted with tyrosine, valine, phenylalanine, or arginine;
(d) a substitution in which the amino acid at position 20 is substituted with valine or phenylalanine;
(e) a substitution in which the amino acid at position 22 is substituted with glutamic acid;
(f) a substitution in which the amino acid at position 32 is substituted with cysteine;
(g) a substitution in which the amino acid at position 35 is substituted with cysteine or glutamic acid;
(h) a substitution in which the amino acid at position 38 is substituted with alanine or aspartic acid;
(i) a substitution in which the amino acid at position 42 is substituted with lysine, alanine, or tryptophan;
(j) a substitution in which the amino acid at position 43 is substituted with cysteine, glutamic acid, or glutamine;
(k) a substitution in which the amino acid at position 45 is substituted with alanine;
(l) a substitution in which the amino acid at position 48 is substituted with cysteine;
(m) a substitution in which the amino acid at position 49 is substituted with cysteine;
(n) a substitution in which the amino acid at position 61 is substituted with glutamine, arginine, or aspartic acid;
(o) a substitution in which the amino acid at position 68 is substituted with aspartic acid or glutamine;
(p) a substitution in which the amino acid at position 69 is substituted with glycine;
(q) a substitution in which the amino acid at position 74 is substituted with histidine or alanine;
(r) a substitution in which the amino acid at position 76 is substituted with cysteine;
(s) a substitution in which the amino acid at position 80 is substituted with phenylalanine, tyrosine, valine, aspartic acid, or tryptophan;
(t) a substitution in which the amino acid at position 81 is substituted with aspartic acid, glutamic acid, or asparagine;
(u) a substitution in which the amino acid at position 82 is substituted with glycine or valine;
(v) a substitution in which the amino acid at position 84 is substituted with glutamic acid, valine, or phenylalanine;
(w) a substitution in which the amino acid at position 85 is substituted with valine, alanine, glycine, tryptophan, tyrosine, threonine, isoleucine, glutamic acid, or phenylalanine;
(x) a substitution in which the amino acid at position 86 is substituted with valine, alanine, glycine, or leucine;
(y) a substitution in which the amino acid at position 87 is substituted with cysteine;
(z) a substitution in which the amino acid at position 88 is substituted with glutamine, valine, or phenylalanine;
(aa) a substitution in which the amino acid at position 89 is substituted with phenylalanine;
(ab) a substitution in which the amino acid at position 91 is substituted with threonine, phenylalanine, or glutamic acid;
(ac) a substitution in which the amino acid at position 92 is substituted with phenylalanine, leucine, tyrosine, or tryptophan;
(ad) a substitution in which the amino acid at position 94 is substituted with phenyl alanine or valine;
(ae) a substitution in which the amino acid at position 95 is substituted with aspartic acid;
(af) a substitution in which the amino acid at position 96 is substituted with phenylalanine, valine, or isoleucine;
(ag) a substitution in which the amino acid at position 125 is substituted with serine; and
(ah) a substitution in which the amino acid at position 126 is substituted with threonine.

The formulation according to any one of the preceding specific examples, in which the long-acting conjugate includes any one of the following interleukin 2 analogues:
(a) an interleukin 2 analogue in which amino acid at position 1 is deleted and amino acids at positions 125, 38, 42, 80, 81 and 92 are substituted with different amino acids in native interleukin 2; (b) an interleukin 2 analogue in which amino acid at position 1 is deleted and amino acids at positions 125, 80, 81, 85, 86 and 92 are substituted with different amino acids in native interleukin 2; (c) an interleukin 2 analogue in which amino acid at position 1 is deleted and amino acids at positions 125, 18, 22, 80, 81, 85, 86, and 92 are substituted with different amino acids in native interleukin 2; (d) an interleukin 2 analogue in which amino acid at position 1 is deleted and amino acids at positions 125, 38, 42, 80, 81, 85, 86, and 92 are substituted with different amino acids in native interleukin 2; (e) an interleukin 2 analogue in which amino acid at position 1 is deleted and amino acids at positions 125, 18, 22, 61, 80, 81, 85, 86, and 92 are substituted with different amino acids in native interleukin 2; and (f) an interleukin 2 analogue in which amino acid at position 1 is deleted and amino acids at positions 125, 18, 22, 68, 80, 81, 85, 86, and 92 are substituted with different amino acids in native interleukin 2.

The formulation according to any one of the preceding specific examples, in which the long-acting conjugate includes an interleukin 2 analog including any one or more amino acid substitutions selected from the group consisting of the following amino acid substitutions:
(a) substitution of amino acid at position 18 with arginine; (b) substitution of amino acid at position 22 with glutamic acid; (c) substitution of amino acid at position 38 with alanine; (d) substitution of amino acid at position 42 with lysine; (e) substitution of amino acid at position 61 with aspartic acid; (f) substitution of amino acid at position 68 with aspartic acid; (g) substitution of amino acid at position 80 with phenylalanine; (h) substitution of amino acid at position 81 with glutamic acid; (i) substitution of amino acid at position 85 with valine; (j) substitution of amino acid at position 86 with valine; (k) substitution of amino acid at position 92 with phenylalanine; and (l) substitution of amino acid at position 125 with serine.

The formulation according to any one of the preceding specific examples, in which the interleukin 2 analog of the long-acting conjugate includes any one sequence selected from the group consisting of SEQ ID NOs: 3 to 106.

The formulation according to any one of the preceding specific examples, in which the interleukin 2 analog of the long-acting conjugate includes any one sequence selected from the group consisting of amino acid sequences of SEQ ID NOs: 10, 13, 14, 15, 16, 17, 20, 21, 22, 32, 35, 36, 42, 53, 54, 56, 58, 59, 60, 62, 71, 72, 74, 75, 76, 77, 78, 85, 87, 89, 91, 92, 93, 94, 95, 98, 99, 100, 101, 103, 104, 105, and 106.

The formulation according to any one of the preceding specific examples, in which the interleukin 2 analog of the long-acting conjugate includes any one sequence selected from the group consisting of amino acid sequences of SEQ ID NOs: 10, 13, 14, 16, 17, 20, 21, 22, 32, 35, 36, 42, 53, 54, 87, 89, 91, 92, 93, 94, 98, 99, 100, 101, 103, 104, and 105.

The formulation according to any one of the preceding specific examples, in which the interleukin 2 analog of the long-acting conjugate includes any one sequence selected from the group consisting of amino acid sequences of SEQ ID NOs: 22, 42, 53, 87, 105, and 106.

The formulation according to any one of the preceding specific examples, in which the long-acting conjugate includes an immunoglobulin Fc region that is derived from IgG, IgA, IgD, IgE, or IgM or is a combination thereof or a hybrid thereof.

The formulation according to any one of the preceding specific examples, in which the long-acting conjugate includes an IgG4 Fc region.

The formulation according to any one of the preceding specific examples, in which the long-acting conjugate includes an aglycosylated immunoglobulin Fc region.

The formulation according to any one of the preceding specific examples, in which the immunoglobulin Fc region of the long-acting conjugate is derived from a human IgG4-derived aglycosylated Fc region.

The formulation according to any one of the preceding specific examples, in which the immunoglobulin Fc region of the long-acting conjugate is a structure in which two polypeptide chains are linked by a disulfide bond and is linked only via a nitrogen atom of one of the two chains.

The formulation according to any one of the preceding specific examples, in which the immunoglobulin Fc region of the long-acting conjugate includes a monomer having an amino acid sequence of SEQ ID NO: 129.

The formulation according to any one of the preceding specific examples, in which the immunoglobulin Fc region of the long-acting conjugate is a homodimer of monomers having an amino acid sequence of SEQ ID NO: 129.

The formulation according to any one of the preceding specific examples, in which the immunoglobulin Fc region of the long-acting conjugate is linked via a nitrogen atom of its N-terminal proline.

The formulation according to any one of the preceding specific examples, in which L of the long-acting conjugate contains an ethylene glycol repeating unit, and a formula weight of the ethylene glycol repeating unit moiety is in a range of 1 to 100 kDa.

The formulation according to any one of the preceding specific examples, in which in the long-acting conjugate, one molecule of X is covalently linked to one Fc region in the immunoglobulin Fc region in a dimeric form via the polyethylene glycol linker.

The formulation according to any one of the preceding specific examples, in which in the long-acting conjugate, the ethylene glycol repeating unit is [OCH₂CH₂]n, where n is a natural number and is determined so that an average molecular weight of the [OCH₂CH₂]n moiety in the interleukin 2 analog conjugate, for example, the number average molecular weight, is 1 to 100 kDa.

The formulation according to any one of the preceding specific examples, in which in the long-acting conjugate, the value of n is determined so that an average molecular weight of the [OCH₂CH₂]n moiety in the interleukin 2 analog conjugate, for example, the number average molecular weight, is 3.4 kDa.

The formulation according to any one of the preceding specific examples, in which in the long-acting conjugate, the a is 1, and one molecule of X is covalently linked to one Fc region chain of the dimeric immunoglobulin Fc region in a dimeric form via a linker containing the ethylene glycol repeating unit.

The formulation according to any one of the preceding specific examples, in which in the long-acting conjugate, one end of the linker is linked to only one of the two Fc region chains of the immunoglobulin Fc region in a dimeric form.

The formulation according to any one of the preceding specific examples, in which in the long-acting conjugate, one end of L is linked to F by a covalent bond formed through a reaction with an amine group or thiol group of F and the other end of L is linked to X by a covalent bond formed through a reaction with an amine group or thiol group of X, respectively.

Another aspect implementing the present invention is a method for preparing a formulation comprising the interleukin 2 analog conjugate.

Still another aspect implementing the present invention is a method for reconstructing a lyophilized formulation comprising the interleukin 2 analog conjugate.

### [Detailed Description of the Invention]

The specific details for carrying out the present invention are as follows. Meanwhile, each description and each embodiment disclosed in this application can also be applied to other descriptions and other embodiments, respectively. In other words, all combinations of the various elements disclosed in this application fall within the scope of the present invention. Additionally, the scope of the present invention is not limited by the specific description described below. Additionally, a number of papers and patent documents are referenced and cited throughout this specification. The disclosures of the cited papers and patent documents are incorporated herein by reference in their entirety to more clearly explain the level of the technical field to which the present invention belongs and the content of the present invention.

Throughout this specification, conventional one-letter and three-letter codes for amino acids are used. Additionally, amino acids referred to as abbreviations in this specification are described according to the IUPAC-IUB nomenclature.

| | |
|---|---|
| Alanine A | Arginine R |
| Asparagine N | Aspartic acid D |
| Cysteine C | Glutamic acid E |
| Glutamine Q | Glycine G |
| Histidine H | Isoleucine I |
| Leucine L | Lysine K |
| Methionine M | Phenylalanine F |
| Proline P | Serine S |
| Threonine T | Tryptophan W |
| Tyrosine Y | Valine V |

In this specification, "Aib" may be used interchangeably with "2-aminoisobutyric acid" or "aminoisobutyric acid", and 2-aminoisobutyric acid and aminoisobutyric acid may be used interchangeably.

An aspect implementing the present invention provides a formulation comprising a long-acting conjugate including an interleukin 2 analog.

Specifically, the present invention relates to a formulation comprising a long-acting conjugate including an interleukin 2 analog at a pharmacologically effective amount and a buffer. The formulation of the present invention may additionally comprise, but is not limited to, an antioxidant, a surfactant, sugar, an amino acid, a tonicity agent, a preservative, or a combination thereof. **The** formulation of the present invention may be, but is not limited to, a liquid formulation or a lyophilized formulation.

A specific aspect of the present invention provides a liquid formulation comprising a long-acting conjugate including an interleukin 2 analog.

**In** the present invention, the term "liquid formulation" means a liquid drug formulated in the form of a pharmaceutical product, and includes both oral liquids and topical liquids.

**The** liquid formulation of the present invention comprises a substance capable of stably maintaining and/or storing an interleukin 2 analog exhibiting a pharmacological effect for a certain period of time when the interleukin 2 analog is formulated into a liquid form.

**In** the liquid formulation of the long-acting conjugate of the present invention, storage stability is important to ensure an accurate dosage, and it has been verified that the liquid formulation of the present invention is stable even when the long-acting conjugate is stored for a long period of time, whereby a new formulation of the present invention is provided.

**The** concentration of the long-acting conjugate contained in the liquid formulation of the present invention may be about 10 to about 3000 nmol/mL, about 50 to about 3000 nmol/mL, about 70 to about 2800 nmol/mL, about 70 to about 2600 nmol/mL, about 100 to about 2000 nmol/mL, about 300 to about 1500 nmol/mL, about 300 to about 800 nmol/mL, about 300 to about 600 nmol/mL, about 76.7 to about 2515.8 nmol/mL, or about 306.8 nmol/mL to about 536.9 nmol/mL, but is not limited thereto. Specifically, the concentration of the long-acting conjugate contained in the liquid formulation of the present invention may be about 18.4 nmol/mL, 153.4 nmol/mL, 306.8 nmol/mL, 383.5 nmol/mL, 536.9 nmol/mL, 581.4 nmol/mL, 584.5 nmol/mL, 630.5 nmol/mL, 687.2 nmol/mL, 696.4 nmol/mL, 716.4 nmol/mL, 822.2 nmol/mL, 900.5 nmol/mL, 946.5 nmol/mL, 966.4 nmol/mL, or 1073.8 nmol/mL, but is not limited thereto.

In the present invention, the term "about" is a range that includes ±0.5, ±0.4, ±0.3, ±0.2, ±0.1, ±0.01, and the like, and includes all numerical values equal to or similar to the numerical value following the term about, but is not limited thereto.

The liquid formulation of the present invention may comprise a stabilizer to stabilize the interleukin 2 analog exhibiting a pharmacological effect, and an ingredient contained in addition to the long-acting conjugate of the liquid formulation may be used interchangeably with the stabilizer. In the present invention, the term "stabilizer" refers to a substance that maintains ingredients such as the effective ingredient in the formulation stable for a certain period of time.

It is preferable that the stabilizer of the present invention does not contain albumin. Human serum albumin, which can be used as a protein stabilizer, is manufactured from human blood and therefore has the potential for contamination by pathogenic viruses of human origin, and gelatin and bovine serum albumin may cause illness or allergic reactions in some patients. The albumin-free stabilizer of the present invention does not contain any foreign proteins such as serum albumin or purified gelatin derived from humans or animals, so there is little concern about viral infection.

**The** buffer, which is a component contained in the liquid formulation of the present invention, is a solution for maintaining the **pH** of the liquid formulation so that the long-acting conjugate is stable. As the buffer of the present invention, any buffer can be used without limitation in kind as long as it can maintain the **pH at** which the long-acting conjugate, which is the desired stabilization target substance, can be stabilized.

**The** buffer may contain a buffering substance, which may also be referred to as a buffer system, and examples thereof include **pH** buffering substances, including phosphoric acid and its conjugate alkali salts (for example, phosphates: sodium phosphate, potassium phosphate or their hydrogen or dihydrogen salts), citric acid and its salts (for example, sodium citrate), acetic acid and its salts (for example, sodium acetate), and histidine and its salts, and mixtures of these buffering substances may also be contained in the buffer, but the buffer is not limited thereto.

The liquid formulation of the present invention may comprise a buffer containing the buffering substance as a solvent of the liquid formulation, specifically, the buffer may be selected from the group consisting of a citric acid buffer (for example, a sodium citrate buffer), an acetic acid buffer (for example, a sodium acetate buffer), a phosphate buffer (for example, a sodium phosphate buffer), a histidine buffer, and a combination thereof, and the buffering substance (citric acid and its salts, acetic acid and its salts, histidine and its salts, phosphoric acid and its salts, or a combination thereof) in the buffer or liquid formulation may be contained at a concentration sufficient to maintain the desired pH of the liquid formulation.

Meanwhile, in the preparation of the formulation, the ingredients may be dissolved in water (for example WFI) and the pH of the buffer or formulation may be adjusted to the desired pH using HCl] and/or NaOH, and the like, and this is a method already commonly used in the art. Therefore, even if there is no separate mention of a pH regulator in the claims, it will be understood by those skilled in the art that the formulation can have an adjusted pH by such a method.

The pH of the liquid formulation may be, but is not particularly limited to, about 6 to 8, about 6 to 7, about 6.5 to 7.5, or about 6.5, 6.6, 6.7, 6.8, 6.9, 7.0, 7.0, 7.1, 7.2, 7.3, 7.4. or 7.5.

The concentration of the buffering substance that enables the desired pH to be achieved may be, but is not particularly limited to, about 1 mM to about 200 mM, about 1 mM to about 100 mM, about 1 mM to about 50 mM, about 5 mM to about 100 mM, about 5 mM to about 80 mM, about 5 mM to about 30 mM, or about 5 mM to about 25 mM.

As a specific example, the buffer may contain 5 to 25 mM histidine and have a pH of about 6.5 to 7.5, but is not limited thereto.

The liquid formulation of the present invention may additionally comprise an antioxidant, and a specific example of the antioxidant may be methionine, but is not limited thereto.

The antioxidant may be contained in the liquid formulation of the present invention at about 0.01 to about 1 mg/mL, about 0.05 to about 1 mg/mL, about 0.05 to about 1 mg/mL, about 0.05 to about 0.5 mg/mL, about 0.05 to about 0.2 mg/mL, or about 0.1 to about 0.2 mg/mL, but is not limited thereto.

The liquid formulation according to the present invention may additionally comprise a surfactant. The surfactant can lower the surface tension of a protein solution, and prevent proteins from adsorbing or aggregating on hydrophobic surfaces.

Specific examples of the surfactant that can be used in the present invention include polysorbates (for example polysorbate 20 (polyoxyethylene (20) sorbitan monolaurate), polysorbate 40 (polyoxyethylene (20) sorbitan monopalmitate), polysorbate 60 (polyoxyethylene (20) sorbitan monostearate), polysorbate 80 (polyoxyethylene (20) sorbitan monooleate); the number (20) behind the polyoxyethylene means the total number of oxyethylene groups (-(CH₂CH₂O)-), poloxamer (PEO-PPO-PEO copolymer; PEO: poly(ethylene oxide), PPO: poly(propylene oxide)), polyethylene-polypropylene glycol, polyoxyethylene compounds (for example, polyoxyethylene-stearate, polyoxyethylene alkyl ether (alkyl: C1-C30), polyoxyethylene monoallyl ether, alkylphenyl polyoxyethylene copolymer (alkyl: C1-C30), and the like), sodium dodecyl sulphate (SDS), and examples thereof include polysorbate, poloxamer, or sodium lauryl sulfate, and these may also be used singly or in the combined form of two or more thereof.

Specifically, the surfactant may be poloxamer 188, polysorbate 20, polysorbate 40, polysorbate 60, polysorbate 80, or sodium lauryl sulfate, and these may be used in combination, but the surfactant is not particularly limited thereto.

In the present invention, the surfactant may be contained in the liquid formulation of the present invention at about 0.001 % to about 2% (w/v), about 0.001 % to about 1.5% (w/v), about 0.005% to about 1% (w/v), about 0.005% to about 0.02% (w/v), about 0.005% to about 0.1% (w/v), about 0.01% to about 0.8% (w/v), about 0.015% to about 0.5% (w/v), or about 0.015% to about 0.2% (w/v), but is not particularly limited thereto.

A specific example of the liquid formulation according to the present invention may comprise, but is not limited to, poloxamer 188, specifically poloxamer 188 at about 0.005% to about 0.1% (w/v).

The liquid formulation according to the present invention may additionally comprise sugar. The sugar refers to monosaccharides, disaccharides, polysaccharides, oligosaccharides, and the like, and can enhance the stability of a long-acting conjugate in the liquid formulation. Specific examples thereof include mannose, glucose, fructose, galactose, fucose, lactose, maltose, sucrose, trehalose, raffinose, dextran, or a combination thereof. As a specific example, the sugar may be, but is not limited to, glucose, fructose, galactose, lactose, maltose, sucrose, trehalose, or a combination thereof. For example, the sugar may be, but is not particularly limited to, sucrose.

The sugar may be present at a concentration of about 0.5% to 20% (w/v), about 1% to 20% (w/v), about 1% to 17% (w/v), about 3% to 17% (w/v), about 3% to 12% (w/v), about 3% to 10% (w/v), about 5% to 12% (w/v), about 5% to 10% (w/v), about 8% to 20% (w/v), about 8% to 17% (w/v), about 8% to 14% (w/v), about 8% to 12% (w/v), about 8% to 10% (w/v), about 10% to 20% (w/v), about 10% to 17% (w/v), about 10% to 14% (w/v), about 10% to 12% (w/v), or about 0.0% (w/v), about 4.0% (w/v), about 5.0% (w/v), about 8.0% (w/v), about 9.0% (w/v), about 10.0% (w/v), about 12.0% (w/v), about 14.0% (w/v), or about 17.0% (w/v) with respect to the entire solution of the liquid formulation, but is not particularly limited thereto.

A specific example of the liquid formulation according to the present invention may be, but is not limited to, a liquid formulation comprising sugar at 8% (w/v) or more, specifically sugar at 10% (w/v) or more, more specifically sucrose at 8% to 14% (w/v) or 10% to 14% (w/v).

As another kind of stabilizer contained in the liquid formulation of the present invention, an amino acid may be contained. The amino acid can suppress the production of impurities that may occur by oxidation reactions of proteins, but is not particularly limited thereto. The amino acid may be, but is not limited to, arginine, serine, alanine, glutamic acid, proline, or a combination thereof.

The amino acid may be present in the liquid formulation of the present invention at about 0.0% to 5.0% (w/v), about 0.5% to 5.0% (w/v), about 1.0% to 4.0% (w/v), about 1.3% to 4.0% (w/v), or about 2.0% to 5.0% (w/v), about 2.0% to 4.0% (w/v), about 2.5% to 5% (w/v), about 3.0% to 5% (w/v), or about 0.7% (w/v), about 1.0% (w/v), about 1.3% (w/v), about 1.4% (w/v), about 2.0% (w/v), about 3.0% (w/v), about 4.0% (w/v), or about 5.0% (w/v), but is not particularly limited thereto.

A specific example of the liquid formulation according to the present invention may be a liquid formulation comprising arginine at about 2% (w/v) or more, or about 3% (w/v) or more, specifically about 2% to 4% (w/v), about 3% to 4% (w/v), about 3% (w/v), or about 4.0% (w/v), but is not limited thereto.

The liquid formulation according to the present invention may additionally comprise a tonicity agent. The tonicity agent is a substance that is added to adjust the osmotic pressure of the liquid formulation, and any tonicity agent may be contained in the liquid formulation of the present invention without limitation as long as it can contribute to stabilization of the long-acting conjugate while maintaining an appropriate osmotic pressure.

The tonicity agent may include a water-soluble inorganic salt, and specifically, the liquid formulation of the present invention may comprise sodium chloride as a tonicity agent, but is not limited thereto.

The concentration of sodium chloride used in the present invention may be about 0 to 300 mM, specifically, about 0.1 mM to 300 mM, about 5 mM to 300 mM, about 5 mM to 200 mM, about 5 mM to 150 mM, about 5 mM to 100 mM, about 10 mM to 200 mM, about 10 mM to 150 mM, about 10 mM to 100 mM, about 30 mM to 200 mM, about 30 mM to 150 mM, about 30 mM to 100 mM, about 30 mM to 70 mM, or about 50 mM to 100 mM, but is not limited thereto. The ingredients contained in the formulation may be adjusted to appropriate contents so that the solution formulation comprising a mixture of the respective ingredients becomes an isotonic solution, depending on the kinds and amounts of the ingredients.

A specific example of the liquid formulation according to the present invention may be, but is not limited to, a liquid formulation comprising 30 mM or more and 100 mM or less sodium chloride, or 30 mM to 100 mM sodium chloride.

The liquid formulation according to the present invention may additionally comprise a preservative. The preservative is a substance that substantially diminishes the action of bacteria and fungi in the formulation, and is a compound contained in the formulation to facilitate the production of a formulation for multiple doses. Examples of potential preservatives include octadecyldimethyl-benzyl ammonium chloride, hexamethonium chloride, benzalkonium chloride (a mixture of alkylbenzyldimethylammonium chlorides in which the alkyl group is a long-chain compound), and benzethonium chloride. Other types of preservatives include, but are not limited to, aromatic alcohols, for example, phenol, butyl, and benzyl alcohol; alkyl parabens, for example, methyl or propyl paraben; catechol, resorcinol, cyclohexanol, 3-pentanol, m-cresol, and the like. The concentration of the preservative may be, but is not limited to, 0.001% to 1.0% (w/v).

Meanwhile, in addition to the buffer, tonicity agent, amino acid, surfactant, preservative and antioxidant described above, the liquid formulation of the present invention may optionally further comprise other ingredients or substances known in the art within a range in which the effects of the present invention are not impaired, but is not limited thereto.

The liquid formulation according to the present invention may additionally comprise pharmaceutically acceptable carriers, excipients, and the like, and such carriers and excipients may be non-naturally occurring.

Here, it is clear that the contents described above or below are all applicable to the kind and concentration of each component constituting the stabilizer or the pH.

A specific example of the liquid formulation according to the present invention may be a liquid formulation comprising a long-acting conjugate represented by Chemical Formula 1; and one or more stabilizers selected from the group consisting of histidine and a salt thereof in an amount in which the pH of the liquid formulation becomes 6 to 8; 8% to 20% of sugar; 1% to 5% of amino acid; and 0.001% to 1.5% of surfactant.

Another specific example of the liquid formulation according to the present invention may be a liquid formulation comprising a long-acting conjugate represented by Chemical Formula 1; methionine; histidine and a salt thereof in an amount in which the pH of the liquid formulation becomes 6 to 7; 10% to 20% (w/v) of sucrose; 2% to 5% (w/v) of arginine; and 0.001% to 0.1% (w/v) of poloxamer 188.

Still another specific example of the liquid formulation according to the present invention may be a liquid formulation comprising a long-acting conjugate represented by Chemical Formula 1; methionine; 5 to 25 mM histidine and a salt thereof in an amount in which the pH of the liquid formulation becomes 6 to 7; 10% to 20% (w/v) of sucrose; 2% to 5% (w/v) of arginine; and 0.001% to 0.1% (w/v) of poloxamer 188.

Still another specific example of the liquid formulation according to the present invention may be a liquid formulation comprising a long-acting conjugate represented by Chemical Formula 1; methionine; 5 to 25 mM histidine and a salt thereof in an amount in which the pH of the liquid formulation becomes 6 to 7; 10% to 14% (w/v) of sucrose; 2% to 4% (w/v) of arginine; and 0.001% to 0.02% (w/v) of poloxamer 188.

Another aspect implementing the present invention provides a lyophilized formulation comprising a long-acting conjugate including an interleukin 2 analog.

Specifically, the lyophilized formulation relates to a lyophilized formulation comprising a mixture obtained by lyophilizing an aqueous solution containing a long-acting conjugate including an interleukin 2 analog in a pharmacologically effective amount and a buffer. **The** aqueous solution may additionally contain, but is not limited to, sugar, a surfactant, an amino acid, a tonicity agent, a preservative, an antioxidant, or a combination thereof.

In the present invention, the term "lyophilized formulation" refers to a drug formulated in the form of a pharmaceutical product by lyophilization. Specifically, the long-acting conjugate may be lyophilized together with a substance such as an excipient for stabilizing the long-acting conjugate and may exist in a solid state. Other than the long-acting conjugate that exhibits the pharmacological effect of the lyophilized formulation, ingredients contained in the lyophilized formulation correspond to stabilizers, and any stabilizer may be contained without limitation as long as it can stabilize the long-acting conjugate in a lyophilized state.

In the present invention, the lyophilized formulation is a concept including a lyophilized substance. The lyophilized formulation is prepared through a lyophilization and drying process from the form of a pre-formulation containing both a pre-lyophilized formulation containing a stabilizer for stabilizing a long-acting conjugate and the long-acting conjugate. In the present invention, the lyophilized formulation of the long-acting conjugate may comprise the long-acting conjugate in a therapeutically effective amount, and the therapeutically effective amount of the long-acting conjugate may be contained in a single-use container or a multi-use container, but is not limited thereto.

The lyophilized formulation of the present invention has a composition capable of stabilizing the long-acting conjugate during the lyophilization process, and the stability of the formulation may be maintained also in a case of reconstruction after storage.

The lyophilized formulation comprising a long-acting conjugate according to the present invention can be stored in a container and reconstructed when administration to a subject is required.

In the present invention, the term "reconstruction" means liquefying the lyophilized substance in a solid state so that the long-acting conjugate can be administered. At this time, the concentration of the long-acting conjugate contained in the lyophilized formulation of the present invention may be about 10 to about 3000 nmol/mL, about 50 to about 3000 nmol/mL, about 70 to about 2800 nmol/mL, about 70 to about 2600 nmol/mL, about 100 to about 2000 nmol/mL, or about 300 to about 700 nmol/mL at the time of reconstruction, but is not limited thereto. The concentration of the pre-formulation during the lyophilization process may differ from the concentration after reconstruction.

The lyophilized formulation of the present invention includes the composition of a stabilizer capable of stabilizing the structure of the conjugate so that the pharmacological effect of the long-acting conjugate can be maintained for a long time even when stored for a long period of time. Such a lyophilized formulation of the present invention comprises a mixture obtained by lyophilizing an aqueous solution containing a long-acting conjugate and a buffer.

In the present invention, the term "aqueous solution" means a substance that contains a long-acting conjugate and allows the long-acting conjugate to be stably stored and maintain stability during the lyophilization process and reconstruction. In particular, the aqueous solution contains an excipient that stabilizes the long-acting conjugate and imparts stability to the long-acting conjugate during the lyophilization process and enables the preparation of a lyophilized formulation exhibiting storage stability. The aqueous solution may additionally contain a buffer, a surfactant, sugar, an amino acid, a preservative, an antioxidant, or a combination thereof. The aqueous solution may be used interchangeably with the "pre-formulation" in the present invention.

Since the concentration of the long-acting conjugate may be controlled by adjusting the volume of the reconstructing solution added to the lyophilized formulation, the concentration of the long-acting conjugate in the aqueous solution is not particularly limited.

For example, the aqueous solution of the present invention may contain the long-acting conjugate at a concentration of about 1 to 90 mg/mL, about 1 to 70 mg/mL, about 1 to 50 mg/mL, about 5 to 50 mg/mL, about 5 to 90 mg/mL, about 10 to 50 mg/mL, or about 10 to 30 mg/mL, but is not limited thereto. The reconstructed formulation may comprise, but is not limited to, the long-acting conjugate at about 300 to about 700 nmol/mL.

The pH of the lyophilized formulation of the present invention, the buffer, buffering substance, amino acid, surfactant, tonicity agent, sugar, preservative, and antioxidant contained in the lyophilized formulation are as described above.

As an example, an aqueous solution for preparing the lyophilized formulation of the present invention may
have a pH of about 6 to 8;
contain an antioxidant at 0.05 to 0.5 mg/mL;
contain sugar at 8% to 20% (w/v);
contain a surfactant at 0.001% to 1.5% (w/v); and
contain an amino acid at 1% to 5% (w/v), but is not limited thereto.

As another example, an aqueous solution for preparing the lyophilized formulation of the present invention may
have a pH of about 6 to 7;
contain methionine at 0.05 to 0.5 mg/mL;
contain sucrose at 8% to 14% (w/v);
contain polysorbate 20 at 0.001% to 0.1% (w/v); and
contain arginine at 2% to 5% (w/v), but is not limited thereto.

As still another example, an aqueous solution for preparing the lyophilized formulation of the present invention may
have a pH of about 6 to 7 and contain 5 to 25 mM histidine or a salt thereof;
contain methionine at 0.05 to 0.5 mg/mL;
contain sucrose at 8% to 12% (w/v);
contain polysorbate 20 at 0.01% to 0.1% (w/v); and
arginine at 2% to 4% (w/v), but is not limited thereto.

Meanwhile, in addition to the buffer, amino acid, surfactant, tonicity agent, sugar, preservative, and antioxidant described above, the aqueous solution (pre-formulation) for preparing the lyophilized formulation of the present invention may optionally further contain other ingredients or substances known in the art within a range in which the effects of the present invention are not impaired, but is not limited thereto.

The aqueous solution for preparing the lyophilized formulation or the lyophilized formulation according to the present invention may additionally contain pharmaceutically acceptable carriers, excipients, and the like, and such carriers and excipients may be non-naturally occurring.

The aqueous solution for preparing the lyophilized formulation of the present invention can be frozen and dried under appropriate freezing and drying conditions known in the art. The drying may be completed in a single process, or in multiple processes of two or more processes.

The lyophilized formulation of the present invention may be obtained by preparing a pre-formulation by containing a long-acting conjugate at an appropriate concentration in consideration of the intended dosage, lyophilizing the pre-formulation, and reconstructing the pre-formulation so as to be suitable for administration to a subject. The pre-formulation may be diluted to increase the volume and lyophilized, and at the time of reconstruction, may be diluted with the reconstructing solution in a smaller volume than the volume at the time of lyophilization, but is not limited thereto. In the present invention, the lyophilized formulation may mean a mixture obtained by lyophilizing an aqueous solution containing a long-acting conjugate and a stabilizer (buffer, amino acid, and the like), and may further mean a formulation in which the lyophilized formulation is reconstructed with a reconstructing solution, but is not limited thereto.

Meanwhile, the long-acting conjugate including an interleukin 2 analog, which is an effective ingredient contained in the formulation of the present invention, will be described in more detail below. With regard to the interleukin 2 analogue and a conjugate thereof of the present invention, KR 10-2022-0136285 A or WO2022-211537 A1 is incorporated herein by reference.

The formulation of the present invention may comprise a long-acting conjugate including an interleukin 2 analog as an active ingredient.

The long-acting conjugate according to the present invention may be one in which an immunoglobulin Fc region for increasing the half-life of the interleukin 2 analog and an interleukin 2 analog are linked, and may be represented by the following Chemical Formula 1:

[Chemical Formula 1] X - Lₐ - F

where, X is an interleukin 2 analog;
L is a polyethylene glycol linker;
a is 0 or a natural number, provided that the respective Ls are independent of each other when a is 2 or more;
F is an immunoglobulin Fc region in a dimeric form; and
   - indicates linkage between X and L and between L and F by a covalent bond.

More specifically, in Chemical Formula 1, X and L and L and F may be linked to each other by a covalent bond, and at this time, the conjugate may be a conjugate in which X, L, and F are linked to each other through a covalent bond in the order of Chemical Formula 1.

The F may be directly linked to X (that is, a is 0 in Chemical Formula 1) or may be linked via a linker (L).

In the present invention, the long-acting conjugate can exhibit increased duration of efficacy compared to an interleukin 2 analog that is not bound to an immunoglobulin Fc region. In the present invention, such a conjugate is referred to as a "long-acting conjugate" or "conjugate".

Meanwhile, such a conjugate may be non-naturally occurring.

The interleukin 2 analog of the long-acting conjugate of the present invention has a changed binding affinity to the interleukin 2 receptor, particularly enhanced binding affinity to the interleukin 2 beta receptor when the interleukin 2 analog exists alone without forming a part of the conjugate. Specifically, the interleukin 2 analog of the present invention may have enhanced binding affinity to the interleukin 2 beta receptor compared to native interleukin 2 or known aldesleukin when the interleukin 2 analog exists alone without forming a part of the conjugate, more specifically may have changed (increased or decreased) binding affinity to the interleukin 2 alpha receptor, and may include a sequence in which one or more amino acids in native interleukin 2 are mutated.

In the present invention, the term "interleukin 2 (IL-2)" refers to an immunomodulator, a type of cytokine that transmits signals in the immune system in a living body. Interleukin 2 is generally known as an important immunostimulant of about 15 kDa.

In the present invention, the term "interleukin 2 analog" means one in which one or more amino acids in the native sequence are mutated, and in the present invention, may particularly refer to an interleukin 2 analog in which an amino acid in the native interleukin 2 is mutated so that the binding affinity to the interleukin 2 receptor is decreased or increased compared to the native interleukin 2. Specifically, the interleukin 2 analog of the present invention may be non-naturally occurring.

The native interleukin 2 may be human interleukin 2, the sequence of which can be obtained from a known database and the like. Specifically, the native interleukin 2 may be the amino acid sequence of SEQ ID NO: 1, but is not limited thereto.

In the present invention, the meaning that the native interleukin 2 may be the amino acid sequence of SEQ ID NO: 1 means that not only the sequence identical to SEQ ID NO: 1, but also the sequence having 80%, 85%, 90%, 91%, 92%, 93%, 94%, 95%, 96%, 97%, 98%, or 99% or more homology with SEQ ID NO: 1 belongs to the category of native interleukin 2 of the present invention. The mutation position of an amino acid means that the corresponding position in the amino acid sequence of SEQ ID NO: 1 is mutated when the sequences having 80%, 85%, 90%, 91%, 92%, 93%, 94%, 95%, 96%, 97%, 98%, or 99% or more homology with SEQ ID NO: 1 are aligned.

Specifically, the interleukin 2 analog of the present invention may include a sequence in which one or more of the amino acids corresponding to positions 1, 12, 18, 19, 20, 22, 32, 35, 38, 42, 43, 45, 48, 49, 61, 68, 69, 74, 76, 80, 81, 82, 84, 85, 86, 87, 88, 89, 91, 92, 94, 95, 96, 125, 126, and 133 in native interleukin 2 are mutated. Specifically, the interleukin 2 analog of the present invention is one in which amino acid at position 1 is deleted and amino acid at position 125 is substituted with a different amino acid in native interleukin 2, and may additionally include 1, 2, 3, 4, 5, 6, 7, 8, 9, 10 or more amino acid substitutions. Although not limited thereto, cysteine, the amino acid at position 125, may be substituted with serine, and the amino acids that undergo additional substitutions may be amino acids corresponding to positions 12, 18, 19, 20, 22, 32, 35, 38, 42, 43, 45, 48, 49, 61, 68, 69, 74, 76, 80, 81, 82, 84, 85, 86, 87, 88, 89, 91, 92, 94, 95, 96, 126 and 133, but are not limited thereto.

In the present invention, the term "aldesleukin" or "interleukin 2 analog (aldesleukin)" refers to a commercially available interleukin 2 analog, may be aldesleukin (trade name: Proleukin^{®}), and specifically, may have an amino acid sequence of SEQ ID NO: 2. The term "aldesleukin" is used interchangeably with "interleukin 2 analog 1" in the present invention. The interleukin 2 analog according to the present invention may have changed interleukin 2 alpha receptor binding affinity and/or increased interleukin 2 beta receptor binding affinity compared to the interleukin 2 analog 1.

Therefore, the interleukin 2 analog with increased binding affinity to the interleukin 2 beta receptor of the present invention can have a therapeutic effect with increased therapeutic effects such as tumor suppression and death, and decreased side effects.

In the present invention, the interleukin 2 analog may include a sequence in which amino acid at position 1 is deleted and amino acid at position 125 is substituted with a different amino acid in native interleukin 2, and may additionally include 1 to 10 amino acid mutations. For example, the interleukin 2 analog may include a sequence in which amino acid at position 125 is substituted with serine, and one or more of amino acids at positions 12, 18, 19, 20, 22, 32, 35, 38, 42, 43, 45, 48, 49, 61, 68, 69, 74, 76, 80, 81, 82, 84, 85, 86, 87, 88, 89, 91, 92,94, 95, 96, and 126 are additionally substituted with different amino acids, and/or one or more amino acids are added to amino acid at position 133, but is not limited thereto. Interleukin 2 analogues having changed interleukin 2 alpha receptor binding affinity and increased interleukin 2 beta receptor binding affinity compared to native interleukin 2 and/or aldesleukin are included without limitation.

As an example of the interleukin 2 analog of the present invention, the interleukin 2 analog may have one or more amino acids added to the amino acid corresponding to position 133, but is not limited thereto.

As another example, the interleukin 2 analogue may be one in which amino acid at position 1 is deleted, amino acid at position 125 is substituted with a different amino acid, and one, two, three, four, five, six, seven, eight, nine or more amino acids among amino acids at positions 18, 19, 20, 22, 38, 42, 43, 45, 61, 68, 69, 74, 80, 81, 84, 85, 86, 88, 89, 91, 92, 94, and 96 are substituted with different amino acids in native interleukin 2, but is not limited thereto.

As still another example, the interleukin 2 analogue may be one in which amino acid at position 1 is deleted, amino acid at position 125 is substituted with a different amino acid, and one, two, three, four, five, six, seven, eight, nine or more amino acids among amino acids at position 18, 19, 22, 38, 42, 43, 45, 61, 68, 74, 80, 81, 84, 85, 86, 88, 91, 92, 94, and 96 are additionally substituted with different amino acids in native interleukin 2, but is not limited thereto.

As still another example, the interleukin 2 analogue may be one in which amino acid at position 1 is deleted, amino acid at position 125 is substituted with a different amino acid, and one or more amino acids among amino acids corresponding to positions 18, 22, 38, 42, 61, 68, 80, 81, 85, 86 and 92 are additionally substituted with a different amino acid in native interleukin 2, but is not limited thereto.

As still another example, the interleukin 2 analog may be any one selected from the group consisting of the following analogs:
(a) an interleukin 2 analog in which the amino acid at position 1 is removed and the amino acids at positions 125 and 32 are substituted with different amino acids in native interleukin 2;
(b) an interleukin 2 analog in which the amino acid at position 1 is removed and the amino acids at positions 125 and 35 are substituted with different amino acids in native interleukin 2;
(c) an interleukin 2 analog in which the amino acid at position 1 is removed and the amino acids at positions 125 and 38 are substituted with different amino acids in native interleukin 2;
(d) an interleukin 2 analog in which the amino acid at position 1 is removed and the amino acids at positions 125 and 42 are substituted with different amino acids in native interleukin 2;
(e) an interleukin 2 analog in which the amino acid at position 1 is removed and the amino acids at positions 125 and 43 are substituted with different amino acids in native interleukin 2;
(f) an interleukin 2 analog in which the amino acid at position 1 is removed and the amino acids at positions 125 and 48 are substituted with different amino acids in native interleukin 2;
(g) an interleukin 2 analog in which the amino acid at position 1 is removed and the amino acids at positions 125 and 49 are substituted with different amino acids in native interleukin 2;
(h) an interleukin 2 analog in which the amino acid at position 1 is removed and the amino acids at positions 125 and 76 are substituted with different amino acids in native interleukin 2;
(i) an interleukin 2 analog in which the amino acid at position 1 is removed and the amino acids at positions 125, 38, 42, 81, 92, 94, and 96 are substituted with different amino acids in native interleukin 2;
(j) an interleukin 2 analog in which the amino acid at position 1 is removed and the amino acids at positions 125 and 87 are substituted with different amino acids in native interleukin 2;
(k) an interleukin 2 analog in which the amino acid at position 1 is removed and the amino acids at positions 125, 38, and 42 are substituted with different amino acids in native interleukin 2;
(l) an interleukin 2 analog in which the amino acid at position 1 is removed and the amino acids at positions 125, 38, and 80 are substituted with different amino acids in native interleukin 2;
(m) an interleukin 2 analog in which the amino acid at position 1 is removed and the amino acids at positions 125, 38, and 84 are substituted with different amino acids in native interleukin 2;
(n) an interleukin 2 analog in which the amino acid at position 1 is removed and the amino acids at positions 125, 19, 38, and 42 are substituted with different amino acids in native interleukin 2;
(o) an interleukin 2 analog in which the amino acid at position 1 is removed and the amino acids at positions 125, 12, 38, and 42 are substituted with different amino acids in native interleukin 2;
(p) an interleukin 2 analog in which the amino acid at position 1 is removed and the amino acids at positions 125, 38, 42, and 61 are substituted with different amino acids in native interleukin 2;
(q) an interleukin 2 analog in which the amino acid at position 1 is removed and the amino acids at positions 125, 38, 42, and 84 are substituted with different amino acids in native interleukin 2;
(r) an interleukin 2 analog in which the amino acid at position 1 is removed and the amino acids at positions 125, 38, 42, and 88 are substituted with different amino acids in native interleukin 2;
(s) an interleukin 2 analog in which the amino acid at position 1 is removed and the amino acids at positions 125, 38, 42, and 89 are substituted with different amino acids in native interleukin 2;
(t) an interleukin 2 analog in which the amino acid at position 1 is removed and the amino acids at positions 125, 38, 42, and 91 are substituted with different amino acids in native interleukin 2;
(u) an interleukin 2 analog in which the amino acid at position 1 is removed and the amino acids at positions 125, 38, 42, and 94 are substituted with different amino acids in native interleukin 2;
(v) an interleukin 2 analog in which the amino acid at position 1 is removed and the amino acids at positions 125, 38, 42, and 126 are substituted with different amino acids in native interleukin 2;
(w) an interleukin 2 analog in which the amino acid at position 1 is removed and the amino acids at positions 125, 38, 80, and 84 are substituted with different amino acids in native interleukin 2;
(x) an interleukin 2 analog in which the amino acid at position 1 is removed and the amino acids at positions 125, 38, 94, and 96 are substituted with different amino acids in native interleukin 2;
(y) an interleukin 2 analog in which the amino acid at position 1 is removed and the amino acids at positions 125, 38, 81, and 92 are substituted with different amino acids in native interleukin 2;
(z) an interleukin 2 analog in which the amino acid at position 1 is removed and the amino acids at positions 125, 61, 81, and 92 are substituted with different amino acids in native interleukin 2;
(aa) an interleukin 2 analog in which the amino acid at position 1 is removed and the amino acids at positions 125, 38, 42, 81, and 92 are substituted with different amino acids in native interleukin 2;
(ab) an interleukin 2 analog in which the amino acid at position 1 is removed and the amino acids at positions 125, 38, 80, 81, and 92 are substituted with different amino acids in native interleukin 2;
(ac) an interleukin 2 analog in which the amino acid at position 1 is removed and the amino acids at positions 125, 38, 81, 84, and 92 are substituted with different amino acids in native interleukin 2;
(ad) an interleukin 2 analog in which the amino acid at position 1 is removed and the amino acids at positions 125, 20, 38, 42, 81, and 92 are substituted with different amino acids in native interleukin 2;
(ae) an interleukin 2 analog in which the amino acid at position 1 is removed and the amino acids at positions 125, 38, 42, 80, 81, and 92 are substituted with different amino acids in native interleukin 2;
(af) an interleukin 2 analog in which the amino acid at position 1 is removed and the amino acids at positions 125, 38, 42, 74, 81, and 92 are substituted with different amino acids in native interleukin 2;
(ag) an interleukin 2 analog in which the amino acid at position 1 is removed and the amino acids at positions 125, 38, 42, 81, 84, and 92 are substituted with different amino acids in native interleukin 2;
(ah) an interleukin 2 analog in which the amino acid at position 1 is removed and the amino acids at positions 125, 38, 42, 81, 88, and 92 are substituted with different amino acids in native interleukin 2;
(ai) an interleukin 2 analog in which the amino acid at position 1 is removed and the amino acids at positions 125, 38, 42, 85, 86, and 92 are substituted with different amino acids in native interleukin 2;
(aj) an interleukin 2 analog in which the amino acid at position 1 is removed and the amino acids at positions 125, 38, 80, 81, 85, and 92 are substituted with different amino acids in native interleukin 2;
(ak) an interleukin 2 analog in which the amino acid at position 1 is removed and the amino acids at positions 125, 38, 80, 81, 86, and 92 are substituted with different amino acids in native interleukin 2;
(al) an interleukin 2 analog in which the amino acid at position 1 is removed and the amino acids at positions 125, 80, 81, 85, 86, and 92 are substituted with different amino acids in native interleukin 2;
(am) an interleukin 2 analog in which the amino acid at position 1 is removed and the amino acids at positions 125, 38, 42, 45, 74, 81, and 92 are substituted with different amino acids in native interleukin 2;
(an) an interleukin 2 analog in which the amino acid at position 1 is removed and the amino acids at positions 125, 38, 42, 74, 80, 81, and 92 are substituted with different amino acids in native interleukin 2;
(ao) an interleukin 2 analog in which the amino acid at position 1 is removed and the amino acids at positions 125, 38, 42, 80, 81, 84, and 92 are substituted with different amino acids in native interleukin 2;
(ap) an interleukin 2 analog in which the amino acid at position 1 is removed and the amino acids at positions 125, 38, 42, 45, 80, 85, 86, and 92 are substituted with different amino acids in native interleukin 2;
(aq) an interleukin 2 analog in which the amino acid at position 1 is removed and the amino acids at positions 125, 38, 80, 81, 85, 86, and 92 are substituted with different amino acids in native interleukin 2;
(ar) an interleukin 2 analog in which the amino acid at position 1 is removed and the amino acids at positions 125, 42, 80, 81, 85, 86, and 92 are substituted with different amino acids in native interleukin 2;
(as) an interleukin 2 analog in which the amino acid at position 1 is removed and the amino acids at positions 125, 61, 80, 81, 85, 86, and 92 are substituted with different amino acids in native interleukin 2;
(at) an interleukin 2 analog in which the amino acid at position 1 is removed and the amino acids at positions 125, 69, 80, 81, 85, 86, and 92 are substituted with different amino acids in native interleukin 2;
(au) an interleukin 2 analog in which the amino acid at position 1 is removed and the amino acids at positions 125, 80, 81, 85, 86, 91, and 92 are substituted with different amino acids in native interleukin 2;
(av) an interleukin 2 analog in which the amino acid at position 1 is removed and the amino acids at positions 125, 18, 22, 80, 81, 85, 86, and 92 are substituted with different amino acids in native interleukin 2;
(aw) an interleukin 2 analog in which the amino acid at position 1 is removed and the amino acids at positions 125, 38, 42, 80, 81, 85, 86, and 92 are substituted with different amino acids in native interleukin 2;
(ax) an interleukin 2 analog in which the amino acid at position 1 is removed and the amino acids at positions 125, 38, 42, 45, 74, 80, 81, and 92 are substituted with different amino acids in native interleukin 2;
(ay) an interleukin 2 analog in which the amino acid at position 1 is removed and the amino acids at positions 125, 38, 68, 80, 81, 85, 86, and 92 are substituted with different amino acids in native interleukin 2;
(az) an interleukin 2 analog in which the amino acid at position 1 is removed and the amino acids at positions 125, 69, 74, 80, 81, 85, 86, and 92 are substituted with different amino acids in native interleukin 2;
(ba) an interleukin 2 analog in which the amino acid at position 1 is removed and the amino acids at positions 125, 80, 81, 84, 85, 86, 91, and 92 are substituted with different amino acids in native interleukin 2;
(bb) an interleukin 2 analog in which the amino acid at position 1 is removed and the amino acids at positions 125, 80, 81, 85, 86, 92, 94, and 96 are substituted with different amino acids in native interleukin 2;
(bc) an interleukin 2 analog in which the amino acid at position 1 is removed and the amino acids at positions 125, 18, 19, 22, 80, 81, 85, 86, and 92 are substituted with different amino acids in native interleukin 2;
(bd) an interleukin 2 analog in which the amino acid at position 1 is removed and the amino acids at positions 125, 18, 22, 38, 80, 81, 85, 86, and 92 are substituted with different amino acids in native interleukin 2;
(be) an interleukin 2 analog in which the amino acid at position 1 is removed and the amino acids at positions 125, 18, 22, 61, 80, 81, 85, 86, and 92 are substituted with different amino acids in native interleukin 2;
(bf) an interleukin 2 analog in which the amino acid at position 1 is removed and the amino acids at positions 125, 18, 22, 68, 80, 81, 85, 86, and 92 are substituted with different amino acids in native interleukin 2;
(bg) an interleukin 2 analog in which the amino acid at position 1 is removed and the amino acids at positions 125, 35, 38, 42, 80, 81, 85, 86, and 92 are substituted with different amino acids in native interleukin 2;
(bh) an interleukin 2 analog in which the amino acid at position 1 is removed and the amino acids at positions 125, 38, 42, 45, 80, 81, 85, 86, and 92 are substituted with different amino acids in native interleukin 2;
(bi) an interleukin 2 analog in which the amino acid at position 1 is removed and the amino acids at positions 125, 38, 42, 80, 81, 85, 86, 91, and 92 are substituted with different amino acids in native interleukin 2;
(bj) an interleukin 2 analog in which the amino acid at position 1 is removed and the amino acids at positions 125, 38, 42, 80, 81, 85, 86, 92, and 95 are substituted with different amino acids in native interleukin 2;
(bk) an interleukin 2 analog in which the amino acid at position 1 is removed and the amino acids at positions 125, 35, 38, 42, 74, 80, 81, 85, 86, and 92 are substituted with different amino acids in native interleukin 2;
(bl) an interleukin 2 analog in which the amino acid at position 1 is removed and the amino acids at positions 125, 38, 42, 43, 61, 80, 81, 85, 86, and 92 are substituted with different amino acids in native interleukin 2;
(bm) an interleukin 2 analog in which the amino acid at position 1 is removed and the amino acids at positions 125, 38, 42, 80, 81, 85, 86, 91, 92, and 95 are substituted with different amino acids in native interleukin 2;
(bn) an interleukin 2 analog in which the amino acid at position 1 is removed and the amino acids at positions 125, 35, 38, 42, 74, 80, 81, 82, 85, 86, and 92 are substituted with different amino acids in native interleukin 2;
(bo) an interleukin 2 analog in which the amino acid at position 1 is removed and the amino acids at positions 125, 80, 81, 86, and 92 are substituted with different amino acids in native interleukin 2; and
(bp) an interleukin 2 analog in which the amino acid at position 1 is removed and the amino acids at positions 125, 80, 81, 85, and 86 are substituted with different amino acids in native interleukin 2.

Here, the amino acid substitution contained in the interleukin 2 analogue may be any one or more selected from the group consisting of the following amino acid substitutions:
(a) a substitution in which the amino acid at position 12 is substituted with valine or phenylalanine;
(b) a substitution in which the amino acid at position 18 is substituted with arginine;
(c) a substitution in which the amino acid at position 19 is substituted with tyrosine, valine, phenylalanine, or arginine;
(d) a substitution in which the amino acid at position 20 is substituted with valine or phenylalanine;
(e) a substitution in which the amino acid at position 22 is substituted with glutamic acid;
(f) a substitution in which the amino acid at position 32 is substituted with cysteine;
(g) a substitution in which the amino acid at position 35 is substituted with cysteine or glutamic acid;
(h) a substitution in which the amino acid at position 38 is substituted with alanine or aspartic acid;
(i) a substitution in which the amino acid at position 42 is substituted with lysine, alanine, or tryptophan;
(j) a substitution in which the amino acid at position 43 is substituted with cysteine, glutamic acid, or glutamine;
(k) a substitution in which the amino acid at position 45 is substituted with alanine;
(l) a substitution in which the amino acid at position 48 is substituted with cysteine;
(m) a substitution in which the amino acid at position 49 is substituted with cysteine;
(n) a substitution in which the amino acid at position 61 is substituted with glutamine, arginine, or aspartic acid;
(o) a substitution in which the amino acid at position 68 is substituted with aspartic acid or glutamine;
(p) a substitution in which the amino acid at position 69 is substituted with glycine;
(q) a substitution in which the amino acid at position 74 is substituted with histidine or alanine;
(r) a substitution in which the amino acid at position 76 is substituted with cysteine;
(s) a substitution in which the amino acid at position 80 is substituted with phenylalanine, tyrosine, valine, aspartic acid, or tryptophan;
(t) a substitution in which the amino acid at position 81 is substituted with aspartic acid, glutamic acid, or asparagine;
(u) a substitution in which the amino acid at position 82 is substituted with glycine or valine;
(v) a substitution in which the amino acid at position 84 is substituted with glutamic acid, valine, or phenylalanine;
(w) a substitution in which the amino acid at position 85 is substituted with valine, alanine, glycine, tryptophan, tyrosine, threonine, isoleucine, glutamic acid, or phenylalanine;
(x) a substitution in which the amino acid at position 86 is substituted with valine, alanine, glycine, or leucine;
(y) a substitution in which the amino acid at position 87 is substituted with cysteine;
(z) a substitution in which the amino acid at position 88 is substituted with glutamine, valine, or phenylalanine;
(aa) a substitution in which the amino acid at position 89 is substituted with phenylalanine;
(ab) a substitution in which the amino acid at position 91 is substituted with threonine, phenylalanine, or glutamic acid;
(ac) a substitution in which the amino acid at position 92 is substituted with phenylalanine, leucine, tyrosine, or tryptophan;
(ad) a substitution in which the amino acid at position 94 is substituted with phenyl alanine or valine;
(ae) a substitution in which the amino acid at position 95 is substituted with aspartic acid;
(af) a substitution in which the amino acid at position 96 is substituted with phenylalanine, valine, or isoleucine;
(ag) a substitution in which the amino acid at position 125 is substituted with serine; and
(ah) a substitution in which the amino acid at position 126 is substituted with threonine.

As still another example, the interleukin 2 analog may be any one selected from the following interleukin 2 analogs:
(a) an interleukin 2 analogue in which amino acid at position 1 is deleted and amino acids at positions 125, 38, 42, 80, 81 and 92 are substituted with different amino acids in native interleukin 2; (b) an interleukin 2 analogue in which amino acid at position 1 is deleted and amino acids at positions 125, 80, 81, 85, 86 and 92 are substituted with different amino acids in native interleukin 2; (c) an interleukin 2 analogue in which amino acid at position 1 is deleted and amino acids at positions 125, 18, 22, 80, 81, 85, 86, and 92 are substituted with different amino acids in native interleukin 2; (d) an interleukin 2 analogue in which amino acid at position 1 is deleted and amino acids at positions 125, 38, 42, 80, 81, 85, 86, and 92 are substituted with different amino acids in native interleukin 2; (e) an interleukin 2 analogue in which amino acid at position 1 is deleted and amino acids at positions 125, 18, 22, 61, 80, 81, 85, 86, and 92 are substituted with different amino acids in native interleukin 2; and (f) an interleukin 2 analogue in which amino acid at position 1 is deleted and amino acids at positions 125, 18, 22, 68, 80, 81, 85, 86, and 92 are substituted with different amino acids in native interleukin 2.

Here, the amino acid substitution contained in the interleukin 2 analogue may be any one or more selected from the group consisting of the following amino acid substitutions:
(a) substitution of amino acid at position 18 with arginine; (b) substitution of amino acid at position 22 with glutamic acid; (c) substitution of amino acid at position 38 with alanine; (d) substitution of amino acid at position 42 with lysine; (e) substitution of amino acid at position 61 with aspartic acid; (f) substitution of amino acid at position 68 with aspartic acid; (g) substitution of amino acid at position 80 with phenylalanine; (h) substitution of amino acid at position 81 with glutamic acid; (i) substitution of amino acid at position 85 with valine; (j) substitution of amino acid at position 86 with valine; (k) substitution of amino acid at position 92 with phenylalanine; and (l) substitution of amino acid at position 125 with serine.

In this specification, the term "corresponding to" refers to an amino acid residue at a position listed in the peptide, or an amino acid residue that is similar, identical, or homologous to a residue listed in the peptide. Identifying the amino acid at the corresponding position may be determining a specific amino acid in a sequence that references a specific sequence.

For example, an arbitrary amino acid sequence may be aligned with SEQ ID NO: 1, and based on this, each amino acid residue of the amino acid sequence may be numbered by referring to the numerical position of the amino acid residue corresponding to the amino acid residue of SEQ ID NO: 1.

For such alignment, the Needleman-Wunsch algorithm (Needleman and Wunsch, 1970, J. Mol. Biol. 48: 443-453), the Needle program of the EMBOSS package (EMBOSS: The European Molecular Biology Open Software Suite, Rice et al., 2000), Trends Genet. 16: 276-277), and the like may be used, but the alignment is not limited thereto, and any sequence alignment program known in the art, pairwise sequence comparison algorithm, and the like may be appropriately used.

Even if it is expressed as a specific position of an amino acid in a peptide in the present invention, it may mean a corresponding position in the reference sequence.

As still another example, the interleukin 2 analog may include, essentially consist of, or consist of, but is not limited to, an amino acid sequence selected from the group consisting of SEQ ID NOs: 3 to 106.

Even if it is described as 'an interleukin 2 analog consisting of a specific sequence number' in this specification, it does not exclude meaningless sequence additions before and after the amino acid sequence of the corresponding sequence number, mutations that may occur naturally, or silent mutations thereof as long as it has the same or corresponding activity as the interleukin 2 analog consisting of an amino acid sequence of the corresponding sequence number, and it is self-evident that it falls within the scope of the present invention even if it has such sequence additions or mutations.

The interleukin 2 analog of the present invention may include an amino acid sequence having 40%, 45%, 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% or more homology or identity with the amino acid sequence of SEQ ID NOs: 3 to 106, but is not limited thereto.

As a specific example, the interleukin 2 analog of the present invention may include, essentially consist of, or consist of, an amino acid sequence selected from the group consisting of SEQ ID NOs: 22, 42, 53, 87, 105, and 106, but is not limited thereto.

In the present invention, the term 'homology' or 'identity' means the degree to which two given amino acid sequences or base sequences are related to each other, and may be expressed as a percentage.

Sequence homology or identity of conserved polynucleotides or polypeptides is determined by standard alignment algorithms, and a default gap penalty established by the program being used may be used in conjunction with this. In practice, homologous or identical sequences can generally hybridize with all or part of the sequence under moderate or high stringent conditions. It is self-evident that hybridization also includes hybridization with polynucleotides containing common codons or codons taking codon degeneracy into account in the polynucleotide.

The terms homology and identity are often used interchangeably.

Whether any two base sequences or peptide sequences are homologous, similar or identical may be determined by well-known computer algorithms such as the "FASTA" program, for example, using default parameters such as those in Pearson et al (1988) [Proc. Natl. Acad. Sci. USA 85]: 2444. Alternatively, the homology, similarity, or identity may be determined using the Needleman-Wunsch algorithm (Needleman and Wunsch, 1970, J. Mol. Biol. 48: 443-453) as performed by the Needleman program of the EMBOSS package (EMBOSS: The European Molecular Biology Open Software Suite, Rice et al., 2000, Trends Genet. 16: 276-277) (version 5.0.0 or later) (including the GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, the homology, similarity, or identity may be determined using BLAST from the National Center for Biotechnology Information Database, or ClustalW.

The homology, similarity or identity of base sequences or peptides may be determined by comparing sequence information using, for example, the GAP computer program, such as Needleman et al. (1970), J Mol Biol. 48: 443, for example, as announced in Smith and Waterman, Adv. Appl. Math (1981) 2:482. In summary, the GAP program defines the homology, similarity or identity as the value acquired by dividing the total number of symbols in the shorter of the two sequences by the number of similarly arranged symbols (that is, nucleotides or amino acids). The default parameters for the GAP program may include (1) a binary comparison matrix (containing values of 1 for equality and 0 for non-equality) and a weighted comparison matrix from Gribskov et al(1986) Nucl. Acids Res. 14: 6745 (or EDNAFULL (the EMBOSS version of NCBI NUC4.4)) as described by Schwartz and Dayhoff, eds., Atlas Of Protein Sequence And Structure, National Biomedical Research Foundation, pp. 353-358 (1979); (2) a penalty of 3.0 for each gap and an additional penalty of 0.10 for each symbol in each gap (or a gap opening penalty of 10 and a gap extension penalty of 0.5); and (3) no penalty for terminal gaps. Therefore, as used in the present invention, the term "homology" or "identity" indicates relevance between sequences.

The above may be applied to other specific examples or other aspects of the present invention, but the present invention is not limited thereto.

The interleukin 2 analog according to the present invention may be in a modified form in which the N-terminus and/or C-terminus, and the like are chemically modified or protected with an organic group, or an amino acid is added to the peptide terminus, and the like in order to protect the interleukin 2 analog against protein cleavage enzymes in the living body and to increase stability of the interleukin 2 analog.

In particular, in the case of chemically synthesized peptides, since the N-terminus and C-terminus are charged, the N-terminus may be acetylated and/or the C-terminus may be amidated to remove the charge, but these are not particularly limited thereto.

The interleukin 2 analog of the present invention can be synthesized through a solid-phase synthesis method, can be produced by a recombinant method, and can be manufactured by commercial request, but is not limited thereto.

The interleukin 2 analog of the present invention can be synthesized by a method well known in the art depending on its length, for example, using an automatic peptide synthesizer, or can be produced by genetic engineering techniques.

Specifically, the interleukin 2 analog of the present invention can be prepared by standard synthetic methods, recombinant expression systems, or any other method in the art. Accordingly, the interleukin 2 analogue according to the present invention can be synthesized by a number of methods, including, for example, the following methods:
(a) a method in which a peptide is synthesized stepwise by means of a solid-phase or liquid-phase method or by fragment assembly and the final peptide product is isolated and purified; or
(b) a method in which a nucleic acid construct encoding a peptide is expressed in a host cell and the expression product is recovered from the host cell culture; or
(c) a method in which cell-free *in vitro* expression of a nucleic acid construct encoding a peptide is performed and the expression product is recovered; or
a method in which fragments of a peptide are obtained by any combination of (a), (b), and (c), then the fragments are linked to obtain a peptide, and the peptide is recovered.

In the present invention, the interleukin 2 analog may correspond to a component of a moiety constituting the conjugate. The interleukin 2 analog may specifically correspond to X in Chemical Formula 1. In the conjugate, F is a substance capable of increasing the half-life of X, that is, an interleukin 2 analog, and corresponds to a component of a moiety constituting the conjugate of the present invention.

The F and X may be bound to each other by a covalent chemical bond, and F and X may be bound to each other via L by a covalent chemical bond.

Specifically, the F is an immunoglobulin Fc region, and the immunoglobulin Fc region may be an IgG Fc region or an aglycosylated IgG4 Fc region, but is not particularly limited thereto.

As a specific example of the present invention, the conjugate may have a structure in which the F (immunoglobulin Fc region) is a dimer composed of two polypeptide chains and one end of L is linked to only one of the two polypeptide chains, but is not limited thereto.

In the present invention, "immunoglobulin Fc region" means a region including the heavy chain constant region 2 (CH2) and/or the heavy chain constant region 3 (CH3) portion, excluding the heavy chain and light chain variable regions of immunoglobulin. The immunoglobulin Fc region may be a component forming a moiety of the conjugate of the present invention. Specifically, the immunoglobulin Fc region corresponds to F in Chemical Formula 1.

In this specification, the term Fc region includes not only the native sequence obtained from papain digestion of immunoglobulin, but also derivatives thereof, for example, variants such as a sequence in which one or more amino acid residues in the native sequence are changed by deletion, insertion, non-conservative or conservative substitution, or a combination thereof, and the sequence is thus different from the native form. It is assumed that the derivative, substitution product, and variant have the ability to bind to FcRn. In the present invention, F may be, but is not limited to, a human immunoglobulin region. In this specification, "biocompatible substance" or "carrier" may refer to the Fc region.

The F (immunoglobulin Fc region) is a structure in which two polypeptide chains are linked by a disulfide bond, and may be a structure in which two polypeptide chains are linked by only the nitrogen atom of one of the two chains, but is not limited thereto. The linkage via a nitrogen atom may be achieved through reductive amination to the epsilon amino atom of lysine or to the N-terminal amino group.

Reductive amination reaction refers to a reaction in which the amine group or amino group of a reactant reacts with the aldehyde of another reactant (that is, a functional group capable of reductive amination) to generate an amine, and then an amine bond is formed through a reduction reaction, and is an organic synthesis reaction that is widely known in the art.

As a specific example of the long-acting conjugate of the present invention, the long-acting conjugate may be one in which the immunoglobulin Fc region is linked to a linker via its N-terminal nitrogen atom.

Such an immunoglobulin Fc region may include, but is not limited to, a hinge region in the heavy chain constant region.

In the present invention, the immunoglobulin Fc region may include a specific hinge sequence at the N-terminus.

The term "hinge sequence" of the present invention refers to a site that is located in the heavy chain where a dimer of the immunoglobulin Fc region is formed through a disulfide bond.

In the present invention, the hinge sequence may be mutated to have only one cysteine residue by deleting a portion of the hinge sequence having the following amino acid sequence, but is not limited thereto:
Glu-Ser-Lys-Tyr-Gly-Pro-Pro-Cys-Pro-Ser-Cys-Pro (SEQ ID NO: 107).

The hinge sequence may be one in which the 8th or 11th cysteine residue of the hinge sequence of SEQ ID NO: 107 is deleted, thereby containing only one cysteine residue. The hinge sequence of the present invention may be composed of 3 to 12 amino acids, including only one cysteine residue, but is not limited thereto. More specifically, the hinge sequence of the present invention may have the following sequence:

Glu-Ser-Lys-Tyr-Gly-Pro-Pro-Pro-Ser-Cys-Pro (SEQ ID NO: 108), Glu-Ser-
Lys-Tyr-Gly-Pro-Pro-Cys-Pro-Ser-Pro(SEQ ID NO: 109), Glu-Ser-Lys-Tyr-Gly-Pro-
Pro-Cys-Pro-Ser(SEQ ID NO: 110), Glu-Ser-Lys-Tyr-Gly-Pro-Pro-Cys-Pro-
Pro(SEQ ID NO: 111), Lys-Tyr-Gly-Pro-Pro-Cys-Pro-Ser(SEQ ID NO: 112), Glu-
Ser-Lys-Tyr-Gly-Pro-Pro-Cys(SEQ ID NO: 113), Glu-Lys-Tyr-Gly-Pro-Pro-
Cys(SEQ ID NO: 114), Glu-Ser-Pro-Ser-Cys-Pro (SEQ ID NO: 115), Glu-Pro-Ser-
Cys-Pro (SEQ ID NO: 116), Pro-Ser-Cys-Pro (SEQ ID NO: 117), Glu-Ser-Lys-Tyr-
Gly-Pro-Pro-Ser-Cys-Pro(SEQ ID NO: 118), Lys-Tyr-Gly-Pro-Pro-Ser-Cys-
Pro(SEQ ID NO: 119), Glu-Ser-Lys-Tyr-Gly-Pro-Ser-Cys-Pro(SEQ ID NO: 120),
Glu-Ser-Lys-Tyr-Gly-Pro-Pro-Cys(SEQ ID NO: 121), Lys-Tyr-Gly-Pro-Pro-Cys-
Pro(SEQ ID NO: 122), Glu-Ser-Lys-Pro-Ser-Cys-Pro(SEQ ID NO: 123), Glu-Ser-
Pro-Ser-Cys-Pro (SEQ ID NO: 124), Glu-Pro-Ser-Cys (SEQ ID NO: 125), and Ser-
Cys-Pro (SEQ ID NO: 126).

Still more specifically, the hinge sequence may include, but is not limited to, an amino acid sequence of SEQ ID NO: 117 (Pro-Ser-Cys-Pro) or SEQ ID NO: 126 (Ser-Cys-Pro).

In a more specific form of the long-acting conjugate of the present invention, the N-terminus of the immunoglobulin Fc region in the conjugate is proline, and this conjugate is one in which the Fc region is linked to a linker via the nitrogen atom of the proline.

In a specific embodiment, the immunoglobulin Fc region may be in a dimeric form in which two chains of the immunoglobulin Fc region form a homodimer or a heterodimer due to the presence of a hinge sequence. The conjugate represented by Chemical Formula 1 of the present invention may be in a form in which one end of the linker is linked to one chain of the immunoglobulin Fc region of the dimer, but is not limited thereto.

The term "N-terminus" of the present invention means the amino terminus of a protein or polypeptide, and may include the most end of the amino terminus, or 1, 2, 3, 4, 5, 6, 7, 8, 9, or 10 or more amino acids from the most end. The immunoglobulin Fc region of the present invention may include a hinge sequence at the N-terminus, but is not limited thereto.

The immunoglobulin Fc region of the present invention may be an extended Fc region including part or all of the heavy chain constant region 1 (CH1) and/or the light chain constant region 1 (CL1), excluding only the heavy and light chain variable regions of the immunoglobulin, as long as it has substantially equivalent or improved effects compared to the natural form. The immunoglobulin Fc may be a region in which a relatively long portion of the amino acid sequence corresponding to CH2 and/or CH3 is deleted.

For example, the immunoglobulin Fc region of the present invention may be 1) a CH1 domain, a CH2 domain, a CH3 domain and a CH4 domain, 2) a CH1 domain and a CH2 domain, 3) a CH1 domain and a CH3 domain, 4) a CH2 domain and a CH3 domain, 5) a combination of one or more of a CH1 domain, CH2 domain, CH3 domain or CH4 domain with an immunoglobulin hinge region (or a part of the hinge region), or 6) a dimer of each domain of a heavy chain constant region and a light chain constant region, but is not limited thereto.

In the present invention, the immunoglobulin Fc region may be in the form of a dimer or multimer composed of single-chain immunoglobulins consisting of domains of the same origin, but is not limited thereto.

As an embodiment of the long-acting conjugate of the present invention, the immunoglobulin Fc region F is a dimer consisting of two polypeptide chains, at this time, the Fc region dimer F and X are covalently linked to each other by one and the same linker L containing an ethylene glycol repeating unit. In a specific example of this embodiment, X is covalently linked to only one of the two polypeptide chains of such Fc region dimer F via the linker L. In a more specific example of this embodiment, only one molecule of X is covalently linked via L to one polypeptide chain to which X is linked of the two polypeptide chains of such Fc region dimer F. In the most specific example of this embodiment, the F is a homodimer.

In another specific example, the immunoglobulin Fc region F is a dimer consisting of two polypeptide chains, and one end of L may be linked to only one of the two polypeptide chains, but the immunoglobulin Fc region F is not limited thereto.

In still another embodiment of the long-acting conjugate of the present invention, it is also possible for two molecules of X to bind symmetrically to one Fc region in a dimeric form. At this time, the immunoglobulin Fc region and X may be linked to each other by L. However, the long-acting conjugate is not limited to the examples described above.

The immunoglobulin Fc region of the present invention includes not only a native amino acid sequence but also a sequence derivative thereof. An amino acid sequence derivative means one that has a different sequence from the native amino acid sequence due to deletion, insertion, non-conservative or conservative substitution of one or more amino acid residues in the native amino acid sequence, or a combination thereof.

For example, in the case of IgG Fc, amino acid residues 214 to 238, 297 to 299, 318 to 322 or 327 to 331, which are known to be important for binding, may be used as suitable sites for modification.

Various types of derivatives are possible, such as those in which the site capable of forming a disulfide bond may be deleted, those in which several amino acids at the N-terminus of the native Fc may be deleted, or those in which a methionine residue may be added to the N-terminus of the native Fc. To eliminate the effector function, complement binding sites, for example, the C1q binding site, may be deleted, or the ADCC (antibody dependent cell mediated cytotoxicity) site may be deleted. Techniques for preparing such sequence derivatives of the immunoglobulin Fc region are disclosed in WO 97/34631, WO 96/32478, and the like.

Amino acid exchanges in proteins and peptides that do not alter the overall activity of the molecule are known in the art (H.Neurath, R.L.Hill, The Proteins, Academic Press, New York, 1979). The most commonly occurring exchange is between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, modification may be achieved by phosphorylation, sulfation, acrylation, glycosylation, methylation, farnesylation, acetylation, and amidation.

The Fc derivative described above exhibits biological activity equivalent to the Fc region of the present invention and may have enhanced structural stability of the Fc region with respect to heat, pH, and the like.

Such an Fc region may be obtained from a natural form isolated from living animals such as humans, cows, goats, pigs, mice, rabbits, hamsters, rats or guinea pigs, or may be a recombinant form obtained from a transformed animal cell or microorganism, or a derivative thereof. Here, the method of obtaining the Fc region from a natural form may be a method in which the entire immunoglobulin is isolated from a human or animal living body and then treated with a protease. The immunoglobulin is cleaved into Fab and Fc when treated with papain, and is cleaved into pF'c and F(ab)2 when treated with pepsin. Fc or pF'c can be separated by size exclusion chromatography and the like. In a more specific embodiment, the Fc region is a recombinant immunoglobulin Fc region obtained from a microorganism, which is a human-derived Fc region.

The immunoglobulin Fc region may have natural glycans or increased or decreased glycans compared to the natural type, or be in a deglycosylated form. Conventional methods such as chemical methods, enzymatic methods, and genetic engineering methods using microorganisms may be used to increase or decrease or delete such glycans of the immunoglobulin Fc. Here, the immunoglobulin Fc region in which the glycans is deleted from Fc has significantly reduced binding affinity to complement (C1q), reduced or eliminated antibody-dependent cytotoxicity or complement-dependent cytotoxicity, and thus does not induce unnecessary immune responses *in vivo.* Based thereon, a deglycosylated or aglycosylated immunoglobulin Fc region may be more suitable as a drug carrier in view of the objects of the present invention.

As used herein, the term "deglycosylation" refers to a Fc region from which glycan is removed using an enzyme and the term "aglycosylation" refers to a Fc region that is not glycosylated and produced in prokaryotes, more specifically E. *coli.*

Meanwhile, the immunoglobulin Fc region can be of animal origin such as human or cow, goat, pig, mouse, rabbit, hamster, rat, or guinea pig, and in a more specific embodiment, of human origin.

The immunoglobulin Fc region may be an Fc region derived from IgG, IgA, IgD, IgE, or IgM or by a combination thereof or a hybrid thereof. The immunoglobulin Fc region is derived from IgG or IgM, which is most abundant in human blood in a more specific embodiment, and is derived from IgG, which is known to enhance the half-life of ligand-binding protein in a still more specific embodiment. In a yet still more specific embodiment, the immunoglobulin Fc region is an IgG4 Fc region. In the most specific embodiment, the immunoglobulin Fc region is, but is not limited to, an aglycosylated Fc region derived from human IgG4.

As a specific example, the immunoglobulin Fc region may be a fragment of human IgG4 Fc, which may be a homodimer in which two monomers are linked via a disulfide bond (inter-chain form) between cysteines, the 3rd amino acid of each monomer. At this time, the homodimer has/can have two disulfide bonds (intra-chain form), that is, a disulfide bond between cysteines at positions 35 and 95 and between cysteines at positions 141 and 199 in each monomer.

The number of amino acids in each monomer may be 221 amino acids, and the number of amino acids forming a homodimer may be a total of 442 amino acids, but is not limited thereto. Specifically, in the immunoglobulin Fc fragment, two monomers having the amino acid sequence (consisting of 221 amino acids) of SEQ ID NO: 129 may form a homodimer through a disulfide bond between cysteines, the 3rd amino acid of each monomer, and the monomers of the homodimer may independently form an internal disulfide bond between cysteines at positions 35 and 95 and an internal disulfide bond between cysteines at positions 141 and 199, but the immunoglobulin Fc fragment is not limited thereto.

F in Chemical Formula 1 may include a monomer having an amino acid sequence of SEQ ID NO: 129, and the F may be a homodimer of a monomer having the amino acid sequence of SEQ ID NO: 129, but is not limited thereto.

As an example, the immunoglobulin Fc region may be a homodimer including an amino acid sequence of SEQ ID NO: 130 (consisting of 442 amino acids), but is not limited thereto.

As a specific example, the immunoglobulin Fc region and X may be not glycosylated, but are not limited thereto.

Meanwhile, in the present invention, the term "combination" means that a polypeptide encoding a single-chain immunoglobulin Fc region of the same origin forms a bond with a single-chain polypeptide of different origin when a dimer or multimer is formed. In other words, it is possible to prepare a dimer or multimer from two or more fragments selected from the group consisting of the Fc fragments of IgG Fc, IgA Fc, IgM Fc, IgD Fc, and IgE.

In the present invention, the term "hybrid" means that there are sequences corresponding to two or more immunoglobulin Fc fragments of different origin within a single-chain immunoglobulin constant region. In the present invention, various types of hybrids are possible. In other words, a hybrid of domains consisting of one to four domains from the group consisting of CH1, CH2, CH3 and CH4 of IgG Fc, IgM Fc, IgA Fc, IgE Fc and IgD Fc is possible, and may include hinges.

Meanwhile, IgG can also be divided into subclasses of IgG1, IgG2, IgG3, and IgG4, and combinations or hybridizations of these are also possible in the present invention. Specifically, this is a subclass of IgG2 and IgG4, and is most specifically, the Fc region of IgG4, which has little effector function such as complement dependent cytotoxicity (CDC).

The conjugate described above may have increased duration of efficacy compared to native interleukin 2 or aldesleukin or compared to X that is not modified with F, and such a conjugate includes, but is not limited to, not only the forms described above but also forms encapsulated in biodegradable nanoparticles.

In Chemical Formula 1, the L may be a polyethylene glycol linker.

In the present invention, the "polyethylene glycol linker" includes a biocompatible polymer in which two or more ethylene glycol repeating units are bound. The repeating units are linked to each other via an arbitrary covalent bond rather than a peptide bond. The polyethylene glycol linker may be a component forming a moiety of the conjugate of the present invention and corresponds to L in Chemical Formula 1. In this specification, the linker may be used interchangeably with "non-peptide linker" or "non-peptide polymer".

In the La, a may be 1 or more, and the respective Ls are independent of each other when a is 2 or more.

In a specific embodiment, in the conjugate, F and X may be covalently linked to each other via a non-peptide linker having a reactive group capable of binding to F (specifically an immunoglobulin Fc region) and a reactive group capable of binding to X (specifically an interleukin 2 analog) at both ends.

Specifically, in the present invention, the non-peptide linker has reactive groups at the ends, and can form a conjugate through reactions with other components constituting the conjugate. In a case where a non-peptide linker having reactive functional groups at both ends binds to X and F of Chemical Formula 1 through the respective reactive groups to form a conjugate, the non-peptide linker or non-peptide polymer may be referred to as a non-peptide polymer linker moiety or a non-peptide linker moiety.

In a specific embodiment, the L (polyethylene glycol linker) may be a linker containing an ethylene glycol repeating unit, for example, polyethylene glycol, but is not limited thereto. In this specification, the term polyethylene glycol encompasses, but is not particularly limited to, all forms of ethylene glycol homopolymer, PEG copolymer, or monomethyl-substituted PEG polymer (mPEG). Derivatives thereof already known in the art and derivatives that can be easily prepared within the level of technology in the art are also included in the scope of the present invention.

The polyethylene glycol linker may include an ethylene glycol repeating unit and may have a functional group that is used in the preparation of a conjugate at the end before being formed into the conjugate. The long-acting conjugate according to the present invention may be in a form in which X and F are linked via the functional group, but is not limited thereto. In the present invention, the non-peptide linker may have two, three, or more functional groups, and the respective functional groups may be the same as or different from each other, but the non-peptide linker is not limited thereto.

Specifically, the linker may be, but is not limited to, polyethylene glycol (PEG) represented by the following Chemical Formula 5: 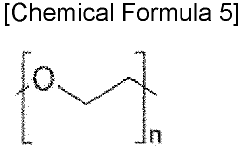 where, n = 10 to 2400, n = 10 to 480, or n = 50 to 250, but is not limited thereto.

In the long-acting conjugate, the PEG moiety may contain, but is not limited to, a -(CH₂CH₂O)ₙ- structure as well as an oxygen atom intervening between the linking element and -(CH₂CH₂O)ₙ-.

As a specific example, the ethylene glycol repeating unit may be represented by, for example, [OCH₂CH₂]ₙ, and the value of n is a natural number and may be determined so that the average molecular weight of the [OCH₂CH₂]ₙ moiety in the interleukin 2 analog conjugate, for example, the number average molecular weight is more than 0 to about 100 kDa, but is not limited thereto. As another example, the value of n is a natural number, and the average molecular weight of the [OCH₂CH₂]ₙ moiety in the interleukin 2 analog conjugate, for example, the number average molecular weight is about 1 to about 100 kDa, about 1 to about 80 kDa, about 1 to about 50 kDa, about 1 to about 30 kDa, about 1 to about 25 kDa, about 1 to about 20 kDa, about 1 to about 15 kDa, about 1 to about 13 kDa, about 1 to about 11 kDa, about 1 to about 10 kDa, about 1 to about 8 kDa, about 1 to about 5 kDa, about 1 to about 3.4 kDa, about 3 to about 30 kDa, about 3 to about 27 kDa, about 3 to about 25 kDa, about 3 to about 22 kDa, about 3 to about 20 kDa, about 3 to about 18 kDa, about 3 to about 16 kDa, about 3 to about 15 kDa, about 3 to about 13 kDa, about 3 to about 11 kDa, about 3 to about 10 kDa, about 3 to about 8 kDa, about 3 to about 5 kDa, about 3 to about 3.4 kDa, about 8 to about 30 kDa, about 8 to about 27 kDa, about 8 to about 25 kDa, about 8 to about 22 kDa, about 8 to about 20 kDa, about 8 to about 18 kDa, about 8 to about 16 kDa, about 8 to about 15 kDa, about 8 to about 13 kDa, about 8 to about 11 kDa, about 8 to about 10 kDa, about 9 to about 15 kDa, about 9 to about 14 kDa, about 9 to about 13 kDa, about 9 to about 12 kDa, about 9 to about 11 kDa, about 9.5 to about 10.5 kDa, or about 10 kDa, but is not limited thereto.

In a specific embodiment, the conjugate may be a structure in which an interleukin 2 analog and an immunoglobulin Fc region (F) are covalently linked via a linker (L) containing an ethylene glycol repeating unit, but is not limited thereto.

In another specific embodiment, in the long-acting conjugate, L may be a linker containing an ethylene glycol repeating unit, a may be 1, and F may be an immunoglobulin Fc region in a dimeric form. More specifically, one molecule of X may be covalently linked to one Fc region in the immunoglobulin Fc region in a dimeric form via the linker containing an ethylene glycol repeating unit, but the long-acting conjugate is not limited thereto. In another specific embodiment, one end of the linker containing an ethylene glycol repeating unit may be linked to only one of the two Fc region chains of the immunoglobulin Fc region in a dimeric form, but the long-acting conjugate is not limited thereto.

The molecular weight of the polyethylene glycol linker that can be used in the present invention is in the range of more than 0 to 200 kDa, specifically, in the range of about 1 to 100 kDa, in the range of about 1 to 50 kDa, in the range of about 1 to 30 kDa, in the range of about 2 to 30 kDa, in the range of about 1 to 20 kDa, more specifically in the range of about 3.4 kDa to 10 kDa, still more specifically about 3.4 kDa, but is not limited thereto. As the non-peptide linker of the present invention, which is bound to the polypeptide corresponding to the F, not only one kind of polymer but also a combination of different kinds of polymers may be used.

In the present invention, the term "about" is a range that includes ±0.5, ±0.4, ±0.3, ±0.2, ±0.1, and the like, and includes all numerical values in a range equal to or similar to the numerical value following the term "about", but is not limited thereto.

Specifically, the non-peptide linker may have reactive groups at both ends in a state of not being bound to F and X, and may bind to F and X via the reactive groups.

As a specific example, both ends of the linker may be bound to a thiol group, an amino group, and a hydroxyl group of the immunoglobulin Fc region and a thiol group, an amino group, an azide group, and a hydroxyl group of an interleukin 2 analog (X), but are not limited thereto.

Specifically, the linker may contain, but is not limited to, a reactive group that can be bound to an immunoglobulin Fc region and an interleukin 2 analog (X) at the two ends, respectively, and specifically a reactive group that can be bound to a thiol group of cysteine in the immunoglobulin Fc region; an amino group located at the N-terminus, lysine, arginine, glutamine and/or histidine; and/or a hydroxyl group located at the C-terminus, and a reactive group that can be bound to a thiol group of cysteine of an interleukin 2 analog (X); an amino group of lysine, arginine, glutamine and/or histidine; an azide group of azidolysine; and/or a hydroxyl group.

More specifically, the reactive group of the linker may be, but is not limited to, one or more selected from the group consisting of an aldehyde group, a maleimide group, and a succinimide derivative.

In the above, examples of the aldehyde group include, but are not limited to, a propionaldehyde group or a butyraldehyde group.

In the above, as the succinimide derivative, succinimidyl valerate, succinimidyl methylbutanoate, succinimidyl methylpropionate, succinimidyl butanoate, succinimidyl propionate, N-hydroxysuccinimide, hydroxysuccinimidyl, succinimidyl carboxymethyl or succinimidyl carbonate may be used, but the succinimide derivative is not limited thereto.

The linker may be converted into a linker moiety by being linked to F, an immunoglobulin Fc region, and X, an interleukin 2 analog, via a reactive group as described above.

The final product produced by reductive alkylation via an aldehyde bond is much more stable than those linked via an amide bond. The aldehyde reactive group reacts selectively at the N-terminus at a low pH, and can form a covalent bond with a lysine residue at a high pH, for example, pH 9.0.

The reactive groups at both ends of the linker may be the same as or different from each other, for example, the linker may have an aldehyde group at both ends, or a maleimide group at one end and an aldehyde group, a propionaldehyde group, or a butyraldehyde group at the other end. However, the linker is not particularly limited thereto, as long as F, specifically an immunoglobulin Fc region, and X can be bound to the ends of the linker, respectively.

For example, the linker may have a maleimide group at one end and an aldehyde group, a propionaldehyde group, a butyraldehyde group or the like at the other end as a reactive group.

In a case of using polyethylene glycol having a hydroxyl reactive group at both ends as a linker, the interleukin 2 analog long-acting conjugate of the present invention can be prepared by activating the hydroxyl group with the various reactive groups by known chemical reactions, or by using commercially available polyethylene glycol having modified reactive groups.

In a specific embodiment, the linker may be linked to a cysteine residue of X, more specifically a -SH group of cysteine, but is not limited thereto.

Specifically, the reactive group of the linker may be linked to the -SH group of the cysteine residue, and all of the above-described contents are applied to the reactive group. In a case of using maleimide-PEG-aldehyde, the maleimide group may be linked to the -SH group of X by a thioether bond, and the aldehyde group may be linked to the -NH₂ group of F, specifically, immunoglobulin Fc, through a reductive amination reaction, but the linker is not limited thereto.

In another specific embodiment, the linker may be linked to a lysine residue of X, more specifically to an amino group of lysine, but is not limited thereto.

In the conjugate, the reactive group of the linker may be linked to -NH₂ located at the N-terminus of the immunoglobulin Fc region, but is not limited thereto.

In the conjugate, the interleukin 2 analog according to the present invention can be linked to the linker having a reactive group via the C-terminus, but this is an example.

In the present invention, the "C-terminus" refers to the carboxyl terminus of a peptide, and for the purpose of the present invention, refers to a location capable of binding to the linker. For example, the C-terminus may include, but is not limited to, not only the most terminal amino acid residue at the C-terminus but also all amino acid residues surrounding the C-terminus, and specifically may include the first to twentieth amino acid residues from the most end, but is not limited thereto.

The above-described conjugate may have increased duration of efficacy compared to X that is not modified with F, and such a conjugate includes not only the forms described above but also forms encapsulated in biodegradable nanoparticles.

As an example, the formulation of the present invention may comprise, but is not limited to, a long-acting conjugate in which an interleukin 2 analogue including any one amino acid sequence selected from the group consisting of amino acid sequences of SEQ ID NOs: 3 to 106 and an immunoglobulin Fc region are linked via a linker:
As another example, the formulation of the present invention may comprise, but is not limited to, a long-acting conjugate of an interleukin 2 analog represented by the following Chemical Formula 2:

   [Chem. 2] X - Z - Fc
where, X is an interleukin 2 analog including any one polypeptide sequence selected from the amino acid sequences of SEQ ID NOs: 3 to 106;
Z is a polyethylene glycol linker having a molecular weight of 2 kDa to 30 kDa;
Fc is an immunoglobulin Fc region in a dimeric form; and
   - indicates linkage between X and Z and between Z and Fc by a covalent bond,
in the long-acting conjugate, one end of Z is covalently linked to only one polypeptide chain in the Fc region in a dimeric form, and one molecule of X is covalently linked to the other end of Z.

The interleukin 2 analog may include, essentially consist of, or consist of, any one sequence selected from the group consisting of amino acid sequences of SEQ ID NOs: 10, 13, 14, 15, 16, 17, 20, 21, 22, 32, 35, 36, 42, 53, 54, 56, 58, 59, 60, 62, 71, 72, 74, 75, 76, 77, 78, 85, 87, 89, 91, 92, 93, 94, 95, 98, 99, 100, 101, 103, 104, 105, and 106, but is not limited thereto.

The interleukin 2 analog may include, essentially consist of, or consist of, any one sequence selected from the group consisting of amino acid sequences of SEQ ID NOs: 10, 13, 14, 16, 17, 20, 21, 22, 32, 35, 36, 42, 53, 54, 87, 89, 91, 92, 93, 94, 98, 99, 100, 101, 103, 104, and 105, but is not limited thereto.

The interleukin 2 analog may include, essentially consist of, or consist of, any one sequence selected from the group consisting of amino acid sequences of SEQ ID NOs: 22, 42, 53, 87, 105, and 106, but is not limited thereto.

The interleukin 2 analog may additionally include one or more amino acids at the C-terminus, but is not limited thereto.

As another example, the formulation of the present invention may comprise, but is not limited to, a long-acting conjugate of an interleukin 2 analog represented by the following Chemical Formula 3:

[Chemical Formula 3] X' - Z - Fc

where, X' is an interleukin 2 analogue including an amino acid sequence represented by the following General Formula 1, [General Formula 1] 
X1-P-T-S-S-S-T-K-K-T-Q-L-Q-L-E-H-L-X18-X19-D-L-X22-M-I-L-N-G-I-N-N-
Y-K-N-P-K-L-T-X38-M-L-T-X42-X43-F-X45-M-P-K-K-A-T-E-L-K-H-L-Q-C-L-E-X61-
E-L-K-P-L-E-X68-V-L-N-L-A-X74-S-K-N-F-H-X80-X81-P-R-X84-X85-X86-S-N-I-N-
X91-X92-V-X94-E-X96-K-G-S-E-T-T-F-M-C-E-Y-A-D-E-T-A-T-I-V-E-F-L-N-R-W-I-
T-F-S-Q-S-I-I-S-T-L-T (General Formula 1, SEQ ID NO: 127)
 in General Formula 1,
X1 is deletion,
X18 is leucine (L), or arginine (R),
X19 is leucine (L), or tyrosine (Y),
X22 is glutamic acid (E), or glutamine (Q),
X38 is alanine (A), aspartic acid (D), or arginine (R),
X42 is alanine (A), phenylalanine (F), lysine (K), or tryptophan (W),
X43 is glutamic acid (E), lysine (K), or glutamine (Q),
X45 is alanine (A), or tyrosine (Y),
X61 is aspartic acid (D), glutamic acid (E), glutamine (Q), or arginine (R),
X68 is aspartic acid (D), or glutamic acid (E),
X74 is histidine (H), or glutamine (Q),
X80 is phenylalanine (F), leucine (L), valine (V), or tyrosine (Y),
X81 is aspartic acid (D), glutamic acid (E), or arginine (R),
X84 is aspartic acid (D), or glutamic acid (E),
X85 is alanine (A), glutamic acid (E), glycine (G), leucine (L), valine (V), tryptophan (W), or tyrosine (Y),
X86 is alanine (A), glycine (G), isoleucine (I), or valine (V),
X91 is threonine (T), or valine (V),
X92 is phenylalanine (F), isoleucine (I), or tyrosine (Y),
X94 is phenylalanine (F) or leucine (L), and
X96 is phenylalanine (F) or leucine (L);
Z is a polyethylene glycol linker having a molecular weight of 2 kDa to 30 kDa;
Fc is an immunoglobulin Fc region in a dimeric form; and
   - indicates linkage between X' and Z and between Z and Fc by a covalent bond,
in the long-acting conjugate, one end of Z is covalently linked to only one polypeptide chain in the Fc region in a dimeric form, and one molecule of X' is covalently linked to the other end of Z.

In General Formula 1, one or more amino acids may be added to threonine (T) corresponding to X133, but the long-acting conjugate is not limited thereto.

Specifically, the interleukin 2 analog may include, essentially consist of, or consist of, any one sequence selected from the group consisting of amino acid sequences of SEQ ID NOs: 10, 13, 14, 15, 16, 17, 20, 21, 22, 32, 35, 36, 42, 53, 54, 56, 58, 59, 60, 62, 71, 72, 74, 75, 76, 77, 78, 85, 87, 89, 91, 92, 93, 94, 95, 98, 99, 100, 101, 103, 104, 105, and 106, but is not limited thereto.

Such an interleukin 2 analog may have increased beta receptor binding affinity compared to aldesleukin or native interleukin 2, but is not limited thereto.

In a specific embodiment, in General Formula 1,
X43 may be lysine (K),
X45 may be tyrosine (Y),
X61 may be aspartic acid (D), glutamic acid (E), or glutamine (Q),
X68 may be glutamic acid (E),
X74 may be glutamine (Q),
X80 may be phenylalanine (F), or leucine (L),
X85 may be leucine (L), valine (V), or tyrosine (Y),
X86 may be isoleucine (I), or valine (V), and
X92 may be phenylalanine (F), or isoleucine (I), but are not limited thereto.

Specifically, the interleukin 2 analog includes any one sequence selected from the group consisting of amino acid sequences of SEQ **ID** NOs: 10, 13, 14, 16, 17, 20, 21, 22, 32, 35, 36, 42, 53, 54, 87, 89, 91, 92, 93, 94, 98, 99, 100, 101, 103, 104, and 105.

The interleukin 2 analog may additionally include one or more amino acids at the C-terminus, but is not limited thereto.

As another example, the formulation of the present invention, another aspect of the present invention may comprise, but is not limited to, a long-acting conjugate of an interleukin 2 analog represented by the following Chemical Formula 4:

[Chemical Formula 4] X" - Z - Fc

where, X" is an interleukin 2 analogue including an amino acid sequence represented by the following General Formula 2: 
[General Formula 2]
X1-P-T-S-S-S-T-K-K-T-Q-L-Q-L-E-H-L-X18-L-D-L-X22-M-I-L-N-G-I-N-N-Y-
K-N-P-K-L-T-X38-M-L-T-X42-K-F-Y-M-P-K-K-A-T-E-L-K-H-L-Q-C-L-E-X61-E-L-K-
P-L-E-X68-V-L-N-L-A-Q-S-K-N-F-H-F-X81-P-R-D-X85-X86-S-N-I-N-V-F-V-L-E-L-
K-G-S-E-T-T-F-M-C-E-Y-A-D-E-T-A-T-I-V-E-F-L-N-R-W-I-T-F-S-Q-S-I-I-S-T-L-T
(General Formula 2, SEQ ID NO: 128)
X1 is deletion,
X18 is leucine (L) or arginine (R),
X22 is glutamic acid (E) or glutamine (Q),
X38 is alanine (A) or arginine (R),
X42 is phenylalanine (F) or lysine (K),
X61 is aspartic acid (D) or glutamic acid (E),
X68 is aspartic acid (D) or glutamic acid (E),
X81 is aspartic acid (D) or glutamic acid (E),
X85 is leucine (L) or valine (V), and
X86 is isoleucine (I) or valine (V);
Z is a polyethylene glycol linker having a molecular weight of 2 kDa to 30 kDa;
Fc is an immunoglobulin Fc region in a dimeric form; and
   - indicates linkage between X" and Z and between Z and Fc by a covalent bond,
in the long-acting conjugate, one end of Z is covalently linked to only one polypeptide chain in the Fc region in a dimeric form, and one molecule of X" is covalently linked to the other end of Z.

Specifically, the interleukin 2 analog may include any one sequence selected from the group consisting of amino acid sequences of SEQ **ID** NOs: 22, 42, 53, 87, 105, and 106, but is not limited thereto.

One or more amino acids may be added to threonine (T) corresponding to X133 in General Formula 2, or the interleukin 2 analog may additionally include one or more amino acids at the C-terminus, but is not limited thereto.

Still another aspect implementing the present invention provides a method for preparing the formulation.

Specifically, the preparation method is a method for preparing a lyophilized formulation, which may comprise lyophilizing an aqueous solution containing a long-acting conjugate including an interleukin 2 analog and a buffer.

The aqueous solution or lyophilized formulation may further comprise one or more ingredients selected from the group consisting of an amino acid, a tonicity agent, sugar, a surfactant, and an antioxidant, but is not limited thereto.

The aqueous solution, lyophilized formulation and the components constituting these are as described above.

Still another aspect implementing the present invention provides a method for reconstructing the lyophilized formulation, which comprises adding a reconstructing solution to the lyophilized formulation.

The aqueous solution, lyophilized formulation and reconstruction are as described above.

In the present invention, the term "reconstructing solution" means a solution added to a lyophilized formulation in a solid state to reconstruct the lyophilized formulation. Examples of such a reconstructing solution include, but are not limited to, distilled water (water).

The reconstructed formulation may comprise a long-acting conjugate at a concentration suitable for administration to a subject, specifically a long-acting conjugate at 100 to 2000 nmol/mL, but is not limited thereto.

The reconstructed formulation may be obtained by reconstructing the lyophilized formulation according to the present invention so as to be suitable for subcutaneous administration to a subject, but is not limited thereto.

Meanwhile, unless the context requires otherwise in this specification, it should be understood that the expressions "includes," "including," "containing," and the like imply the inclusion of a specified integer or group of integers, but not the exclusion of other integers or sets of integers.

Hereinafter, the present invention will be described in more detail through Examples. These Examples are only intended to illustrate the present invention more specifically, and the scope of the present invention is not limited by these Examples.

### Example 1: Construction of native interleukin 2 and interleukin 2 analog expression vectors

In order to construct a native interleukin 2 expression vector encoding 133 amino acids, interleukin 2 synthesized based on the reported interleukin 2 sequence (NM_000586.3; SEQ ID NO: 1) was cloned into the pET-22b vector (Novagen). In addition, an interleukin 2 analog in which amino acids of interleukin 2 were modified using the interleukin 2 as a template was constructed. The interleukin 2 analog of the present invention was prepared by the method disclosed in KR 10-2022-0136285 A or WO 2022-211537 A1, which patent documents are incorporated herein by reference.

Specifically, the PCR conditions for interleukin 2 analog amplification were at 95°C for 30 seconds, at 55°C for 60 seconds, and at 65°C for 6.5 minutes, and this process was repeated 16 times. Sequence analysis was performed on the mutagenesis product obtained under the conditions to determine whether the amino acid at the desired site was normally changed, and it has been verified that each interleukin 2 analog has the mutation presented in Table 1 below based on the native form at the desired mutation location. The expression vectors thus obtained were named pET22b-interleukin 2 analogs 1 to 105.

Table 1 below presents the changed sequence of each amino acid and the analog name. In order to construct these interleukin 2 analogs, forward (F) and reverse (R) primers were synthesized, and then PCR was performed to amplify each analog gene.

Table 1 below summarizes the kinds of interleukin 2 analogs, mutation locations, and their changed sequences, and analog 1 corresponds to aldesleukin.

**[Table 1]**

| Analog | Mutation location and changed sequence |
|---|---|
| 1 | desA1, C125S |
| 2 | desA1, C1258, S87C |
| 3 | desA1, C125S, K32C |
| 4 | desA1, C125S, K35C |
| 5 | desA1, C125S, K43C |
| 6 | desA1, C125S, K48C |
| 7 | desA1, C125S, K49C |
| 8 | desA1, C125S, K76C |
| 9 | desA1, C125S, R38A |
| 10 | desA1, C125S, F42K |
| 11 | desA1, C125S, F42A |
| 12 | desA1, C125S, R38A, F42K |
| 13 | desA1, C125S, R38A, F42A |
| 14 | desA1, C125S, L19Y, R38A, F42K |
| 15 | desA1, C125S, R38A, F42K, D84E |
| 16 | desA1, C125S, R38A, F42K, N88Q |
| 17 | desA1, C125S, R38A, F42K, V91T |
| 18 | desA1, C125S, R38A, F42K, E61Q |
| 19 | desA1, C125S, R38A, F42K, R81D, I92F |
| 20 | desA1, C125S, R38A, F42K, L85V, I86V, I92F |
| 21 | desA1, C125S, R38A, F42K, L80F, R81D, L85V, I86V, I92F |
| 22 | desA1, C125S, L12V, R38A, F42K |
| 23 | desA1, C125S, L12F, R38A, F42K |
| 24 | desA1, C125S, L19V, R38A, F42K |
| 25 | desA1, C125S, L19F, R38A, F42K |
| 26 | desA1, C125S, R38A, F42K, I89F |
| 27 | desA1, C125S, R38A, F42K, V91F |
| 28 | desA1, C125S, R38A, F42K, L94V |
| 29 | desA1, C125S, R38A, F42K, Q126T |
| 30 | desA1, C125S, R38A, R81D, I92F |
| 31 | desA1, C125S, R38A, D84E |
| 32 | desA1, C125S, R38A, R81D, D84E, I92F |
| 33 | desA1, C125S, R38A, L80F |
| 34 | desA1, C125S, R38A, L80F, D84E |
| 35 | desA1, C125S, R38A, L94F, L96F |
| 36 | desA1, C125S, R38A, L94F, L96V |
| 37 | desA1, CC125S, R38A, L94F, L961 |
| 38 | desA1, C125S, R38A, F42K, R81D, I92F, L94F, L96F |
| 39 | desA1, C125S, R38A, F42K, R81D, I92F, L94F, L96V |
| 40 | desA1, C125S, R38A, F42K, R81D, I92F, L94F, L961 |
| 41 | desA1, C125S, L80F, R81D, L85V, I86V, I92F |
| 42 | desA1, C125S, R38A, F42K, R81E, I92F |
| 43 | desA1, C125S, R38A, F42K, R81D, I92L |
| 44 | desA1, C125S, R38A, F42K, R81D, D84V, I92F |
| 45 | desA1, C125S, R38A, F42K, R81D, D84F, I92F |
| 46 | desA1, C125S, D20V, R38A, F42K, R81D, I92F |
| 47 | desA1, C125S, D20F, R38A, F42K, R81D, I92F |
| 48 | desA1, C125S, R38A, F42K, R81D, N88V, I92F |
| 49 | desA1, C125S, R38A, F42K, R81D, N88F, I92F |
| 50 | desA1, C125S, F42K, L80F, R81D, L85V, I86V, I92F |
| 51 | desA1, C125S, E61Q, R81D, I92F |
| 52 | desA1, C125S, R38A, F42K, L80F, R81D, I92F |
| 53 | desA1, C1255, R38A, F42K, L80F, R81D, D84E, I92F |
| 54 | desA1, C125S, R38A, F42K, Q74H, R81D, I92F |
| 55 | desA1, C125S, R38A, F42K, Q74H, L80F, R81D, I92F |
| 56 | desA1, C125S, R38A, F42K, Y45A, Q74H, R81D, I92F |
| 57 | desA1, C1255, R38A, F42K, Y45A, Q74H, L80F, R81D, I92F |
| 58 | desA1, C125S, R38A, F42K, L80F, R81D, L85A, I86A, I92F |
| 59 | desA1, C125S, R38A, F42K, L80F, R81D, L85A, I86A, I92Y |
| 60 | desA1, C125S, R38A, F42K, Y45A, L80Y, L85A, I86A, I92Y |
| 61 | desA1, C125S, R38A, F42K, L80Y, R81D, L85G, I86V, I92Y |
| 62 | desA1, C125S, R38A, F42K, L80W, R81E, L85G, I86A, I92F |
| 63 | desA1, C125S, R38A, F42K, L80D, R81E, L85T, I86G, I92F |
| 64 | desA1, C125S, R38A, F42K, L80Y, R81N, L85V, I86V, I92Y |
| 65 | desA1, C125S, R38A, F42K, L80Y, R81E, L85V, I86V, I92F |
| 66 | desA1, C125S, R38A, F42K, L80F, R81E, L85F, I86V, I92F |
| 67 | desA1, C125S, R38A, F42K, L80Y, R81D, L85F, I86V, I92W, E95D |
| 68 | desA1, C125S, R38A, F42K, L80F, R81E, L85I, I86V, V91E, I92F |
| 69 | desA1, C125S, R38A, F42K, L80Y, R81E, L85F, I86L, V91E, I92W, E95D |
| 70 | desA1, C1255, R38A, F42K, L80Y, R81D, L85V, I86V, I92F |
| 71 | desA1, C125S, R38A, F42K, L80F, R81E, L85V, I86V, I92F |
| 72 | desA1, C125S, R38A, F42K, L80F, R81D, L85V, I86G, 192F |
| 73 | desA1, C125S, R38A, F42K, L80F, R81D, L85W, I86V, I92F |
| 74 | desA1, C125S, R38D, F42K, L80Y, R81D, L85V, I86V, I92F |
| 75 | desA1, C125S, R38A, F42K, Y45A, L80F, R81E, L85V, I86V, I92F |
| 76 | desA1, C1255, R38A, F42K, K43Q, E61R, L80F, R81D, L85V, I86G, I92F |
| 77 | desA1, C125S, R38A, F42K, K43E, E61R, L80F, R81D, L85W, I86V, 192F |
| 78 | desA1, C125S, K35E, R38A, F42K, L80F, R81E, L85V, I86V, I92F |
| 79 | desA1, C125S, K35E, R38A, F42K, Q74H, L80F, R81E, L85V, I86V, I92F |
| 80 | desA1, C125S, K35E, R38A, F42K, Q74H, L80F, R81E, P82G, L85V, I86V, I92F |
| 81 | desA1, C125S, K35E, R38A, F42K, Q74H, L80F, R81E, P82V, L85V, I86V, I92F |
| 82 | desA1, C1255, L18R, Q22E, L80F, R81D, L85E, I86V, I92F |
| 83 | desA1, C125S, L18R, L19R, Q22E, L80F, R81D, L85E, I86V, I92F |
| 84 | desA1, C125S, L18R, Q22E, L80V, R81D, L85E, I86V, I92F |
| 85 | desA1, C125S, L80F, R81E, L85V, I86V, I92F |
| 86 | desA1, C1255, L18R, Q22E, L80F, R81E, L85V, I86V, I92F |
| 87 | desA1, C125S, L18R, L19R, Q22E, L80F, R81E, L85V, I86V, I92F |
| 88 | desA1, C125S, E61D, L80F, R81E, L85V, I86V, I92F |
| 89 | desA1, C125S, R38A, E68Q, L80F, R81E, L85V, I86V, I92F |
| 90 | desA1, C125S, F42W, L80F, R81E, L85V, I86V, I92F |
| 91 | desA1, C125S, E61Q, L80F, R81E, L85V, I86V, I92F |
| 92 | desA1, C125S, L80F, R81E, L85V, I86V, V91T, I92F |
| 93 | desA1, C125S, L80F, R81E, D84E, L85V, I86V, V91T, I92F |
| 94 | desA1, C125S, L80F, R81E, L85V, I86V, I92F, L94F, L96F |
| 95 | desA1, C125S, V69G, L80F, R81E, L85V, I86V, I92F |
| 96 | desA1, C125S, V69G, Q74A, L80F, R81E, L85V, I86V, 192F |
| 97 | desA1, C125S, R38A, L80F, R81D, I92F |
| 98 | desA1, C125S, R38A, L80F, R81E, L85V, I92F |
| 99 | desA1, C125S, R38A, L80F, R81E, I86V, I92F |
| 100 | desA1, C125S, L80F, R81E, L85Y, I86V, I92F |
| 101 | desA1, C125S, L80F, R81E, I86A, I92F |
| 102 | desA1, C125S, L80F, R81E, L85V, I86V |
| 103 | desA1, C1255, L18R, Q22E, R38A, L80F, R81E, L85V, I86V, I92F |
| 104 | desA1, C125S, L18R, Q22E, E61D, L80F, R81E, L85V, I86V, I92F |
| 105 | desA1, C125S, L18R, Q22E, E68D, L80F, R81E, L85V, I86V, I92F |

desA1 means deletion of alanine, the first amino acid of interleukin 2. Table 2 below presents the full-length protein sequences of interleukin 2 analogs. Table 2 below summarizes the interleukin 2 analog sequences, and the bold text in Table 2 indicates the mutation location.

**[Table 2]**

| Analog | Protein sequence | SEQ ID NO: of protein sequence |
|---|---|---|
| 1 | PTSSSTKKT QLQLEHLLLD LQMILNGINN YKNPKLTRML TFKFYMPKKA TELKHLQCLE EELKPLEEVL NLAQSKNFHL RPRDLISNIN VIVLELKGSE TTFMCEYADE TATIVEFLNR WITFSQSIIS TLT | 2 |
| 2 | PTSSSTKKT QLQLEHLLLD LQMILNGINN YKNPKLTRML TFKFYMPKKA TELKHLQCLE EELKPLEEVL NLAQSKNFHL RPRDLICNIN VIVLELKGSE TTFMCEYADE TATIVEFLNR WITFSQSIIS TLT | 3 |
| 3 | PTSSSTKKT QLQLEHLLLD LQMILNGINN YCNPKLTRML TFKFYMPKKA TELKHLQCLE EELKPLEEVL NLAQSKNFHL RPRDLISNIN VIVLELKGSE TTFMCEYADE TATIVEFLNR WITFSQSIIS TLT | 4 |
| 4 | PTSSSTKKT QLQLEHLLLD LQMILNGINN YKNPCLTRML TFKFYMPKKA TELKHLQCLE EELKPLEEVL NLAQSKNFHL RPRDLISNIN VIVLELKGSE TTFMCEYADE TATIVEFLNR WITFSQSIIS TLT | 5 |
| 5 | PTSSSTKKT QLQLEHLLLD LQMILNGINN YKNPKLTRML TFCFYMPKKA TELKHLQCLE EELKPLEEVL NLAQSKNFHL RPRDLISNIN VIVLELKGSE TTFMCEYADE TATIVEFLNR WITFSQSIIS TLT | 6 |
| 6 | PTSSSTKKT QLQLEHLLLD LQMILNGINN YKNPKLTRML TFKFYMPCKA TELKHLQCLE EELKPLEEVL NLAQSKNFHL RPRDLISNIN VIVLELKGSE TTFMCEYADE TATIVEFLNR WITFSQSIIS TLT | 7 |
| 7 | PTSSSTKKT QLQLEHLLLD LQMILNGINN YKNPKLTRML TFKFYMPKCA TELKHLQCLE EELKPLEEVL NLAQSKNFHL RPRDLISNIN VIVLELKGSE TTFMCEYADE TATIVEFLNR WITFSQSIIS TLT | 8 |
| 8 | PTSSSTKKT QLQLEHLLLD LQMILNGINN YKNPKLTRML TFKFYMPKKA TELKHLQCLE EELKPLEEVL NLAQSCNFHL RPRDLISNIN VIVLELKGSE TTFMCEYADE TATIVEFLNR WITFSQSIIS TLT | 9 |
| 9 | PTSSSTKKT QLQLEHLLLD LQMILNGINN YKNPKLTAML TFKFYMPKKA TELKHLQCLE EELKPLEEVL NLAQSKNFHL RPRDLISNIN VIVLELKGSE TTFMCEYADE TATIVEFLNR WITFSQSIIS TLT | 10 |
| 10 | PTSSSTKKT QLQLEHLLLD LQMILNGINN YKNPKLTRML TKKFYMPKKA TELKHLQCLE EELKPLEEVL NLAQSKNFHL RPRDLISNIN VIVLELKGSE TTFMCEYADE TATIVEFLNR WITFSQSIIS TLT | 11 |
| 11 | PTSSSTKKT QLQLEHLLLD LQMILNGINN YKNPKLTRML TAKFYMPKKA TELKHLQCLE EELKPLEEVL NLAQSKNFHL RPRDLISNIN VIVLELKGSE TTFMCEYADE TATIVEFLNR WITFSQSIIS TLT | 12 |
| 12 | PTSSSTKKT QLQLEHLLLD LQMILNGINN YKNPKLTAML TKKFYMPKKA TELKHLQCLE EELKPLEEVL NLAQSKNFHL RPRDLISNIN VIVLELKGSE TTFMCEYADE TATIVEFLNR WITFSQSIIS TLT | 13 |
| 13 | PTSSSTKKT QLQLEHLLLD LQMILNGINN YKNPKLTAML TAKFYMPKKA TELKHLQCLE EELKPLEEVL NLAQSKNFHL RPRDLISNIN VIVLELKGSE TTFMCEYADE TATIVEFLNR WITFSQSIIS TLT | 14 |
| 14 | PTSSSTKKT QLQLEHLLYD LQMILNGINN YKNPKLTAML TKKFYMPKKA TELKHLQCLE EELKPLEEVL NLAQSKNFHL RPRDLISNIN VIVLELKGSE TTFMCEYADE TATIVEFLNR WITFSQSIIS TLT | 15 |
| 15 | PTSSSTKKT QLQLEHLLLD LQMILNGINN YKNPKLTAML TKKFYMPKKA TELKHLQCLE EELKPLEEVL NLAQSKNFHL RPRELISNIN VIVLELKGSE TTFMCEYADE TATIVEFLNR WITFSQSIIS TLT | 16 |
| 16 | PTSSSTKKT QLQLEHLLLD LQMILNGINN YKNPKLTAML TKKFYMPKKA TELKHLQCLE EELKPLEEVL NLAQSKNFHL RPRDLISQIN VIVLELKGSE TTFMCEYADE TATIVEFLNR WITFSQSIIS TLT | 17 |
| 17 | PTSSSTKKT QLQLEHLLLD LQMILNGINN YKNPKLTAML TKKFYMPKKA TELKHLQCLE EELKPLEEVL NLAQSKNFHL RPRDLISNIN TIVLELKGSE TTFMCEYADE TATIVEFLNR WITFSQSIIS TLT | 18 |
| 18 | PTSSSTKKT QLQLEHLLLD LQMILNGINN YKNPKLTAML TKKFYMPKKA TELKHLQCLE QELKPLEEVL NLAQSKNFHL RPRDLISNIN VIVLELKGSE TTFMCEYADE TATIVEFLNR WITFSQSIIS TLT | 19 |
| 19 | PTSSSTKKT QLQLEHLLLD LQMILNGINN YKNPKLTAML TKKFYMPKKA TELKHLQCLE EELKPLEEVL NLAQSKNFHL DPRDLISNIN VFVLELKGSE TTFMCEYADE TATIVEFLNR WITFSQSIIS TLT | 20 |
| 20 | PTSSSTKKT QLQLEHLLLD LQMILNGINN YKNPKLTAML TKKFYMPKKA TELKHLQCLE EELKPLEEVL NLAQSKNFHL RPRDVVSNIN VFVLELKGSE TTFMCEYADE TATIVEFLNR WITFSQSIIS TLT | 21 |
| 21 | PTSSSTKKT QLQLEHLLLD LQMILNGINN YKNPKLTAML TKKFYMPKKA TELKHLQCLE EELKPLEEVL NLAQSKNFHF DPRDVVSNIN VFVLELKGSE TTFMCEYADE TATIVEFLNR WITFSQSIIS TLT | 22 |
| 22 | PTSSSTKKT QVQLEHLLLD LQMILNGINN YKNPKLTAML TKKFYMPKKA TELKHLQCLE EELKPLEEVL NLAQSKNFHL RPRDLISNIN VIVLELKGSE TTFMCEYADE TATIVEFLNR WITFSQSIIS TLT | 23 |
| 23 | PTSSSTKKT QFQLEHLLLD LQMILNGINN YKNPKLTAML TKKFYMPKKA TELKHLQCLE EELKPLEEVL NLAQSKNFHL RPRDLISNIN VIVLELKGSE TTFMCEYADE TATIVEFLNR WITFSQSIIS TLT | 24 |
| 24 | PTSSSTKKT QLQLEHLLVD LQMILNGINN YKNPKLTAML TKKFYMPKKA TELKHLQCLE EELKPLEEVL NLAQSKNFHL RPRDLISNIN VIVLELKGSE TTFMCEYADE TATIVEFLNR WITFSQSIIS TLT | 25 |
| 25 | PTSSSTKKT QLQLEHLLFD LQMILNGINN YKNPKLTAML TKKFYMPKKA TELKHLQCLE EELKPLEEVL NLAQSKNFHL RPRDLISNIN VIVLELKGSE TTFMCEYADE TATIVEFLNR WITFSQSIIS TLT | 26 |
| 26 | PTSSSTKKT QLQLEHLLLD LQMILNGINN YKNPKLTAML TKKFYMPKKA TELKHLQCLE EELKPLEEVL NLAQSKNFHL RPRDLISNFN VIVLELKGSE TTFMCEYADE TATIVEFLNR WITFSQSIIS TLT | 27 |
| 27 | PTSSSTKKT QLQLEHLLLD LQMILNGINN YKNPKLTAML TKKFYMPKKA TELKHLQCLE EELKPLEEVL NLAQSKNFHL RPRDLISNIN FIVLELKGSE TTFMCEYADE TATIVEFLNR WITFSQSIIS TLT | 28 |
| 28 | PTSSSTKKT QLQLEHLLLD LQMILNGINN YKNPKLTAML TKKFYMPKKA TELKHLQCLE EELKPLEEVL NLAQSKNFHL RPRDLISNIN VIVVELKGSE TTFMCEYADE TATIVEFLNR WITFSQSIIS TLT | 29 |
| 29 | PTSSSTKKT QLQLEHLLLD LQMILNGINN YKNPKLTAML TKKFYMPKKA TELKHLQCLE EELKPLEEVL NLAQSKNFHL RPRDLISNIN VIVLELKGSE TTFMCEYADE TATIVEFLNR WITFSTSIIS TLT | 30 |
| 30 | PTSSSTKKT QLQLEHLLLD LQMILNGINN YKNPKLTAML TFKFYMPKKA TELKHLQCLE EELKPLEEVL NLAQSKNFHL DPRDLISNIN VFVLELKGSE TTFMCEYADE TATIVEFLNR WITFSQSIIS TLT | 31 |
| 31 | PTSSSTKKT QLQLEHLLLD LQMILNGINN YKNPKLTAML TFKFYMPKKA TELKHLQCLE EELKPLEEVL NLAQSKNFHL RPRELISNIN VIVLELKGSE TTFMCEYADE TATIVEFLNR WITFSQSIIS TLT | 32 |
| 32 | PTSSSTKKT QLQLEHLLLD LQMILNGINN YKNPKLTAML TFKFYMPKKA TELKHLQCLE EELKPLEEVL NLAQSKNFHL DPRELISNIN VFVLELKGSE TTFMCEYADE TATIVEFLNR WITFSQSIIS TLT | 33 |
| 33 | PTSSSTKKT QLQLEHLLLD LQMILNGINN YKNPKLTAML TFKFYMPKKA TELKHLQCLE EELKPLEEVL NLAQSKNFHF RPRDLISNIN VIVLELKGSE TTFMCEYADE TATIVEFLNR WITFSQSIIS TLT | 34 |
| 34 | PTSSSTKKT QLQLEHLLLD LQMILNGINN YKNPKLTAML TFKFYMPKKA TELKHLQCLE EELKPLEEVL NLAQSKNFHF RPRELISNIN VIVLELKGSE TTFMCEYADE TATIVEFLNR WITFSQSIIS TLT | 35 |
| 35 | PTSSSTKKT QLQLEHLLLD LQMILNGINN YKNPKLTAML TFKFYMPKKA TELKHLQCLE EELKPLEEVL NLAQSKNFHL RPRDLISNIN VIVFEFKGSE TTFMCEYADE TATIVEFLNR WITFSQSIIS TLT | 36 |
| 36 | PTSSSTKKT QLQLEHLLLD LQMILNGINN YKNPKLTAML TFKFYMPKKA TELKHLQCLE EELKPLEEVL NLAQSKNFHL RPRDLISNIN VIVFEVKGSE TTFMCEYADE TATIVEFLNR WITFSQSIIS TLT | 37 |
| 37 | PTSSSTKKT QLQLEHLLLD LQMILNGINN YKNPKLTAML TFKFYMPKKA TELKHLQCLE EELKPLEEVL NLAQSKNFHL RPRDLISNIN VIVFEIKGSE TTFMCEYADE TATIVEFLNR WITFSQSIIS TLT | 38 |
| 38 | PTSSSTKKT QLQLEHLLLD LQMILNGINN YKNPKLTAML TKKFYMPKKA TELKHLQCLE EELKPLEEVL NLAQSKNFHL DPRDLISNIN VFVFEFKGSE TTFMCEYADE TATIVEFLNR WITFSQSIIS TLT | 39 |
| 39 | PTSSSTKKT QLQLEHLLLD LQMILNGINN YKNPKLTAML TKKFYMPKKA TELKHLQCLE EELKPLEEVL NLAQSKNFHL DPRDLISNIN VFVFEVKGSE TTFMCEYADE TATIVEFLNR WITFSQSIIS TLT | 40 |
| 40 | PTSSSTKKT QLQLEHLLLD LQMILNGINN YKNPKLTAML TKKFYMPKKA TELKHLQCLE EELKPLEEVL NLAQSKNFHL DPRDLISNIN VFVFEIKGSE TTFMCEYADE TATIVEFLNR WITFSQSIIS TLT | 41 |
| 41 | PTSSSTKKT QLQLEHLLLD LQMILNGINN YKNPKLTRML TFKFYMPKKA TELKHLQCLE EELKPLEEVL NLAQSKNFHF DPRDVVSNIN VFVLELKGSE TTFMCEYADE TATIVEFLNR WITFSQSIIS TLT | 42 |
| 42 | PTSSSTKKT QLQLEHLLLD LQMILNGINN YKNPKLTAML TKKFYMPKKA TELKHLQCLE EELKPLEEVL NLAQSKNFHL EPRDLISNIN VFVLELKGSE TTFMCEYADE TATIVEFLNR WITFSQSIIS TLT | 43 |
| 43 | PTSSSTKKT QLQLEHLLLD LQMILNGINN YKNPKLTAML TKKFYMPKKA TELKHLQCLE EELKPLEEVL NLAQSKNFHL DPRDLISNIN VLVLELKGSE TTFMCEYADE TATIVEFLNR WITFSQSIIS TLT | 44 |
| 44 | PTSSSTKKT QLQLEHLLLD LQMILNGINN YKNPKLTAML TKKFYMPKKA TELKHLQCLE EELKPLEEVL NLAQSKNFHL DPRVLISNIN VFVLELKGSE TTFMCEYADE TATIVEFLNR WITFSQSIIS TLT | 45 |
| 45 | PTSSSTKKT QLQLEHLLLD LQMILNGINN YKNPKLTAML TKKFYMPKKA TELKHLQCLE EELKPLEEVL NLAQSKNFHL DPRFLISNIN VFVLELKGSE TTFMCEYADE TATIVEFLNR WITFSQSIIS TLT | 46 |
| 46 | PTSSSTKKT QLQLEHLLLV LQMILNGINN YKNPKLTAML TKKFYMPKKA TELKHLQCLE EELKPLEEVL NLAQSKNFHL DPRDLISNIN VFVLELKGSE TTFMCEYADE TATIVEFLNR WITFSQSIIS TLT | 47 |
| 47 | PTSSSTKKT QLQLEHLLLF LQMILNGINN YKNPKLTAML TKKFYMPKKA TELKHLQCLE EELKPLEEVL NLAQSKNFHL DPRDLISNIN VFVLELKGSE TTFMCEYADE TATIVEFLNR WITFSQSIIS TLT | 48 |
| 48 | PTSSSTKKT QLQLEHLLLD LQMILNGINN YKNPKLTAML TKKFYMPKKA TELKHLQCLE EELKPLEEVL NLAQSKNFHL DPRDLISVIN VFVLELKGSE TTFMCEYADE TATIVEFLNR WITFSQSIIS TLT | 49 |
| 49 | PTSSSTKKT QLQLEHLLLD LQMILNGINN YKNPKLTAML TKKFYMPKKA TELKHLQCLE EELKPLEEVL NLAQSKNFHL DPRDLISFIN VFVLELKGSE TTFMCEYADE TATIVEFLNR WITFSQSIIS TLT | 50 |
| 50 | PTSSSTKKT QLQLEHLLLD LQMILNGINN YKNPKLTRML TKKFYMPKKA TELKHLQCLE EELKPLEEVL NLAQSKNFHF DPRDVVSNIN VFVLELKGSE TTFMCEYADE TATIVEFLNR WITFSQSIIS TLT | 51 |
| 51 | PTSSSTKKT QLQLEHLLLD LQMILNGINN YKNPKLTRML TFKFYMPKKA TELKHLQCLE QELKPLEEVL NLAQSKNFHL DPRDLISNIN VFVLELKGSE TTFMCEYADE TATIVEFLNR WITFSQSIIS TLT | 52 |
| 52 | PTSSSTKKT QLQLEHLLLD LQMILNGINN YKNPKLTAML TKKFYMPKKA TELKHLQCLE EELKPLEEVL NLAQSKNFHF DPRDLISNIN VFVLELKGSE TTFMCEYADE TATIVEFLNR WITFSQSIIS TLT | 53 |
| 53 | PTSSSTKKT QLQLEHLLLD LQMILNGINN YKNPKLTAML TKKFYMPKKA TELKHLQCLE EELKPLEEVL NLAQSKNFHF DPRELISNIN VFVLELKGSE TTFMCEYADE TATIVEFLNR WITFSQSIIS TLT | 54 |
| 54 | PTSSSTKKT QLQLEHLLLD LQMILNGINN YKNPKLTAML TKKFYMPKKA TELKHLQCLE EELKPLEEVL NLAHSKNFHL DPRDLISNIN VFVLELKGSE TTFMCEYADE TATIVEFLNR WITFSQSIIS TLT | 55 |
| 55 | PTSSSTKKT QLQLEHLLLD LQMILNGINN YKNPKLTAML TKKFYMPKKA TELKHLQCLE EELKPLEEVL NLAHSKNFHF DPRDLISNIN VFVLELKGSE TTFMCEYADE TATIVEFLNR WITFSQSIIS TLT | 56 |
| 56 | PTSSSTKKT QLQLEHLLLD LQMILNGINN YKNPKLTAML TKKFAMPKKA TELKHLQCLE EELKPLEEVL NLAHSKNFHL DPRDLISNIN VFVLELKGSE TTFMCEYADE TATIVEFLNR WITFSQSIIS TLT | 57 |
| 57 | PTSSSTKKT QLQLEHLLLD LQMILNGINN YKNPKLTAML TKKFAMPKKA TELKHLQCLE EELKPLEEVL NLAHSKNFHF DPRDLISNIN VFVLELKGSE TTFMCEYADE TATIVEFLNR WITFSQSIIS TLT | 58 |
| 58 | PTSSSTKKT QLQLEHLLLD LQMILNGINN YKNPKLTAML TKKFYMPKKA TELKHLQCLE EELKPLEEVL NLAQSKNFHF DPRDAASNIN VFVLELKGSE TTFMCEYADE TATIVEFLNR WITFSQSIIS TLT | 59 |
| 59 | PTSSSTKKT QLQLEHLLLD LQMILNGINN YKNPKLTAML TKKFYMPKKA TELKHLQCLE EELKPLEEVL NLAQSKNFHF DPRDAASNIN VYVLELKGSE TTFMCEYADE TATIVEFLNR WITFSQSIIS TLT | 60 |
| 60 | PTSSSTKKT QLQLEHLLLD LQMILNGINN YKNPKLTAML TKKFAMPKKA TELKHLQCLE EELKPLEEVL NLAQSKNFHY RPRDAASNIN VYVLELKGSE TTFMCEYADE TATIVEFLNR WITFSQSIIS TLT | 61 |
| 61 | PTSSSTKKT QLQLEHLLLD LQMILNGINN YKNPKLTAML TKKFYMPKKA TELKHLQCLE EELKPLEEVL NLAQSKNFHY DPRDGVSNIN VYVLELKGSE TTFMCEYADE TATIVEFLNR WITFSQSIIS TLT | 62 |
| 62 | PTSSSSTKKT QLQLEHLLLD LQMILNGINN YKNPKLTAML TKKFYMPKKA TELKHLQCLE EELKPLEEVL NLAQSKNFHW EPRDGASNIN VFVLELKGSE TTFMCEYADE TATIVEFLNR WITFSQSIIS TLT | 63 |
| 63 | PTSSSTKKT QLQLEHLLLD LQMILNGINN YKNPKLTAML TKKFYMPKKA TELKHLQCLE EELKPLEEVL NLAQSKNFHD EPRDTGSNIN VFVLELKGSE TTFMCEYADE TATIVEFLNR WITFSQSIIS TLT | 64 |
| 64 | PTSSSTKKT QLQLEHLLLD LQMILNGINN YKNPKLTAML TKKFYMPKKA TELKHLQCLE EELKPLEEVL NLAQSKNFHY NPRDVVSNIN VYVLELKGSE TTFMCEYADE TATIVEFLNR WITFSQSIIS TLT | 65 |
| 65 | PTSSSTKKT QLQLEHLLLD LQMILNGINN YKNPKLTAML TKKFYMPKKA TELKHLQCLE EELKPLEEVL NLAQSKNFHY EPRDVVSNIN VFVLELKGSE TTFMCEYADE TATIVEFLNR WITFSQSIIS TLT | 66 |
| 66 | PTSSSTKKT QLQLEHLLLD LQMILNGINN YKNPKLTAML TKKFYMPKKA TELKHLQCLE EELKPLEEVL NLAQSKNFHF EPRDFVSNIN VFVLELKGSE TTFMCEYADE TATIVEFLNR WITFSQSIIS TLT | 67 |
| 67 | PTSSSTKKT QLQLEHLLLD LQMILNGINN YKNPKLTAML TKKFYMPKKA TELKHLQCLE EELKPLEEVL NLAQSKNFHY DPRDFVSNIN VWVLDLKGSE TTFMCEYADE TATIVEFLNR WITFSQSIIS TLT | 68 |
| 68 | PTSSSTKKT QLQLEHLLLD LQMILNGINN YKNPKLTAML TKKFYMPKKA TELKHLQCLE EELKPLEEVL NLAQSKNFHF EPRDIVSNIN EFVLELKGSE TTFMCEYADE TATIVEFLNR WITFSQSIIS TLT | 69 |
| 69 | PTSSSTKKT QLQLEHLLLD LQMILNGINN YKNPKLTAML TKKFYMPKKA TELKHLQCLE EELKPLEEVL NLAQSKNFHY EPRDFLSNIN EWVLDLKGSE TTFMCEYADE TATIVEFLNR WITFSQSIIS TLT | 70 |
| 70 | PTSSSTKKT QLQLEHLLLD LQMILNGINN YKNPKLTAML TKKFYMPKKA TELKHLQCLE EELKPLEEVL NLAQSKNFHY DPRDVVSNIN VFVLELKGSE TTFMCEYADE TATIVEFLNR WITFSQSIIS TLT | 71 |
| 71 | PTSSSTKKT QLQLEHLLLD LQMILNGINN YKNPKLTAML TKKFYMPKKA TELKHLQCLE EELKPLEEVL NLAQSKNFHF EPRDVVSNIN VFVLELKGSE TTFMCEYADE TATIVEFLNR WITFSQSIIS TLT | 72 |
| 72 | PTSSSTKKT QLQLEHLLLD LQMILNGINN YKNPKLTAML TKKFYMPKKA TELKHLQCLE EELKPLEEVL NLAQSKNFHF DPRDVGSNIN VFVLELKGSE TTFMCEYADE TATIVEFLNR WITFSQSIIS TLT | 73 |
| 73 | PTSSSTKKT QLQLEHLLLD LQMILNGINN YKNPKLTAML TKKFYMPKKA TELKHLQCLE EELKPLEEVL NLAQSKNFHF DPRDWVSNIN VFVLELKGSE TTFMCEYADE TATIVEFLNR WITFSQSIIS TLT | 74 |
| 74 | PTSSSTKKT QLQLEHLLLD LQMILNGINN YKNPKLTDML TKKFYMPKKA TELKHLQCLE EELKPLEEVL NLAQSKNFHY DPRDVVSNIN VFVLELKGSE TTFMCEYADE TATIVEFLNR WITFSQSIIS TLT | 75 |
| 75 | PTSSSTKKT QLQLEHLLLD LQMILNGINN YKNPKLTAML TKKFAMPKKA TELKHLQCLE EELKPLEEVL NLAQSKNFHF EPRDVVSNIN VFVLELKGSE TTFMCEYADE TATIVEFLNR WITFSQSIIS TLT | 76 |
| 76 | PTSSSTKKT QLQLEHLLLD LQMILNGINN YKNPKLTAML TKQFYMPKKA TELKHLQCLE RELKPLEEVL NLAQSKNFHF DPRDVGSNIN VFVLELKGSE TTFMCEYADE TATIVEFLNR WITFSQSIIS TLT | 77 |
| 77 | PTSSSTKKT QLQLEHLLLD LQMILNGINN YKNPKLTAML TKEFYMPKKA; TELKHLQCLE RELKPLEEVL NLAQSKNFHF DPRDWVSNIN VFVLELKGSE TTFMCEYADE TATIVEFLNR WITFSQSIIS TLT | 78 |
| 78 | PTSSSTKKT QLQLEHLLLD LQMILNGINN YKNPELTAML TKKFYMPKKA TELKHLQCLE EELKPLEEVL NLAQSKNFHF EPRDVVSNIN VFVLELKGSE TTFMCEYADE TATIVEFLNR WITFSQSIIS TLT | 79 |
| 79 | PTSSSTKKT QLQLEHLLLD LQMILNGINN YKNPELTAML TKKFYMPKKA TELKHLQCLE EELKPLEEVL NLAHSKNFHF EPRDVVSNIN VFVLELKGSE TTFMCEYADE TATIVEFLNR WITFSQSIIS TLT | 80 |
| 80 | PTSSSTKKT QLQLEHLLLD LQMILNGINN YKNPELTAML TKKFYMPKKA TELKHLQCLE EELKPLEEVL NLAHSKNFHF EGRDVVSNIN VFVLELKGSE TTFMCEYADE TATIVEFLNR WITFSQSIIS TLT | 81 |
| 81 | PTSSSTKKT QLQLEHLLLD LQMILNGINN YKNPELTAML TKKFYMPKKA TELKHLQCLE EELKPLEEVL NLAHSKNFHF EVRDVVSNIN VFVLELKGSE TTFMCEYADE TATIVEFLNR WITFSQSIIS TLT | 82 |
| 82 | PTSSSTKKT QLQLEHLRLD LEMILNGINN YKNPKLTRML TFKFYMPKKA TELKHLQCLE EELKPLEEVL NLAQSKNFHF DPRDEVSNIN VFVLELKGSE TTFMCEYADE TATIVEFLNR WITFSQSIIS TLT | 83 |
| 83 | PTSSSTKKT QLQLEHLRRD LEMILNGINN YKNPKLTRML TFKFYMPKKA TELKHLQCLE EELKPLEEVL NLAQSKNFHF DPRDEVSNIN VFVLELKGSE TTFMCEYADE TATIVEFLNR WITFSQSIIS TLT | 84 |
| 84 | PTSSSTKKT QLQLEHLRLD LEMILNGINN YKNPKLTRML TFKFYMPKKA TELKHLQCLE EELKPLEEVL NLAQSKNFHV DPRDEVSNIN VFVLELKGSE TTFMCEYADE TATIVEFLNR WITFSQSIIS TLT | 85 |
| 85 | PTSSSTKKT QLQLEHLLLD LQMILNGINN YKNPKLTRML TFKFYMPKKA TELKHLQCLE EELKPLEEVL NLAQSKNFHF EPRDVVSNIN VFVLELKGSE TTFMCEYADE TATIVEFLNR WITFSQSIIS TLT | 86 |
| 86 | PTSSSTKKT QLQLEHLRLD LEMILNGINN YKNPKLTRML TFKFYMPKKA TELKHLQCLE EELKPLEEVL NLAQSKNFHF EPRDVVSNIN VFVLELKGSE TTFMCEYADE TATIVEFLNR WITFSQSIIS TLT | 87 |
| 87 | PTSSSTKKT QLQLEHLRRD LEMILNGINN YKNPKLTRML TFKFYMPKKA TELKHLQCLE EELKPLEEVL NLAQSKNFHF EPRDVVSNIN VFVLELKGSE TTFMCEYADE TATIVEFLNR WITFSQSIIS TLT | 88 |
| 88 | PTSSSTKKT QLQLEHLLLD LQMILNGINN YKNPKLTRML TFKFYMPKKA TELKHLQCLE DELKPLEEVL NLAQSKNFHF EPRDVVSNIN VFVLELKGSE TTFMCEYADE TATIVEFLNR WITFSQSIIS TLT | 89 |
| 89 | PTSSSTKKT QLQLEHLLLD LQMILNGINN YKNPKLTAML TFKFYMPKKA TELKHLQCLE EELKPLEQVL NLAQSKNFHF EPRDVVSNIN VFVLELKGSE TTFMCEYADE TATIVEFLNR WITFSQSIIS TLT | 90 |
| 90 | PTSSSTKKT QLQLEHLLLD LQMILNGINN YKNPKLTRML TWKFYMPKKA TELKHLQCLE EELKPLEEVL NLAQSKNFHF EPRDVVSNIN VFVLELKGSE TTFMCEYADE TATIVEFLNR WITFSQSIIS TLT | 91 |
| 91 | PTSSSTKKT QLQLEHLLLD LQMILNGINN YKNPKLTRML TFKFYMPKKA TELKHLQCLE QELKPLEEVL NLAQSKNFHF EPRDVVSNIN VFVLELKGSE TTFMCEYADE TATIVEFLNR WITFSQSIIS TLT | 92 |
| 92 | PTSSSTKKT QLQLEHLLLD LQMILNGINN YKNPKLTRML TFKFYMPKKA TELKHLQCLE EELKPLEEVL NLAQSKNFHF EPRDVVSNIN TFVLELKGSE TTFMCEYADE TATIVEFLNR WITFSQSIIS TLT | 93 |
| 93 | PTSSSTKKT QLQLEHLLLD LQMILNGINN YKNPKLTRML TFKFYMPKKA TELKHLQCLE EELKPLEEVL NLAQSKNFHF EPREVVSNIN TFVLELKGSE TTFMCEYADE TATIVEFLNR WITFSQSIIS TLT | 94 |
| 94 | PTSSSTKKT QLQLEHLLLD LQMILNGINN YKNPKLTRML TFKFYMPKKA TELKHLQCLE EELKPLEEVL NLAQSKNFHF EPRDVVSNIN VFVFEFKGSE TTFMCEYADE TATIVEFLNR WITFSQSIIS TLT | 95 |
| 95 | PTSSSTKKT QLQLEHLLLD LQMILNGINN YKNPKLTRML TFKFYMPKKA TELKHLQCLE EELKPLEEGL NLAQSKNFHF EPRDVVSNIN VFVLELKGSE TTFMCEYADE TATIVEFLNR WITFSQSIIS TLT | 96 |
| 96 | PTSSSTKKT QLQLEHLLLD LQMILNGINN YKNPKLTRML TFKFYMPKKA TELKHLQCLE EELKPLEEGL NLAASKNFHF EPRDVVSNIN VFVLELKGSE TTFMCEYADE TATIVEFLNR WITFSQSIIS TLT | 97 |
| 97 | PTSSSTKKT QLQLEHLLLD LQMILNGINN YKNPKLTAML TFKFYMPKKA TELKHLQCLE EELKPLEEVL NLAQSKNFHF DPRDLISNIN VFVLELKGSE TTFMCEYADE TATIVEFLNR WITFSQSIIS TLT | 98 |
| 98 | PTSSSTKKT QLQLEHLLLD LQMILNGINN YKNPKLTAML TFKFYMPKKA TELKHLQCLE EELKPLEEVL NLAQSKNFHF EPRDVISNIN VFVLELKGSE TTFMCEYADE TATIVEFLNR WITFSQSIIS TLT | 99 |
| 99 | PTSSSTKKT QLQLEHLLLD LQMILNGINN YKNPKLTAML TFKFYMPKKA TELKHLQCLE EELKPLEEVL NLAQSKNFHF EPRDLVSNIN VFVLELKGSE TTFMCEYADE TATIVEFLNR WITFSQSIIS TLT | 100 |
| 100 | PTSSSTKKT QLQLEHLLLD LQMILNGINN YKNPKLTRML TFKFYMPKKA TELKHLQCLE EELKPLEEVL NLAQSKNFHF EPRDYVSNIN VFVLELKGSE TTFMCEYADE TATIVEFLNR WITFSQSIIS TLT | 101 |
| 101 | PTSSSTKKT QLQLEHLLLD LQMILNGINN YKNPKLTRML TFKFYMPKKA TELKHLQCLE EELKPLEEVL NLAQSKNFHF EPRDLASNIN VFVLELKGSE TTFMCEYADE TATIVEFLNR WITFSQSIIS TLT | 102 |
| 102 | PTSSSTKKT QLQLEHLLLD LQMILNGINN YKNPKLTRML TFKFYMPKKA TELKHLQCLE EELKPLEEVL NLAQSKNFHF EPRDVVSNIN VIVLELKGSE TTFMCEYADE TATIVEFLNR WITFSQSIIS TLT | 103 |
| 103 | PTSSSTKKT QLQLEHLRLD LEMILNGINN YKNPKLTAML TFKFYMPKKA TELKHLQCLE EELKPLEEVL NLAQSKNFHF EPRDVVSNIN VFVLELKGSE TTFMCEYADE TATIVEFLNR WITFSQSIIS TLT | 104 |
| 104 | PTSSSTKKT QLQLEHLRLD LEMILNGINN YKNPKLTRML TFKFYMPKKA TELKHLQCLE DELKPLEEVL NLAQSKNFHF EPRDVVSNIN VFVLELKGSE TTFMCEYADE TATIVEFLNR WITFSQSIIS TLT | 105 |
| 105 | PTSSSTKKT QLQLEHLRLD LEMILNGINN YKNPKLTRML TFKFYMPKKA TELKHLQCLE EELKPLEDVL NLAQSKNFHF EPRDVVSNIN VFVLELKGSE TTFMCEYADE TATIVEFLNR WITFSQSIIS TLT | 106 |

### Example 2: Expression of interleukin 2 analogues

Using the expression vector prepared in Example 1, a recombinant interleukin 2 analog was expressed under the control of the T7 promoter. The expression E. *coli* strain, *E. coli* BL21DE3 (E. *coli B F-dcm ompT* hsdS(rB-mB-) *gal* λ(DE3); Novagen) was transformed with each recombinant interleukin 2 analog expression vector. As the transformation method, the method recommended by Novagen was used. Each single colony transformed with each recombinant expression vector was taken, inoculated into 2X Luria Broth medium containing ampicillin (50 µg/mL), and cultured at 37°C for 15 hours. The recombinant strain culture solution and 2X LB medium containing 30% glycerol were mixed at a ratio of 1 : 1 (v/v), and 1 mL each was dispensed into a cryo-tube and stored at -150°C. This was used as a cell stock for the production of recombinant proteins.

For expression of recombinant interleukin 2 analogs, 1 vial of each cell stock was thawed, inoculated into 500 mL of 2X LB, and cultured with shaking at 37°C for 14 to 16 hours. When the absorbance value at 600 nm was 4.0 or more, the culture was terminated and used as the seed culture solution. Using a 5 L fermenter (Bioflo-320, NBS, USA), the seed culture solution was inoculated into 1.6 L of fermentation medium, and initial fermentation was started. The culture conditions were a temperature of 37°C, an air volume of 2.0 L/min (1 vvm), and a stirring speed of 650 rpm, and the pH was maintained at 6.70 using 30% ammonia water. For fermentation, a feeding solution was added when nutrients in the culture solution were limited, and fed-batch culture was performed. The growth of the strain was monitored by the OD value, and IPTG was introduced at a final concentration of 500 µM when the absorbance value was 70 or more. The culture was further performed for about 23 to 25 hours after the introduction of IPTG, and after the completion of culture, the recombinant strain was harvested using a centrifuge and stored at -80°C until use.

### Example 3: Extraction and refolding of interleukin 2 analogues

In order to convert the interleukin 2 analog from the interleukin 2 analog expressing E. *coli* obtained in Example 2 into a soluble form, the cells were disrupted and refolded. The cell pellet corresponding to 100 mL of culture solution was suspended in 1 to 200 mL of lysis buffer (20 mM Tris-HCl pH 9.0, 1 mM EDTA pH 9.0, 0.2 M NaCl, 0.5% Triton X-100), and then the recombinant E. *coli* was disrupted at 15,000 psi using a microfluidizer. Centrifugation was performed at 13,900 g for 30 minutes, the supernatant was discarded, and the pellet was washed with 400 mL of a first wash buffer (50 mM Tris-HCl pH 8.0, 5 mM EDTA pH 9.0). Centrifugation was performed under the same conditions as above, the supernatant was discarded, and the pellet was washed with 400 mL of a second wash buffer (50 mM Tris-HCl pH 8.0, 5 mM EDTA pH 9.0, 2% Triton X-100). Centrifugation was performed under the same conditions as above, the supernatant was discarded, and the pellet was washed with 400 mL of a third wash buffer (50 mM Tris-HCl pH 8.0, 5 mM EDTA pH 9.0, 1% sodium deoxycholate). Centrifugation was performed under the same conditions as above, the supernatant was discarded, and the pellet was washed with 400 mL of a fourth wash buffer (50 mM Tris-HCl pH 8.0, 5 mM EDTA pH 9.0, 1 M NaCl). A washed *E*. *coli* inclusion body pellet was obtained through centrifugation under the same conditions as above. The washed inclusion body pellet was resuspended in 400 mL of solubilizing/reducing buffer (6 M Guanidine, 100 mM Tris pH 8.0, 2 mM EDTA pH 9.0, 50 mM DTT) and stirred at 50°C for 30 minutes. By adding 100 mL of distilled water to the solubilized/reduced interleukin 2 analog, 6 M guanidine was diluted to 4.8 M guanidine, centrifugation was performed at 13,900 g for 30 minutes, and the pellet was discarded to obtain only the solution. By additionally adding 185.7 mL of distilled water to the diluted solution, 4.8 M guanidine was diluted to 3.5 M guanidine, and then the pH was adjusted to 5.0 using 100% acetic acid. The pH-adjusted solution was stirred at room temperature for 1 hour. The solution in which impurities had precipitated was centrifuged at 13,900 g for 30 minutes, the supernatant was discarded, and the pellet was washed with the final wash buffer (3.5 M Guanidine, 20 mM Sodium Acetate pH 5.0, 5 mM DTT). A pellet was obtained through centrifugation under the same conditions as above. The washed interleukin 2 analog was dissolved in 400 mL of refolding buffer (6 mM Guanidine, 100 mM Tris pH 8.0, 0.1 mM CuCl2). The refolding process was performed by stirring the mixed solution at 4°C for 15 to 24 hours.

### Example 4: Size exclusion column chromatography

The interleukin 2 analog refolding solution obtained in Example 3 was concentrated to 1 mL or less to be applied to a size exclusion column for purification. The column was equilibrated with buffer (2 M Guanidine, 100 mM Tris pH 8.0) before the introduction of the refolding solution, and elution was performed by flowing the buffer after the introduction of the refolding solution. The eluted sample contained guanidine, and was replaced with a stabilizing solution (10 mM Sodium Acetate pH 4.5, 5% Trehalose), and its purity was measured through RP-HPLC and peptide mapping analysis. When the measured purity was 80% or more, the sample was used in the experiment.

### Example 5: Linking reaction of interleukin 2 analog with polyethylene glycol (3.4K PEG) linker and purification of interleukin 2 analog linked body

In order to prepare a long-acting conjugate in which the interleukin 2 analog obtained in Example 4 is bound to an immunoglobulin Fc region, a linked body in which the interleukin 2 analog is linked to one end of a polyethylene glycol (PEG) linker was first prepared. For the preparation of the linked body, the interleukin 2 analogue 86 was used, and polyethylene glycol (ALD(2)3.4K PEG from NOF, Japan) having a molecular weight of 3.4 kDa and hydroxyl hydrogen modified with a propylaldehyde group at both ends was used as the PEG linker, and this was linked to the N-terminus of the interleukin 2 analog. The molar ratio of interleukin 2 analog : PEG linker was set to 1 : 15 to 1 : 20, and the concentration of interleukin 2 analog was set to 1 mg/mL or less, and the reaction was conducted at 2°C to 10°C for 1 hour. At this time, the reaction was conducted under 100 mM potassium phosphate (pH 5.5), and 20 mM sodium cyanoborohydride (SCB) was added as a reducing agent. The reaction solution was changed with 20 mM triethylamine (pH 8.0) buffer using a desalting column, and then purified using a Fractogel^{®} EMD TMAE (S) (Merck Millipore) or Source 15Q (Cytiva) column using a gradient of triethylamine (pH 8.0) and sodium chloride concentration to obtain an interleukin 2 analog-3.4K PEG linked body.

### Example 6: Preparation of interleukin 2 analog-3.4K PEG-immunoglobulin Fc region long-acting conjugate

In order to prepare an interleukin 2 analog-3.4K PEG-immunoglobulin Fc region long-acting conjugate, the molar ratio of the interleukin 2 analog-3.4K PEG linked body obtained using the method of Example 5 and the immunoglobulin Fc region (SEQ ID NO: 129) was set to 1 : 10, the total protein concentration was set to 30 mg/mL, and the reaction was conducted at 2°C to 10°C for 15 to 16 hours. At this time, the reaction solution was 100 mM potassium phosphate (pH 6.0), and 20 mM sodium cyanoborohydride was added as a reducing agent.

In the immunoglobulin region used at this time, two monomers having the amino acid sequence of SEQ ID NO: 129 (consisting of 221 amino acids) form a homodimer through a disulfide bond between cysteine, the 3rd amino acid of each monomer, and the monomers of the homodimer independently form an internal disulfide bond between cysteines at positions 35 and 95 and an internal disulfide bond between cysteines at positions 141 and 199. Table 3 below summarizes the immunoglobulin Fc protein sequence.

**[Table 3]**

| Name | Protein sequence | SEQ ID NO: |
|---|---|---|
| Immunoglobulin Fc | PSCPAPEFLG GPSVFLFPPK PKDTLMISRT PEVTCVVVDV SQEDPEVQFN WYVDGVEVHN AKTKPREEQF NSTYRVVSVL TVLHQDWLNG KEYKCKVSNK GLPSSIEKTI SKAKGQPREP QVYTLPPSQE EMTKNQVSLT CLVKGFYPSD IAVEWESNGQ PENNYKTTPP VLDSDGSFFL YSRLTVDKSR WQEGNVFSCS VMHEALHNHY TQKSLSLSLG K | 129 |

After the reaction was completed, the unreacted immunoglobulin Fc region was removed from Butyl FF (Cytiva) using Bis-Tris (pH 6.5) and sodium chloride, and the reaction solution was purified with Source 15ISO (Cytiva) using sodium citrate buffer (pH 5.5) and ammonium sulfate, thereby obtaining an interleukin 2 analog-3.4 kDa PEG-immunoglobulin Fc region conjugate (long-acting conjugate) in which the N-terminus of the interleukin 2 analog was linked to one end of the 3.4 kDa PEG linker and the other end of the 3.4 kDa PEG linker was linked to the nitrogen of the N-terminal proline of the Fc region.

### Example 7: Evaluation of stability of liquid formulation containing interleukin 2 analog long-acting conjugate depending on pH, kind of surfactant, kind of sugar or sugar alcohol, and kind and concentration of amino acid

A liquid formulation containing the long-acting conjugate of interleukin 2 analog 86 (SEQ ID NO: 87) (hereinafter, "interleukin 2 analog 86 conjugate") prepared in the Examples above was prepared, and its stability was examined.

Specifically, the stability of interleukin 2 analog 86 conjugates depending on the pH, kind and concentration of sugar alcohol or sugar (mannitol or sucrose), kind of surfactant (polysorbate 20 or sodium lauryl sulfate), and the kind and concentration of amino acid (serine, alanine, arginine or glutamic acid) was compared based on liquid formulations containing histidine as a buffer and methionine as an antioxidant.

Using liquid formulations of interleukin 2 analog 86 conjugates (concentration of long-acting conjugate in liquid formulation: about 76.7 to 2515.8 nmol/mL) having the compositions as presented in Table 4 below, the analysis was performed by size exclusion chromatography (SE-HPLC). Candidate liquid formulations No. 2-1 to 2-10 in Table 4 were stored at 25±5°C for 1 week, and then the accelerated storage stability of the samples was examined by SE-HPLC.

SE-HPLC (%) in Table 5 presents the chromatography result value for each liquid formulation disclosed in Table 4 denoted by %area with respect to the main peak.

In the Examples in this application, the 'initial stage' of the experimental period refers to the time when the experiment was conducted, that is, day 0.

**[Table 4]**

| No. | Buffering substance | pH | Sugar alcohol or sugar (w/v) | Amino acid (wv) | Surfactant (w/v) | Antioxidant | Others |
|---|---|---|---|---|---|---|---|
| 1-1 | 20 mM histidine | 6.5 | 4% mannitol | 2% serine | 0.015% sodium lauryl sulfate | 0.1 mg/mL methionine | - |
| 1-2 | 20 mM histidine | 6.5 | 4% mannitol | 2% alanine | 0.015% sodium lauryl sulfate | 0.1 mg/mL methionine | - |
| 1-3 | 20 mM histidine | 6.5 | 4% mannitol | 2% serine | 0.015% sodium lauryl sulfate | 0.1 mg/mL methionine | 15% hydroxypropyl β-cyclodextrin |
| 1-4 | 20 mM histidine | 6.5 | 4% mannitol | 2% serine | 0.02% polysorbate 20 | 0.1 mg/mL methionine | - |
| 1-5 | 20 mM histidine | 6.5 | 8% sucrose | 2% serine | 0.015% sodium lauryl sulfate | 0.1 mg/mL methionine | - |
| 1-6 | 20 mM histidine | 6.5 | 8% sucrose | 2% alanine | 0.015% sodium lauryl sulfate | 0.1 mg/mL methionine | - |
| 1-7 | 20 mM histidine | 6.5 | 8% sucrose | 2% serine | 0.02% polysorbate 20 | 0.1 mg/mL methionine | - |
| 1-8 | 20 mM histidine | 7.5 | 4% mannitol | 2% serine | 0.015% sodium lauryl sulfate | 0.1 mg/mL methionine | - |
| 1-9 | 20 mM histidine | 7.5 | 4% mannitol | 2% alanine | 0.015% sodium lauryl sulfate | 0.1 mg/mL methionine | - |
| 1-10 | 20 mM histidine | 7.5 | 4% mannitol | 2% serine | 0.02% polysorbate 20 | 0.1 mg/mL methionine | - |
| 2-1 | 20 mM histidine | 6.5 | 9% sucrose | - | 0.02% polysorbate 20 | 0.1 mg/mL methionine | - |
| 2-2 | 20 mM histidine | 6.5 | 5% sucrose | 1% alanine | 0.02% polysorbate 20 | 0.1 mg/mL methionine | - |
| 2-3 | 20 mM histidine | 6.5 | 5% sucrose | 1.3% alanine | 0.02% polysorbate 20 | 0.1 mg/mL methionine | - |
| 2-4 | 20 mM histidine | 6.5 | 5% sucrose | 2% alanine | 0.02% polysorbate 20 | 0.1 mg/mL methionine | - |
| 2-5 | 20 mM histidine | 6.5 | 5% sucrose | 0.7% arginine 0.7% glutamic acid | 0.02% polysorbate 20 | 0.1 mg/mL methionine | - |
| 2-6 | 20 mM histidine | 6.5 | 5% sucrose | 2% alanine | 0.015% sodium lauryl sulfate | 0.1 mg/mL methionine | - |
| 2-7 | 20 mM histidine | 7.5 | 5% sucrose | 1.3% alanine | 0.02% polysorbate 20 | 0.1 mg/mL methionine | - |
| 2-8 | 20 mM histidine | 7.5 | 5% sucrose | 2% alanine | 0.02% polysorbate 20 | 0.1 mg/mL methionine | - |
| 2-9 | 20 mM histidine | 7.5 | 5% sucrose | 0.7% arginine 0.7% glutamic acid | 0.02% polysorbate 20 | 0.1 mg/mL methionine | - |
| 2-10 | 20 mM histidine | 7.5 | 5% sucrose | 1.3% alanine | 0.015% sodium lauryl sulfate | 0.1 mg/mL methionine | - |

**[Table 5]**

| No. | Concentration of long-acting conjugate | Period | Phase | SE-HPLC |
|---|---|---|---|---|
| | | | | Main Peak (%) |
| 1-1 | 92.0 nmol/mL | Initial | Clear liquid | 83.3 |
| | 230.1 nmol/mL | | | 82.4 |
| | 859.1 nmol/mL | | | 77.7 |
| | 536.9 nmol/mL | | Gel | 48.4 |
| 1-2 | 92.0 nmol/mL | Initial | Clear liquid | 82.5 |
| | 153.4 nmol/mL | | | 80.2 |
| | 751.7 nmol/mL | | | 77.4 |
| | 1334.6 nmol/mL | | Gel | 51.5 |
| 1-3 | 76.7 nmol/mL | Initial | Clear liquid | 97.2 |
| | 122.7 nmol/mL | | | 96.7 |
| | 444.9 nmol/mL | | | 97.0 |
| | 521.6 nmol/mL | | | 91.1 |
| 1-4 | 122.7 nmol/mL | Initial | Clear liquid | 95.6 |
| | 230.1 nmol/mL | | | 94.7 |
| | 813.0 nmol/mL | | | 92.1 |
| | 2362.4 nmol/mL | | Gel | 31.7 |
| 1-5 | 92.0 nmol/mL | Initial | Clear liquid | 83.6 |
| | 168.7 nmol/mL | | | 81.7 |
| | 644.3 nmol/mL | | | 80.3 |
| | 1119.8 nmol/mL | | Gel | 54.6 |
| 1-6 | 92.0 nmol/mL | Initial | Clear liquid | 84.3 |
| | 184.1 nmol/mL | | | 83.5 |
| | 644.3 nmol/mL | | | 80.1 |
| | 1319.3 nmol/mL | | Gel | 58.3 |
| 1-7 | 122.7 nmol/mL | Initial | Clear liquid | 95.8 |
| | 184.1 nmol/mL | | | 96.2 |
| | 567.6 nmol/mL | | | 94.3 |
| | 2515.8 nmol/mL | | Gel | 51.5 |
| 1-8 | 92.0 nmol/mL | Initial | Clear liquid | 93.5 |
| | 184.1 nmol/mL | | | 93.6 |
| | 889.7 nmol/mL | | | 92.7 |
| | 1687.4 nmol/mL | | Gel | 76.1 |
| 1-9 | 76.7 nmol/mL | Initial | Clear liquid | 93.6 |
| | 138.1 nmol/mL | | | 93.6 |
| | 797.7 nmol/mL | | | 93.0 |
| | 1748.8 nmol/mL | | Gel | 76.3 |
| 1-10 | 138.1 nmol/mL | Initial | Clear liquid | 97.1 |
| | 322.1 nmol/mL | | | 96.3 |
| | 1165.9 nmol/mL | | | 93.7 |
| | 1380.6 nmol/mL | | Gel | 31.3 |
| 2-1 | 1073.8 nmol/mL | Initial | Clear liquid | 88.3 |
| | | 1 week | Insoluble particles | 4.2 |
| 2-2 | 1073.8 nmol/mL | Initial | Clear liquid | 80.1 |
| | | 1 week | Insoluble particles | 3.8 |
| 2-3 | 536.9 nmol/mL | Initial | Clear liquid | 74.7 |
| | | 1 week | Insoluble particles | 6.7 |
| | 1073.8 nmol/mL | Initial | Clear liquid | 86.6 |
| | | 1 week | Insoluble particles | 4.3 |
| 2-4 | 1073.8 nmol/mL | Initial | Clear liquid | 85.0 |
| | | 1 week | Insoluble particles | 4.5 |
| 2-5 | 1073.8 nmol/mL | Initial | Clear liquid | 89.3 |
| | | 1 week | Insoluble particles | 9.9 |
| 2-6 | 1073.8 nmol/mL | Initial | Clear liquid | 73.1 |
| | | 1 week | Insoluble particles | 2.9 |
| 2-7 | 536.9 nmol/mL | Initial | Clear liquid | 79.3 |
| | | 1 week | Insoluble particles | 8.8 |
| | 1073.8 nmol/mL | Initial | Clear liquid | 71.5 |
| | | 1 week | Insoluble particles | 5.0 |
| 2-8 | 1073.8 nmol/mL | Initial | Clear liquid | 72.8 |
| | | 1 week | Insoluble particles | 4.6 |
| 2-9 | 1073.8 nmol/mL | Initial | Clear liquid | 78.9 |
| | | 1 week | Insoluble particles | 10.9 |
| 2-10 | 1073.8 nmol/mL | Initial | Clear liquid | 87.2 |
| | | 1 week | Clear liquid | 25.6 |

As can be seen from the results of the screening of formulations No. 1-1 to No. 1-10, the phase of each formulation having the highest concentration of the long-acting conjugate was a gel except for No. 1-3.

As can be seen from the accelerated 1-week stability results of the screening of formulations No. 2-1 to No. 2-10, the SE-HPLC main peak purity of the long-acting conjugate significantly decreased, and the main impurity was in the form of a high molecular weight species. It was found that foreign substances were produced when the formulations No. 2-1 to No. 2-9, excluding the formulation No. 2-10 containing sodium lauryl sulfate, were stored for one week under an accelerated condition.

Through this Example, interleukin 2 analog long-acting conjugates with a pH ranging from 6.5 to 7.5 were prepared using mannitol or sucrose and several amino acids as a stabilizer. The initial purity of preparation varied depending on the combination of the respective additives, and optimization of the additives used was performed to ensure acceleration stability.

### Example 8: Evaluation of stability of liquid formulation containing interleukin 2 analog long-acting conjugate depending on concentration of sucrose and kind and concentration of surfactant and amino acid

The stability of the interleukin 2 analog 86 conjugate depending on the appropriate combination of the concentration of sucrose, the kind and concentration of surfactant (polysorbate 20, polysorbate 80, sodium lauryl sulfate, or poloxamer 188), and the kind and concentration of amino acid (serine, alanine, arginine, glutamic acid, or proline) was compared based on liquid formulations with pH 6.5 containing histidine as a buffer and methionine as an antioxidant.

Using liquid formulations of interleukin 2 analog 86 conjugates (concentration of long-acting conjugate in liquid formulation: about 18.4 to 1073.8 nmol/mL) having the compositions as presented in Table 6 below, the purity and stability of the liquid formulations were analyzed by size exclusion chromatography and reverse phase chromatography. The accelerated condition liquid formulation was stored at 25±5°C, and the refrigerated condition liquid formulation was stored at 5±3°C before analysis, and the results are presented in Tables 7 and 8, respectively. The SE-HPLC (%) and RP-HPLC (%) in Tables 7 and 8 present the chromatography results for each liquid formulation in Table 6 denoted by %area and area%/start area% (%) with respect to the main peak, and indicate the initial purity of the interleukin 2 analog 86 conjugate and the residual percentage compared to the initial result value.

**[Table 6]**

| No. | Buffering substance | pH | Sugar (w/v) | Amino acid (w/v) | Surfactant (w/v) | Antioxidant | Others |
|---|---|---|---|---|---|---|---|
| 3-1 | 20 mM histidine | 6.5 | 17% sucrose | - | 0.02% polysorbate 20 | 0.1 mg/mL methionine | |
| 3-2 | 20 mM histidine | 6.5 | 8% sucrose | 2% serine | 0.02% polysorbate 20 | 0.1 mg/mL methionine | |
| 3-3 | 20 mM histidine | 6.5 | 8% sucrose | 2% serine | 0.02% poloxamer 188 | 0.1 mg/mL methionine | |
| 3-4 | 20 mM histidine | 6.5 | 8% sucrose | 0.7% arginine 0.7% glutamic acid | 0.02% polysorbate 20 | 0.1 mg/mL methionine | |
| 3-5 | 20 mM histidine | 6.5 | 12% sucrose | 0.7% arginine 0.7% glutamic acid | 0.02% polysorbate 20 | 0.1 mg/mL methionine | |
| 3-6 | 20 mM histidine | 6.5 | 8% sucrose | 2% arginine | 0.02% polysorbate 20 | 0.1 mg/mL methionine | |
| 3-7 | 20 mM histidine | 6.5 | 8% sucrose | 2% serine | 0.015% sodium lauryl sulfate | 0.1 mg/mL methionine | 50 mM NaCl |
| 3-8 | 20 mM histidine | 6.5 | 8% sucrose | 1.3% alanine | 0.015% sodium lauryl sulfate | 0.1 mg/mL methionine | |
| 3-9 | 20 mM histidine | 6.5 | 12% sucrose | 1.3% alanine | 0.015% sodium lauryl sulfate | 0.1 mg/mL methionine | |
| 3-10 | 20 mM histidine | 6.5 | 8% sucrose | 1.3% alanine | 0.03% sodium lauryl sulfate | 0.1 mg/mL methionine | |
| 3-11 | 20 mM histidine | 6.5 | 8% sucrose | 2% proline | 0.015% sodium lauryl sulfate | 0.1 mg/mL methionine | |
| 4-1 | 20 mM histidine | 6.5 | 8% sucrose | 2% arginine | 0.02% polysorbate 20 | 0.1 mg/mL methionine | 50 mM NaCl |
| 4-2 | 20 mM histidine | 6.5 | 8% sucrose | 4% arginine | 0.02% polysorbate 20 | 0.1 mg/mL methionine | |
| 4-3 | 20 mM histidine | 6.5 | 8% sucrose | 2% arginine | 0.02% poloxamer 188 | 0.1 mg/mL methionine | |
| 4-4 | 20 mM histidine | 6.5 | 8% sucrose | 2% arginine | 0.02% poloxamer 188 | 0.1 mg/mL methionine | 50 mM NaCl |
| 4-5 | 20 mM histidine | 6.5 | 8% sucrose | 4% arginine | 0.02% poloxamer 188 | 0.1 mg/mL methionine | |
| 4-6 | 20 mM histidine | 6.5 | 10% sucrose | 3% arginine | 0.02% poloxamer 188 | 0.1 mg/mL methionine | |
| 4-7 | 20 mM histidine | 6.5 | 12% sucrose | 2% arginine | 0.02% poloxamer 188 | 0.1 mg/mL methionine | |
| 5-1 | 20 mM histidine | 6.5 | 10% sucrose | 3% arginine | 0.02% poloxamer 188 | 0.1 mg/mL methionine | 50 mM NaCl |
| 5-2 | 20 mM histidine | 6.5 | 10% sucrose | 3% arginine | 0.02% poloxamer 188 | 0.1 mg/mL methionine | 100 mM NaCl |
| 5-3 | 20 mM histidine | 6.5 | 12% sucrose | 3% arginine | 0.02% poloxamer 188 | 0.1 mg/mL methionine | |
| 5-4 | 20 mM histidine | 6.5 | 14% sucrose | 3% arginine | 0.02% poloxamer 188 | 0.1 mg/mL methionine | |
| 5-5 | 20 mM histidine | 6.5 | 10% sucrose | 4% arginine | 0.005% poloxamer 188 | 0.1 mg/mL methionine | |

Table 7 below summarizes the accelerated storage stability at 25±5°C.

**[Table 7]**

| No. | Concentration | Period | Phase | SE-HPLC | RP-HPLC |
|---|---|---|---|---|---|
| | | | | Main (%) (Area%/Start Area% (%)) | Main (%) (Area%/Start Area% (%)) |
| 3-1 | 153.4 nmol/mL | Initial | Clear liquid | 93.3 (100) | 92.2 (100) |
| | 1073.8 nmol/mL | Initial | | 84.6 (100) | 92.2 (100) |
| | | 1 week | | 5.5 (6.5) | 91.2 (98.9) |
| 3-2 | 153.4 nmol/mL | Initial | Clear liquid | 96.4 (100) | 90.6 (100) |
| | | 5 days | | 63.4 (65.8) | 89.9 (99.2) |
| | | 13 days | | 44.3 (46.0) | 86.5 (95.5) |
| | 306.8 nmol/mL | Initial | Clear liquid | 96.1 (100) | 90.4 (100) |
| | | 5 days | | 34.7 (36.1) | 88.7 (98.1) |
| | | 13 days | | 17.6 (18.3) | 84.5 (93.5) |
| | 536.9 nmol/mL | Initial | Clear liquid | 95.9 (100) | 90.3 (100) |
| | | 5 days | | 20.8 (21.7) | 88.3 (97.8) |
| | | 13 days | | 10.1 (10.5) | 84.6 (93.7) |
| 3-3 | 153.4 nmol/mL | Initial | Clear liquid | 98.4 (100) | 91.0 (100) |
| | | 5 days | | 92.1 (93.6) | 91.6 (100.7) |
| | | 13 days | | 82.9 (84.2) | 89.1 (97.9) |
| | 306.8 nmol/mL | Initial | Clear liquid | 98.3 (100) | 90.9 (100) |
| | | 5 days | | 72.0 (73.2) | 91.3 (100.4) |
| | | 13 days | | 50.8 (51.7) | 88.2 (97.0) |
| | 536.9 nmol/mL | Initial | Clear liquid | 98.2 (100) | 90.8 (100) |
| | | 5 days | | 48.2 (49.1) | 91.0 (100.2) |
| | | 13 days | | 23.0 (23.4) | 88.4 (97.4) |
| 3-4 | 153.4 nmol/mL | Initial | Clear liquid | 99.0 (100) | 90.9 (100) |
| | | 5 days | | 86.9 (87.8) | 91.6 (100.8) |
| | | 13 days | | 71.9 (72.6) | 89.3 (98.2) |
| | 306.8 nmol/mL | Initial | Clear liquid | 98.9 (100) | 90.8 (100) |
| | | 5 days | | 64.0 (64.7) | 91.6 (100.9) |
| | | 13 days | | 43.7 (44.2) | 88.6 (97.6) |
| | 536.9 nmol/mL | Initial | Clear liquid | 98.8 (100) | 90.8 (100) |
| | | 5 days | | 35.7 (36.1) | 90.8 (100) |
| | | 13 days | | 21.6 (21.9) | 87.9 (96.8) |
| 3-5 | 153.4 nmol/mL | Initial | Clear liquid | 99.1 (100) | 90.8 (100) |
| | | 5 days | | 93.8 (94.7) | 91.7 (101.0) |
| | | 13 days | | 85.4 (86.2) | 89.6 (98.7) |
| | 306.8 nmol/mL | Initial | Clear liquid | 98.8 (100) | 90.7 (100) |
| | | 5 days | | 67.2 (68.0) | 91.4 (100.8) |
| | | 13 days | | 46.3 (46.9) | 89.3 (98.5) |
| | 536.9 nmol/mL | Initial | Clear liquid | 98.8 (100) | 90.7 (100) |
| | | 5 days | | 48.0 (48.6) | 91.4 (100.8) |
| | | 13 days | | 29.3 (29.7) | 89.0 (98.1) |
| 3-6 | 153.4 nmol/mL | Initial | Clear liquid | 99.3 (100) | 90.5 (100) |
| | | 5 days | | 98.6 (99.3) | 91.8 (101.4) |
| | | 13 days | | 95.4 (96.1) | 89.1 (98.5) |
| | | 4 weeks | | 93.5 (94.2) | 88.6 (97.9) |
| | 306.8 nmol/mL | Initial | Clear liquid | 99.4 (100) | 90.4 (100) |
| | | 5 days | | 94.3 (94.9) | 91.6 (101.3) |
| | | 13 days | | 85.8 (86.3) | 88.8 (98.2) |
| | | 4 weeks | | 76.7 (77.2) | 88.4 (97.8) |
| | 536.9 nmol/mL | Initial | Clear liquid | 99.3 (100) | 90.7 (100) |
| | | 5 days | | 69.0 (69.5) | 91.4 (100.8) |
| | | 13 days | | 49.3 (49.6) | 88.4 (97.5) |
| | | 4 weeks | | 40.0 (40.3) | 88.4 (97.5) |
| 3-7 | 687.2 nmol/mL | Initial | Clear liquid | 93.6 (100) | 91.9 (100) |
| | 822.2 nmol/mL | | Sticky liquid | 91.2 (100) | 92.2 (100) |
| 3-8 | 696.4 nmol/mL | Initial | Clear liquid | 94.4 (100) | 92.0 (100) |
| | 966.4 nmol/mL | | Sticky liquid | 81.2 (100) | 92.2 (100) |
| 3-9 | 584.5 nmol/mL | Initial | Clear liquid | 95.9 (100) | 92.4 (100) |
| | 900.5 nmol/mL | | Sticky liquid | 85.0 (100) | 92.2 (100) |
| 3-10 | 581.4 nmol/mL | Initial | Clear liquid | 64.1 (100) | 91.8 (100) |
| | 630.5 nmol/mL | | Gel | 42.8 (100) | 92.0 (100) |
| 3-11 | 716.4 nmol/mL | Initial | Clear liquid | 93.6 (100) | 91.9 (100) |
| | 946.5 nmol/mL | | Sticky liquid | 82.7 (100) | 92.3 (100) |
| 4-1 | 306.8 nmol/mL | Initial | Clear liquid | 98.9 (100) | 92.4 (100) |
| | | 1 week | | 93.4 (94.4) | 91.4 (98.9) |
| | | 2 weeks | | 85.9 (86.9) | 90.3 (97.7) |
| | 383.5 nmol/mL | Initial | Clear liquid | 98.9 (100) | 92.4 (100) |
| | | 1 week | | 89.2 (90.2) | 91.4 (98.9) |
| | | 2 weeks | | 78.4 (79.3) | 90.2 (97.6) |
| | 536.9 nmol/mL | Initial | Clear liquid | 99.0 (100) | 92.3 (100) |
| | | 1 week | | 75.4 (76.2) | 91.6 (99.2) |
| | | 2 weeks | | 59.1 (59.7) | 90.7 (98.3) |
| 4-2 | 306.8 nmol/mL | Initial | Clear liquid | 98.9 (100) | 91.4 (100) |
| | | 1 week | | 95.0 (96.1) | 90.2 (98.7) |
| | | 2 weeks | | 83.5 (84.4) | 87.1 (95.3) |
| | 383.5 nmol/mL | Initial | Clear liquid | 98.8 (100) | 91.6 (100) |
| | | 1 week | | 92.8 (93.9) | 90.0 (98.3) |
| | | 2 weeks | | 79.0 (80.0) | 86.7 (94.7) |
| | 536.9 nmol/mL | Initial | Clear liquid | 98.7 (100) | 91.7 (100) |
| | | 1 week | | 83.8 (84.9) | 89.5 (97.6) |
| | | 2 weeks | | 62.0 (62.8) | 85.9 (93.7) |
| 4-3 | 306.8 nmol/mL | Initial | Clear liquid | 98.5 (100) | 92.5 (100) |
| | | 1 week | | 96.4 (97.9) | 91.3 (98.7) |
| | | 2 weeks | | 94.5 (95.9) | 90.1 (97.4) |
| | 383.5 nmol/mL | Initial | Clear liquid | 98.5 (100) | 92.4 (100) |
| | | 1 week | | 96.4 (97.9) | 91.4 (98.9) |
| | | 2 weeks | | 94.1 (95.5) | 90.3 (97.7) |
| | 536.9 nmol/mL | Initial | Clear liquid | 98.4 (100) | 92.4 (100) |
| | | 1 week | | 92.2 (93.7) | 91.7 (99.2) |
| | | 2 weeks | | 86.9 (88.3) | 90.4 (97.8) |
| 4-4 | 306.8 nmol/mL | Initial | Clear liquid | 98.4 (100) | 92.6 (100) |
| | | 1 week | | 97.5 (99.1) | 91.4 (98.7) |
| | | 2 weeks | | 95.9 (97.5) | 90.5 (97.7) |
| | 383.5 nmol/mL | Initial | Clear liquid | 98.4 (100) | 92.6 (100) |
| | | 1 week | | 96.5 (98.1) | 91.2 (98.5) |
| | | 2 weeks | | 94.3 (95.8) | 90.3 (97.5) |
| | 536.9 nmol/mL | Initial | Clear liquid | 98.4 (100) | 92.6 (100) |
| | | 1 week | | 93.9 (95.4) | 91.4 (98.7) |
| | | 2 weeks | | 89.2 (90.7) | 90.7 (97.9) |
| 4-5 | 18.4 nmol/mL | Initial | Clear liquid | 88.8 (100) | 92.0 (100) |
| 4-6 | 306.8 nmol/mL | Initial | Clear liquid | 97.9 (100) | 92.3 (100) |
| | | 1 week | | 97.5 (99.6) | 91.2 (98.8) |
| | | 2 weeks | | 96.4 (98.5) | 89.9 (97.4) |
| | 383.5 nmol/mL | Initial | Clear liquid | 98.0 (100) | 92.3 (100) |
| | | 1 week | | 97.2 (99.2) | 91.1 (98.7) |
| | | 2 weeks | | 95.8 (97.8) | 90.0 (97.5) |
| | 536.9 nmol/mL | Initial | Clear liquid | 97.9 (100) | 92.6 (100) |
| | | 1 week | | 95.8 (97.9) | 91.3 (98.6) |
| | | 2 weeks | | 93.2 (95.2) | 90.2 (97.4) |
| 4-7 | 306.8 nmol/mL | Initial | Clear liquid | 98.0 (100) | 92.4 (100) |
| | | 1 week | | 97.3 (99.3) | 91.5 (99.0) |
| | | 2 weeks | | 95.7 (97.7) | 90.4 (97.8) |
| | 383.5 nmol/mL | Initial | Clear liquid | 97.9 (100) | 92.3 (100) |
| | | 1 week | | 96.6 (98.7) | 91.3 (98.9) |
| | | 2 weeks | | 94.6 (96.6) | 90.2 (97.7) |
| | 536.9 nmol/mL | Initial | Clear liquid | 98.0 (100) | 92.3 (100) |
| | | 1 week | | 95.3 (97.2) | 91.4 (99.0) |
| | | 2 weeks | | 91.9 (93.8) | 90.4 (97.9) |
| 5-1 | 306.8 nmol/mL | Initial | Clear liquid | 98.3 (100) | 90.2 (100) |
| | | 1 week | | 98.3 (100) | 91.0 (100.9) |
| | | 4 weeks | | 96.2 (97.9) | 90.1 (99.9) |
| | 383.5 nmol/mL | Initial | Clear liquid | 98.3 (100) | 90.3 (100) |
| | | 1 week | | 98.0 (99.7) | 90.6 (100.3) |
| | | 4 weeks | | 95.3 (96.9) | 89.8 (98.9) |
| | 536.9 nmol/mL | Initial | Clear liquid | 98.2 (100) | 90.0 (100) |
| | | 1 week | | 97.5 (99.3) | 90.7 (100.8) |
| | | 4 weeks | | 93.6 (95.3) | 89.8 (99.8) |
| 5-2 | 306.8 nmol/mL | Initial | Clear liquid | 97.3 (100) | 89.6 (100) |
| | | 1 week | | 93.2 (95.8) | 87.3 (97.4) |
| | | 4 weeks | | 80.6 (82.8) | 74.6 (83.3) |
| | 383.5 nmol/mL | Initial | Clear liquid | 97.6 (100) | 89.9 (100) |
| | | 1 week | | 95.9 (98.3) | 88.7 (98.7) |
| | | 4 weeks | | 90.3 (92.5) | 84.1 (93.5) |
| | 536.9 nmol/mL | Initial | Clear liquid | 97.5 (100) | 89.9 (100) |
| | | 1 week | | 95.5 (97.9) | 88.9 (98.9) |
| | | 4 weeks | | 88.9 (91.2) | 84.3 (93.8) |
| 5-3 | 306.8 nmol/mL | Initial | Clear liquid | 98.2 (100) | 89.7 (100) |
| | | 1 week | | 98.2 (100) | 91.1 (101.6) |
| | | 4 weeks | | 95.6 (97.4) | 89.3 (99.6) |
| | 383.5 nmol/mL | Initial | Clear liquid | 98.2 (100) | 89.5 (100) |
| | | 1 week | | 97.9 (99.7) | 90.7 (101.3) |
| | | 4 weeks | | 94.3 (96.0) | 89.3 (99.8) |
| | 536.9 nmol/mL | Initial | Clear liquid | 98.2 (100) | 89.5 (100) |
| | | 1 week | | 97.5 (99.3) | 90.5 (101.1) |
| | | 4 weeks | | 93.2 (94.9) | 89.1 (99.6) |
| 5-4 | 306.8 nmol/mL | Initial | Clear liquid | 98.2 (100) | 89.6 (100) |
| | | 1 week | | 98.2 (100) | 91.1 (101.7) |
| | | 4 weeks | | 95.9 (97.7) | 89.5 (99.9) |
| | 383.5 nmol/mL | Initial | Clear liquid | 98.1 (100) | 89.2 (100) |
| | | 1 week | | 97.9 (99.8) | 90.6 (101.6) |
| | | 4 weeks | | 95.0 (96.8) | 89.5 (100.3) |
| | 536.9 nmol/mL | Initial | Clear liquid | 98.0 (100) | 89.0 (100) |
| | | 1 week | | 97.7 (99.7) | 90.5 (101.7) |
| | | 4 weeks | | 93.9 (95.8) | 90.0 (101.1) |
| 5-5 | 306.8 nmol/mL | Initial | Clear liquid | 98.5 (100) | 89.1 (100) |
| | | 1 week | | 98.5 (100) | 90.7 (101.8) |
| | | 4 weeks | | 95.3 (96.8) | 89.4 (100.3) |
| | 383.5 nmol/mL | Initial | Clear liquid | 98.5 (100) | 89.0 (100) |
| | | 1 week | | 98.0 (99.5) | 90.1 (101.2) |
| | | 4 weeks | | 93.0 (94.4) | 89.4 (100.4) |
| | 536.9 nmol/mL | Initial | Clear liquid | 98.5 (100) | 88.9 (100) |
| | | 1 week | | 97.7 (99.2) | 90.2 (101.5) |
| | | 4 weeks | | 91.9 (93.3) | 89.4 (100.6) |

Table 8 below summarizes the refrigerated storage stability at 5±3°C.

**[Table 8]**

| No. | Concentration | Period | Phase | SE-HPLC | RP-HPLC |
|---|---|---|---|---|---|
| | | | | Main (%) (Area%/Start Area% (%)) | Main (%) (Area%/Start Area% (%)) |
| 3-1 | 153.4 nmol/mL | Initial | Clear liquid | 93.3 (100) | 92.2 (100) |
| | | 1 week | | 88.5 (94.9) | 92.1 (99.9) |
| | 1073.8 nmol/mL | Initial | | 84.6 (100) | 92.2 (100) |
| | | 1 week | | 58.9 (69.6) | 91.7 (99.5) |
| 3-2 | 153.4 nmol/mL | Initial | Clear liquid | 96.4 (100) | 90.6 (100) |
| | | 1 week | | 93.9 (97.4) | 90.4 (99.8) |
| | | 2 weeks | | 93.5 (97.0) | 90.3 (99.7) |
| | | 4 weeks | | 93.2 (96.7) | 89.6 (98.9) |
| | 306.8 nmol/mL | Initial | Clear liquid | 96.1 (100) | 90.4 (100) |
| | | 1 week | | 90.1 (93.8) | 89.8 (99.3) |
| | | 2 weeks | | 89.5 (93.1) | 89.8 (99.3) |
| | | 4 weeks | | 88.6 (92.2) | 89.4 (98.9) |
| | 536.9 nmol/mL | Initial | Clear liquid | 95.9 (100) | 90.3 (100) |
| | | 1 week | | 84.7 (88.3) | 89.6 (99.2) |
| | | 2 weeks | | 82.6 (86.1) | 90.1 (99.8) |
| | | 4 weeks | | 79.8 (83.2) | 89.3 (98.9) |
| 3-3 | 153.4 nmol/mL | Initial | Clear liquid | 98.4 (100) | 91.0 (100) |
| | | 1 week | | 98.2 (99.8) | 92.2 (101.3) |
| | | 2 weeks | | 98.4 (100) | 91.4 (100.4) |
| | | 4 weeks | | 98.5 (100.1) | 91.3 (100.3) |
| | 306.8 nmol/mL | Initial | Clear liquid | 98.3 (100) | 90.9 (100) |
| | | 1 week | | 97.8 (99.5) | 92.3 (101.5) |
| | | 2 weeks | | 97.7 (99.4) | 91.5 (100.7) |
| | | 4 weeks | | 97.5 (99.2) | 91.3 (100.4) |
| | 536.9 nmol/mL | Initial | Clear liquid | 98.2 (100) | 90.8 (100) |
| | | 1 week | | 97.4 (99.2) | 91.6 (100.9) |
| | | 2 weeks | | 96.7 (98.5) | 91.5 (100.8) |
| | | 4 weeks | | 96.3 (98.1) | 91.2 (100.4) |
| 3-4 | 153.4 nmol/mL | Initial | Clear liquid | 99.0 (100) | 90.9 (100) |
| | | 1 week | | 99.0 (100) | 92.5 (101.8) |
| | | 2 weeks | | 98.8 (99.8) | 91.2 (100.3) |
| | | 4 weeks | | 98.9 (99.9) | 91.0 (100.1) |
| | 306.8 nmol/mL | Initial | Clear liquid | 98.9 (100) | 90.8 (100) |
| | | 1 week | | 98.5 (99.6) | 92.0 (101.3) |
| | | 2 weeks | | 98.4 (99.5) | 91.5 (100.8) |
| | | 4 weeks | | 98.3 (99.4) | 91.0 (100.2) |
| | 536.9 nmol/mL | Initial | Clear liquid | 98.8 (100) | 90.8 (100) |
| | | 1 week | | 96.3 (97.5) | 92.4 (101.8) |
| | | 2 weeks | | 95.5 (96.7) | 91.1 (100.3) |
| | | 4 weeks | | 94.4 (95.5) | 90.9 (100.1) |
| 3-5 | 153.4 nmol/mL | Initial | Clear liquid | 99.1 (100) | 90.8 (100) |
| | | 1 week | | 99.1 (100) | 92.4 (101.8) |
| | | 2 weeks | | 99.0 (99.9) | 91.4 (100.7) |
| | | 4 weeks | | 99.2 (100.1) | 91.0 (100.2) |
| | 306.8 nmol/mL | Initial | Clear liquid | 98.8 (100) | 90.7 (100) |
| | | 1 week | | 98.5 (99.7) | 91.9 (101.3) |
| | | 2 weeks | | 98.4 (99.6) | 91.3 (100.7) |
| | | 4 weeks | | 98.4 (99.6) | 91.0 (100.3) |
| | 536.9 nmol/mL | Initial | Clear liquid | 98.8 (100) | 90.7 (100) |
| | | 1 week | | 97.5 (98.7) | 91.8 (101.2) |
| | | 2 weeks | | 97.5 (98.7) | 91.0 (100.3) |
| | | 4 weeks | | 96.9 (98.1) | 91.0 (100.3) |
| 3-6 | 153.4 nmol/mL | Initial | Clear liquid | 99.3 (100) | 90.5 (100) |
| | | 1 week | | 99.5 (100.2) | 91.9 (101.5) |
| | | 2 weeks | | 99.4 (100.1) | 91.4 (101.0) |
| | | 4 weeks | | 99.6 (100.3) | 91.0 (100.6) |
| | 306.8 nmol/mL | Initial | Clear liquid | 99.4 (100) | 90.4 (100) |
| | | 1 week | | 99.4 (100) | 91.9 (101.7) |
| | | 2 weeks | | 99.3 (99.9) | 91.0 (100.7) |
| | | 4 weeks | | 99.6 (100.2) | 90.7 (100.3) |
| | 536.9 nmol/mL | Initial | Clear liquid | 99.3 (100) | 90.7 (100) |
| | | 1 week | | 99.3 (100) | 91.9 (101.3) |
| | | 2 weeks | | 99.1 (99.8) | 90.9 (100.2) |
| | | 4 weeks | | 99.2 (99.9) | 90.9 (100.2) |
| 3-7 | 687.2 nmol/mL | Initial | Clear liquid | 93.6 (100) | 91.9 (100) |
| | 822.2 nmol/mL | | Sticky liquid | 91.2 (100) | 92.2 (100) |
| 3-8 | 696.4 nmol/mL | Initial | Clear liquid | 94.4 (100) | 92.0 (100) |
| | 966.4 nmol/mL | | Sticky liquid | 81.2 (100) | 92.2 (100) |
| 3-9 | 584.5 nmol/mL | Initial | Clear liquid | 95.9 (100) | 92.4 (100) |
| | 900.5 nmol/mL | | Sticky liquid | 85.0 (100) | 92.2 (100) |
| 3-10 | 581.4 nmol/mL | Initial | Clear liquid | 64.1 (100) | 91.8 (100) |
| | 630.5 nmol/mL | | Gel | 42.8 (100) | 92.0 (100) |
| 3-11 | 716.4 nmol/mL | Initial | Clear liquid | 93.6 (100) | 91.9 (100) |
| | 946.5 nmol/mL | | Sticky liquid | 82.7 (100) | 92.3 (100) |
| 4-1 | 306.8 nmol/mL | Initial | Clear liquid | 98.9 (100) | 92.4 (100) |
| | | 1 week | | 99.3 (100.4) | 92.1 (99.7) |
| | | 2 weeks | | 99.3 (100.4) | 91.5 (99.0) |
| | 383.5 nmol/mL | Initial | Clear liquid | 98.9 (100) | 92.4 (100) |
| | | 1 week | | 99.2 (100.3) | 92.1 (99.7) |
| | | 2 weeks | | 99.2 (100.3) | 91.5 (99.0) |
| | 536.9 nmol/mL | Initial | Clear liquid | 99.0 (100) | 92.3 (100) |
| | | 1 week | | 99.2 (100.2) | 92.1 (99.8) |
| | | 2 weeks | | 99.2 (100.2) | 91.5 (99.1) |
| 4-2 | 306.8 nmol/mL | Initial | Clear liquid | 98.9 (100) | 91.4 (100) |
| | | 1 week | | 99.3 (100.4) | 91.7 (100.3) |
| | | 2 weeks | | 98.3 (99.4) | 90.7 (99.2) |
| | 383.5 nmol/mL | Initial | Clear liquid | 98.8 (100) | 91.6 (100) |
| | | 1 week | | 99.3 (100.5) | 91.5 (99.9) |
| | | 2 weeks | | 98.2 (99.4) | 90.5 (98.8) |
| | 536.9 nmol/mL | Initial | Clear liquid | 98.7 (100) | 91.7 (100) |
| | | 1 week | | 99.3 (100.6) | 91.2 (99.5) |
| | | 2 weeks | | 97.7 (99.0) | 90.2 (98.4) |
| 4-3 | 306.8 nmol/mL | Initial | Clear liquid | 98.5 (100) | 92.5 (100) |
| | | 1 week | | 98.8 (100.3) | 92.3 (99.8) |
| | | 2 weeks | | 98.8 (100.3) | 91.3 (98.7) |
| | 383.5 nmol/mL | Initial | Clear liquid | 98.5 (100) | 92.4 (100) |
| | | 1 week | | 98.8 (100.3) | 92.3 (99.9) |
| | | 2 weeks | | 98.9 (100.4) | 91.3 (98.8) |
| | 536.9 nmol/mL | Initial | Clear liquid | 98.4 (100) | 92.4 (100) |
| | | 1 week | | 98.8 (100.4) | 92.5 (100.1) |
| | | 2 weeks | | 98.8 (100.4) | 91.3 (98.8) |
| 4-4 | 306.8 nmol/mL | Initial | Clear liquid | 98.4 (100) | 92.6 (100) |
| | | 1 week | | 98.9 (100.5) | 92.0 (99.4) |
| | | 2 weeks | | 98.9 (100.5) | 91.5 (98.8) |
| | 383.5 nmol/mL | Initial | Clear liquid | 98.4 (100) | 92.6 (100) |
| | | 1 week | | 98.9 (100.5) | 92.0 (99.4) |
| | | 2 weeks | | 98.8 (100.4) | 91.4 (98.7) |
| | 536.9 nmol/mL | Initial | Clear liquid | 98.4 (100) | 92.6 (100) |
| | | 1 week | | 98.9 (100.5) | 91.9 (99.2) |
| | | 2 weeks | | 98.8 (100.4) | 91.4 (98.7) |
| 4-5 | 18.4 nmol/mL | Initial | Clear liquid | 88.0 (100) | 92.0 (100) |
| 4-6 | 306.8 nmol/mL | Initial | Clear liquid | 97.9 (100) | 92.3 (100) |
| | | 1 week | | 98.7 (100.8) | 91.8 (99.5) |
| | | 2 weeks | | 98.5 (100.6) | 91.2 (98.8) |
| | 383.5 nmol/mL | Initial | Clear liquid | 98.0 (100) | 92.3 (100) |
| | | 1 week | | 98.5 (100.5) | 92.1 (99.8) |
| | | 2 weeks | | 98.7 (100.7) | 91.2 (98.8) |
| | 536.9 nmol/mL | Initial | Clear liquid | 97.9 (100) | 92.6 (100) |
| | | 1 week | | 98.5 (100.6) | 91.9 (99.2) |
| | | 2 weeks | | 98.5 (100.6) | 91.3 (98.6) |
| 4-7 | 306.8 nmol/mL | Initial | Clear liquid | 98.0 (100) | 92.4 (100) |
| | | 1 week | | 98.7 (100.7) | 92.2 (99.8) |
| | | 2 weeks | | 98.5 (100.5) | 91.4 (98.9) |
| | 383.5 nmol/mL | Initial | Clear liquid | 97.9 (100) | 92.3 (100) |
| | | 1 week | | 98.5 (100.6) | 92.2 (99.9) |
| | | 2 weeks | | 98.5 (100.6) | 91.5 (99.1) |
| | 536.9 nmol/mL | Initial | Clear liquid | 98.0 (100) | 92.3 (100) |
| | | 1 week | | 98.6 (100.6) | 92.1 (99.8) |
| | | 2 weeks | | 98.5 (100.5) | 91.5 (99.1) |
| 5-1 | 306.8 nmol/mL | Initial | Clear liquid | 98.3 (100) | 90.2 (100) |
| | | 1 week | | 98.5 (100.2) | 91.2 (101.1) |
| | | 4 weeks | | 98.5 (100.2) | 90.8 (100.7) |
| | 383.5 nmol/mL | Initial | Clear liquid | 98.3 (100) | 90.3 (100) |
| | | 1 week | | 98.5 (100.2) | 91.0 (100.8) |
| | | 4 weeks | | 98.6 (100.3) | 90.4 (100.1) |
| | 536.9 nmol/mL | Initial | Clear liquid | 98.2 (100) | 90.0 (100) |
| | | 1 week | | 98.4 (100.2) | 91.1 (101.2) |
| | | 4 weeks | | 98.5 (100.3) | 90.4 (100.4) |
| 5-2 | 306.8 nmol/mL | Initial | Clear liquid | 97.3 (100) | 89.6 (100) |
| | | 1 week | | 96.5 (99.2) | 89.0 (99.3) |
| | | 4 weeks | | 93.2 (95.8) | 84.9 (94.8) |
| | 383.5 nmol/mL | Initial | Clear liquid | 97.6 (100) | 89.9 (100) |
| | | 1 week | | 97.6 (100) | 90.3 (100.4) |
| | | 4 weeks | | 96.4 (98.8) | 88.2 (98.1) |
| | 536.9 nmol/mL | Initial | Clear liquid | 97.5 (100) | 89.9 (100) |
| | | 1 week | | 97.5 (100) | 90.3 (100.4) |
| | | 4 weeks | | 96.4 (98.9) | 88.2 (98.1) |
| 5-3 | 306.8 nmol/mL | Initial | Clear liquid | 98.2 (100) | 89.7 (100) |
| | | 1 week | | 98.4 (100.2) | 91.6 (102.1) |
| | | 4 weeks | | 98.5 (100.3) | 90.3 (100.7) |
| | 383.5 nmol/mL | Initial | Clear liquid | 98.2 (100) | 89.5 (100) |
| | | 1 week | | 98.3 (100.1) | 91.2 (101.9) |
| | | 4 weeks | | 98.3 (100.1) | 90.0 (100.6) |
| | 536.9 nmol/mL | Initial | Clear liquid | 98.2 (100) | 89.5 (100) |
| | | 1 week | | 98.3 (100.1) | 91.1 (101.8) |
| | | 4 weeks | | 98.2 (100) | 90.0 (100.6) |
| 5-4 | 306.8 nmol/mL | Initial | Clear liquid | 98.2 (100) | 89.6 (100) |
| | | 1 week | | 98.3 (100.1) | 91.1 (101.7) |
| | | 4 weeks | | 98.2 (100) | 90.3 (100.8) |
| | 383.5 nmol/mL | Initial | Clear liquid | 98.1 (100) | 89.2 (100) |
| | | 1 week | | 98.3 (100.2) | 90.9 (101.9) |
| | | 4 weeks | | 98.2 (100.1) | 90.1 (101.0) |
| | 536.9 nmol/mL | Initial | Clear liquid | 98.0 (100) | 89.0 (100) |
| | | 1 week | | 98.2 (100.2) | 91.1 (102.4) |
| | | 4 weeks | | 98.2 (100.2) | 90.2 (101.3) |
| 5-5 | 306.8 nmol/mL | Initial | Clear liquid | 98.5 (100) | 89.1 (100) |
| | | 1 week | | 98.6 (100.1) | 90.5 (101.6) |
| | | 4 weeks | | 98.4 (99.9) | 90.2 (101.2) |
| | 383.5 nmol/mL | Initial | Clear liquid | 98.5 (100) | 89.0 (100) |
| | | 1 week | | 98.5 (100) | 90.6 (101.8) |
| | | 4 weeks | | 98.3 (99.8) | 90.0 (101.1) |
| | 536.9 nmol/mL | Initial | Clear liquid | 98.5 (100) | 88.9 (100) |
| | | 1 week | | 98.5 (100) | 90.2 (101.5) |
| | | 4 weeks | | 98.1 (99.6) | 89.9 (101.1) |

As can be seen from the results, in the accelerated stability results in Table 7 and the refrigerated stability results in Table 8, the phase of the liquid formulations No. 3-7 to No. 3-11 containing sodium lauryl sulfate as a surfactant was a sticky liquid or a gel at the highest concentration, but the phase of the liquid formulations No. 3-1 to No. 3-6 and No. 4-1 to No. 4-7 and No. 5-1 to No. 5-5 containing polysorbate 20 or polysorbate 80 or poloxamer 188 was a clear liquid at all concentrations.

As can be seen from the refrigerated stability results in Table 8, in the liquid formulations containing the interleukin 2 analog 86 conjugate, histidine, methionine, sucrose, a surfactant (polysorbate 20, polysorbate 80, sodium lauryl sulfate, or poloxamer 188), and an amino acid (serine, alanine, arginine, glutamic acid, or proline), it was found that the interleukin 2 analog 86 conjugate maintained its initial purity and was stable. As can be seen from the accelerated stability results in Table 7, liquid formulations No. 5-1 and No. 5-4 exhibited the highest stability.

Specifically, the formulation containing poloxamer 188 as a surfactant of the liquid formulation exhibited higher stability than the formulation containing polysorbate 20. The formulations containing arginine as an amino acid exhibited higher stability than the formulations containing other amino acids, and in particular, the formulations containing sucrose at 10% *(w*/*v)* or more and arginine at 2% (w/v) or more were stable.

### Example 9: Evaluation of stability of lyophilized formulation containing interleukin 2 analog long-acting conjugate

It was attempted to prepare a lyophilized formulation containing an interleukin 2 analog 86 conjugate and to examine its stability.

An aqueous solution containing an interleukin 2 analog 86 conjugate was prepared in a volume of 0.2 mL, and Table 9 below presents the amount (mg) of excipient in 0.2 mL of the aqueous solution. The aqueous solution containing an interleukin 2 analog 86 conjugate was filled in a 2R glass vial by 0.2 mL and partly closed with a rubber stopper. After lyophilization, the vial was completely closed with the rubber stopper and an aluminum cap was attached to the glass vial to obtain a lyophilized formulation.

For initial purity analysis and purity analysis of refrigerated and accelerated storage samples, 0.2 mL of distilled water was added to each lyophilized formulation to dissolve the interleukin 2 analog 86 conjugate to a concentration of 536.9 nmol/mL. The solution was gently shaken to obtain a homogeneous solution, which was then analyzed by size exclusion chromatography and reverse phase chromatography. The lyophilized formulations were stored under accelerated conditions of 25±5°C and refrigerated conditions of 5±3°C, and then analyzed. The results are presented in Tables 10 and 11, respectively. The SE-HPLC (%) and RP-HPLC (%) in Table 10 and Table 11 present the chromatography results for the lyophilized formulations in Table 9 denoted by %area and area%/start area% (%) with respect to the main peak, and indicate the initial purity of the interleukin 2 analog 86 conjugate and the residual percentage compared to the initial result value.

**[Table 9]**

| No. | Buffering substance | pH | Sugar | Amino acid | Surfactant | Antioxidant |
|---|---|---|---|---|---|---|
| 4-2 | Histidine 0.6 mg | 6.5 | Sucrose 16 mg | Arginine 8 mg | Polysorbate 20 0.04 mg | Methionine 0.02 mg |

Table 10 below summarizes the accelerated storage stability at 25±5°C.

**[Table 10]**

| No. | Mass | Period | Phase | SE-HPLC | RP-HPLC |
|---|---|---|---|---|---|
| | | | | Main (%) (Area%/Start Area% (%)) | Main (%) (Area%/Start Area% (%)) |
| 4-2 | 107.4 nmol | Initial | White lyophilized cake | 99.0 (100) | 91.5 (100) |
| | | 1 week | | 99.0 (100) | 91.2 (99.7) |
| | | 2 weeks | | 98.2 (99.2) | 91.3 (99.8) |
| | | 4 weeks | | 98.1 (99.1) | 90.0 (98.4) |

Table 11 below summarizes the long-term storage stability at 5±3°C.

**[Table 11]**

| No. | Mass | Period | Phase | SE-HPLC | RP-HPLC |
|---|---|---|---|---|---|
| | | | | Main (%) (Area%/Start Area% (%)) | Main (%) (Area%/Start Area% (%)) |
| 4-2 | 107.4 nmol | Initial | White lyophilized cake | 99.0 (100) | 91.5 (100) |
| | | 1 week | | 99.1 (100.1) | 91.4 (99.9) |
| | | 2 weeks | | 98.2 (99.2) | 91.4 (99.9) |
| | | 4 weeks | | 97.9 (98.9) | 90.5 (98.9) |
| | | 3 months | | 97.8 (98.8) | 90.5 (98.9) |
| | | 6 months | | 98.2 (99.2) | 92.5 (101.1) |
| | | 9 months | | 98.2 (99.2) | 92.9 (101.5) |

The phase "cake" refers to a porous solid in a lyophilized formulation. The lyophilized formulation No. 4-2 was a favorable white cake, which dissolved quickly in water and was reconstructed into a colorless transparent aqueous solution.

As can be seen in the stability results in Tables 10 and 11, the interleukin 2 analog 86 conjugate maintained its initial purity and was stable when the interleukin 2 analog 86 conjugate was stored in the composition of the lyophilized formulation No. 4-2 for 4 weeks under an accelerated condition and was stored under a refrigerated condition for 9 months.

The above experimental results suggest that a long-acting conjugate including an interleukin 2 analogue in which an amino acid mutation is introduced into native interleukin 2 according to the present invention has changed interleukin 2 alpha receptor binding affinity and interleukin 2 beta receptor binding affinity, and can be used to develop various drugs, particularly anticancer drugs utilizing this.

From the above description, those skilled in the art to which the present invention belongs will be able to understand that the present invention can be implemented in other specific forms without changing the technical idea or essential features thereof. In this regard, it should be understood that the embodiments described above are exemplary in all respects and not restrictive. The scope of the present invention is defined by the appended claims rather than by the description preceding them, and therefore all changes or modifications derived from the meaning and scope of the claims and their equivalent concepts should be interpreted as being included in the scope of the present invention.

### [Description of National Support Research and Development]

This study was conducted with the support of the National New Drug Development Project of the Korea Drug Development Fund with funds from the Ministry of Science and ICT, the Ministry of Trade, Industry and Energy, and the Ministry of Health and Welfare (RS-2022-00165557).

## Claims

1. A formulation of a long-acting conjugate, comprising:
a long-acting conjugate represented by the following Chemical Formula 1 at a concentration of 10 to 3000 nmol/mL; and
a buffer having a pH of 6 to 8:
[Chemical Formula 1] X - Lₐ - F
where, X is an interleukin 2 analog;
L is a polyethylene glycol linker;
a is 0 or a natural number, provided that the respective Ls are independent of each other when a is 2 or more;
F is an immunoglobulin Fc region in a dimeric form; and
- indicates covalent linkage between X and L and between L and F by a covalent bond,
wherein the interleukin 2 analogue includes a sequence in which one or more of amino acids corresponding to positions 1, 12, 18, 19, 20, 22, 32, 35, 38, 42, 43, 45, 48, 49, 61, 68, 69, 74, 76, 80, 81, 82, 84, 85, 86, 87, 88, 89, 91, 92, 94, 95, 96, 125, 126 and 133 in native interleukin 2 are mutated.

2. The long-acting formulation according to claim 1, wherein the interleukin 2 analog is an interleukin 2 analogue in which amino acid at position 1 is deleted and amino acid at position 125 is substituted with a different amino acid in native interleukin 2.

3. The formulation according to claim 2, wherein the interleukin 2 analogue additionally includes 1 to 10 amino acid substitutions or one or more amino acids are added to the amino acid corresponding to position 133.

4. The formulation according to claim 3, wherein in the interleukin 2 analog, one or more of amino acids at positions 18, 19, 20, 22, 38, 42, 43, 45, 61, 68, 69, 74, 80, 81, 84, 85, 86, 88, 89, 91, 92, 94, and 96 are additionally substituted with a different amino acid.

5. The formulation according to claim 1, wherein the interleukin 2 analog is any one of the following analogs:
(a) an interleukin 2 analog in which the amino acid at position 1 is removed and the amino acids at positions 125 and 32 are substituted with different amino acids in native interleukin 2;
(b) an interleukin 2 analog in which the amino acid at position 1 is removed and the amino acids at positions 125 and 35 are substituted with different amino acids in native interleukin 2;
(c) an interleukin 2 analog in which the amino acid at position 1 is removed and the amino acids at positions 125 and 38 are substituted with different amino acids in native interleukin 2;
(d) an interleukin 2 analog in which the amino acid at position 1 is removed and the amino acids at positions 125 and 42 are substituted with different amino acids in native interleukin 2;
(e) an interleukin 2 analog in which the amino acid at position 1 is removed and the amino acids at positions 125 and 43 are substituted with different amino acids in native interleukin 2;
(f) an interleukin 2 analog in which the amino acid at position 1 is removed and the amino acids at positions 125 and 48 are substituted with different amino acids in native interleukin 2;
(g) an interleukin 2 analog in which the amino acid at position 1 is removed and the amino acids at positions 125 and 49 are substituted with different amino acids in native interleukin 2;
(h) an interleukin 2 analog in which the amino acid at position 1 is removed and the amino acids at positions 125 and 76 are substituted with different amino acids in native interleukin 2;
(i) an interleukin 2 analog in which the amino acid at position 1 is removed and the amino acids at positions 125, 38, 42, 81, 92, 94, and 96 are substituted with different amino acids in native interleukin 2;
(j) an interleukin 2 analog in which the amino acid at position 1 is removed and the amino acids at positions 125 and 87 are substituted with different amino acids in native interleukin 2;
(k) an interleukin 2 analog in which the amino acid at position 1 is removed and the amino acids at positions 125, 38, and 42 are substituted with different amino acids in native interleukin 2;
(l) an interleukin 2 analog in which the amino acid at position 1 is removed and the amino acids at positions 125, 38, and 80 are substituted with different amino acids in native interleukin 2;
(m) an interleukin 2 analog in which the amino acid at position 1 is removed and the amino acids at positions 125, 38, and 84 are substituted with different amino acids in native interleukin 2;
(n) an interleukin 2 analog in which the amino acid at position 1 is removed and the amino acids at positions 125, 19, 38, and 42 are substituted with different amino acids in native interleukin 2;
(o) an interleukin 2 analog in which the amino acid at position 1 is removed and the amino acids at positions 125, 12, 38, and 42 are substituted with different amino acids in native interleukin 2;
(p) an interleukin 2 analog in which the amino acid at position 1 is removed and the amino acids at positions 125, 38, 42, and 61 are substituted with different amino acids in native interleukin 2;
(q) an interleukin 2 analog in which the amino acid at position 1 is removed and the amino acids at positions 125, 38, 42, and 84 are substituted with different amino acids in native interleukin 2;
(r) an interleukin 2 analog in which the amino acid at position 1 is removed and the amino acids at positions 125, 38, 42, and 88 are substituted with different amino acids in native interleukin 2;
(s) an interleukin 2 analog in which the amino acid at position 1 is removed and the amino acids at positions 125, 38, 42, and 89 are substituted with different amino acids in native interleukin 2;
(t) an interleukin 2 analog in which the amino acid at position 1 is removed and the amino acids at positions 125, 38, 42, and 91 are substituted with different amino acids in native interleukin 2;
(u) an interleukin 2 analog in which the amino acid at position 1 is removed and the amino acids at positions 125, 38, 42, and 94 are substituted with different amino acids in native interleukin 2;
(v) an interleukin 2 analog in which the amino acid at position 1 is removed and the amino acids at positions 125, 38, 42, and 126 are substituted with different amino acids in native interleukin 2;
(w) an interleukin 2 analog in which the amino acid at position 1 is removed and the amino acids at positions 125, 38, 80, and 84 are substituted with different amino acids in native interleukin 2;
(x) an interleukin 2 analog in which the amino acid at position 1 is removed and the amino acids at positions 125, 38, 94, and 96 are substituted with different amino acids in native interleukin 2;
(y) an interleukin 2 analog in which the amino acid at position 1 is removed and the amino acids at positions 125, 38, 81, and 92 are substituted with different amino acids in native interleukin 2;
(z) an interleukin 2 analog in which the amino acid at position 1 is removed and the amino acids at positions 125, 61, 81, and 92 are substituted with different amino acids in native interleukin 2;
(aa) an interleukin 2 analog in which the amino acid at position 1 is removed and the amino acids at positions 125, 38, 42, 81, and 92 are substituted with different amino acids in native interleukin 2;
(ab) an interleukin 2 analog in which the amino acid at position 1 is removed and the amino acids at positions 125, 38, 80, 81, and 92 are substituted with different amino acids in native interleukin 2;
(ac) an interleukin 2 analog in which the amino acid at position 1 is removed and the amino acids at positions 125, 38, 81, 84, and 92 are substituted with different amino acids in native interleukin 2;
(ad) an interleukin 2 analog in which the amino acid at position 1 is removed and the amino acids at positions 125, 20, 38, 42, 81, and 92 are substituted with different amino acids in native interleukin 2;
(ae) an interleukin 2 analog in which the amino acid at position 1 is removed and the amino acids at positions 125, 38, 42, 80, 81, and 92 are substituted with different amino acids in native interleukin 2;
(af) an interleukin 2 analog in which the amino acid at position 1 is removed and the amino acids at positions 125, 38, 42, 74, 81, and 92 are substituted with different amino acids in native interleukin 2;
(ag) an interleukin 2 analog in which the amino acid at position 1 is removed and the amino acids at positions 125, 38, 42, 81, 84, and 92 are substituted with different amino acids in native interleukin 2;
(ah) an interleukin 2 analog in which the amino acid at position 1 is removed and the amino acids at positions 125, 38, 42, 81, 88, and 92 are substituted with different amino acids in native interleukin 2;
(ai) an interleukin 2 analog in which the amino acid at position 1 is removed and the amino acids at positions 125, 38, 42, 85, 86, and 92 are substituted with different amino acids in native interleukin 2;
(aj) an interleukin 2 analog in which the amino acid at position 1 is removed and the amino acids at positions 125, 38, 80, 81, 85, and 92 are substituted with different amino acids in native interleukin 2;
(ak) an interleukin 2 analog in which the amino acid at position 1 is removed and the amino acids at positions 125, 38, 80, 81, 86, and 92 are substituted with different amino acids in native interleukin 2;
(al) an interleukin 2 analog in which the amino acid at position 1 is removed and the amino acids at positions 125, 80, 81, 85, 86, and 92 are substituted with different amino acids in native interleukin 2;
(am) an interleukin 2 analog in which the amino acid at position 1 is removed and the amino acids at positions 125, 38, 42, 45, 74, 81, and 92 are substituted with different amino acids in native interleukin 2;
(an) an interleukin 2 analog in which the amino acid at position 1 is removed and the amino acids at positions 125, 38, 42, 74, 80, 81, and 92 are substituted with different amino acids in native interleukin 2;
(ao) an interleukin 2 analog in which the amino acid at position 1 is removed and the amino acids at positions 125, 38, 42, 80, 81, 84, and 92 are substituted with different amino acids in native interleukin 2;
(ap) an interleukin 2 analog in which the amino acid at position 1 is removed and the amino acids at positions 125, 38, 42, 45, 80, 85, 86, and 92 are substituted with different amino acids in native interleukin 2;
(aq) an interleukin 2 analog in which the amino acid at position 1 is removed and the amino acids at positions 125, 38, 80, 81, 85, 86, and 92 are substituted with different amino acids in native interleukin 2;
(ar) an interleukin 2 analog in which the amino acid at position 1 is removed and the amino acids at positions 125, 42, 80, 81, 85, 86, and 92 are substituted with different amino acids in native interleukin 2;
(as) an interleukin 2 analog in which the amino acid at position 1 is removed and the amino acids at positions 125, 61, 80, 81, 85, 86, and 92 are substituted with different amino acids in native interleukin 2;
(at) an interleukin 2 analog in which the amino acid at position 1 is removed and the amino acids at positions 125, 69, 80, 81, 85, 86, and 92 are substituted with different amino acids in native interleukin 2;
(au) an interleukin 2 analog in which the amino acid at position 1 is removed and the amino acids at positions 125, 80, 81, 85, 86, 91, and 92 are substituted with different amino acids in native interleukin 2;
(av) an interleukin 2 analog in which the amino acid at position 1 is removed and the amino acids at positions 125, 18, 22, 80, 81, 85, 86, and 92 are substituted with different amino acids in native interleukin 2;
(aw) an interleukin 2 analog in which the amino acid at position 1 is removed and the amino acids at positions 125, 38, 42, 80, 81, 85, 86, and 92 are substituted with different amino acids in native interleukin 2;
(ax) an interleukin 2 analog in which the amino acid at position 1 is removed and the amino acids at positions 125, 38, 42, 45, 74, 80, 81, and 92 are substituted with different amino acids in native interleukin 2;
(ay) an interleukin 2 analog in which the amino acid at position 1 is removed and the amino acids at positions 125, 38, 68, 80, 81, 85, 86, and 92 are substituted with different amino acids in native interleukin 2;
(az) an interleukin 2 analog in which the amino acid at position 1 is removed and the amino acids at positions 125, 69, 74, 80, 81, 85, 86, and 92 are substituted with different amino acids in native interleukin 2;
(ba) an interleukin 2 analog in which the amino acid at position 1 is removed and the amino acids at positions 125, 80, 81, 84, 85, 86, 91, and 92 are substituted with different amino acids in native interleukin 2;
(bb) an interleukin 2 analog in which the amino acid at position 1 is removed and the amino acids at positions 125, 80, 81, 85, 86, 92, 94, and 96 are substituted with different amino acids in native interleukin 2;
(bc) an interleukin 2 analog in which the amino acid at position 1 is removed and the amino acids at positions 125, 18, 19, 22, 80, 81, 85, 86, and 92 are substituted with different amino acids in native interleukin 2;
(bd) an interleukin 2 analog in which the amino acid at position 1 is removed and the amino acids at positions 125, 18, 22, 38, 80, 81, 85, 86, and 92 are substituted with different amino acids in native interleukin 2;
(be) an interleukin 2 analog in which the amino acid at position 1 is removed and the amino acids at positions 125, 18, 22, 61, 80, 81, 85, 86, and 92 are substituted with different amino acids in native interleukin 2;
(bf) an interleukin 2 analog in which the amino acid at position 1 is removed and the amino acids at positions 125, 18, 22, 68, 80, 81, 85, 86, and 92 are substituted with different amino acids in native interleukin 2;
(bg) an interleukin 2 analog in which the amino acid at position 1 is removed and the amino acids at positions 125, 35, 38, 42, 80, 81, 85, 86, and 92 are substituted with different amino acids in native interleukin 2;
(bh) an interleukin 2 analog in which the amino acid at position 1 is removed and the amino acids at positions 125, 38, 42, 45, 80, 81, 85, 86, and 92 are substituted with different amino acids in native interleukin 2;
(bi) an interleukin 2 analog in which the amino acid at position 1 is removed and the amino acids at positions 125, 38, 42, 80, 81, 85, 86, 91, and 92 are substituted with different amino acids in native interleukin 2;
(bj) an interleukin 2 analog in which the amino acid at position 1 is removed and the amino acids at positions 125, 38, 42, 80, 81, 85, 86, 92, and 95 are substituted with different amino acids in native interleukin 2;
(bk) an interleukin 2 analog in which the amino acid at position 1 is removed and the amino acids at positions 125, 35, 38, 42, 74, 80, 81, 85, 86, and 92 are substituted with different amino acids in native interleukin 2;
(bl) an interleukin 2 analog in which the amino acid at position 1 is removed and the amino acids at positions 125, 38, 42, 43, 61, 80, 81, 85, 86, and 92 are substituted with different amino acids in native interleukin 2;
(bm) an interleukin 2 analog in which the amino acid at position 1 is removed and the amino acids at positions 125, 38, 42, 80, 81, 85, 86, 91, 92, and 95 are substituted with different amino acids in native interleukin 2;
(bn) an interleukin 2 analog in which the amino acid at position 1 is removed and the amino acids at positions 125, 35, 38, 42, 74, 80, 81, 82, 85, 86, and 92 are substituted with different amino acids in native interleukin 2;
(bo) an interleukin 2 analog in which the amino acid at position 1 is removed and the amino acids at positions 125, 80, 81, 86, and 92 are substituted with different amino acids in native interleukin 2; and
(bp) an interleukin 2 analog in which the amino acid at position 1 is removed and the amino acids at positions 125, 80, 81, 85, and 86 are substituted with different amino acids in native interleukin 2.

6. The formulation according to claim 5, wherein the amino acid substitution is any one selected from the group consisting of the following amino acid substitutions:
(a) a substitution in which the amino acid at position 12 is substituted with valine or phenylalanine;
(b) a substitution in which the amino acid at position 18 is substituted with arginine;
(c) a substitution in which the amino acid at position 19 is substituted with tyrosine, valine, phenylalanine, or arginine;
(d) a substitution in which the amino acid at position 20 is substituted with valine or phenylalanine;
(e) a substitution in which the amino acid at position 22 is substituted with glutamic acid;
(f) a substitution in which the amino acid at position 32 is substituted with cysteine;
(g) a substitution in which the amino acid at position 35 is substituted with cysteine or glutamic acid;
(h) a substitution in which the amino acid at position 38 is substituted with alanine or aspartic acid;
(i) a substitution in which the amino acid at position 42 is substituted with lysine, alanine, or tryptophan;
(j) a substitution in which the amino acid at position 43 is substituted with cysteine, glutamic acid, or glutamine;
(k) a substitution in which the amino acid at position 45 is substituted with alanine;
(l) a substitution in which the amino acid at position 48 is substituted with cysteine;
(m) a substitution in which the amino acid at position 49 is substituted with cysteine;
(n) a substitution in which the amino acid at position 61 is substituted with glutamine, arginine, or aspartic acid;
(o) a substitution in which the amino acid at position 68 is substituted with aspartic acid or glutamine;
(p) a substitution in which the amino acid at position 69 is substituted with glycine;
(q) a substitution in which the amino acid at position 74 is substituted with histidine or alanine;
(r) a substitution in which the amino acid at position 76 is substituted with cysteine;
(s) a substitution in which the amino acid at position 80 is substituted with phenylalanine, tyrosine, valine, aspartic acid, or tryptophan;
(t) a substitution in which the amino acid at position 81 is substituted with aspartic acid, glutamic acid, or asparagine;
(u) a substitution in which the amino acid at position 82 is substituted with glycine or valine;
(v) a substitution in which the amino acid at position 84 is substituted with glutamic acid, valine, or phenylalanine;
(w) a substitution in which the amino acid at position 85 is substituted with valine, alanine, glycine, tryptophan, tyrosine, threonine, isoleucine, glutamic acid, or phenylalanine;
(x) a substitution in which the amino acid at position 86 is substituted with valine, alanine, glycine, or leucine;
(y) a substitution in which the amino acid at position 87 is substituted with cysteine;
(z) a substitution in which the amino acid at position 88 is substituted with glutamine, valine, or phenylalanine;
(aa) a substitution in which the amino acid at position 89 is substituted with phenylalanine;
(ab) a substitution in which the amino acid at position 91 is substituted with threonine, phenylalanine, or glutamic acid;
(ac) a substitution in which the amino acid at position 92 is substituted with phenylalanine, leucine, tyrosine, or tryptophan;
(ad) a substitution in which the amino acid at position 94 is substituted with phenyl alanine or valine;
(ae) a substitution in which the amino acid at position 95 is substituted with aspartic acid;
(af) a substitution in which the amino acid at position 96 is substituted with phenylalanine, valine, or isoleucine;
(ag) a substitution in which the amino acid at position 125 is substituted with serine; and
(ah) a substitution in which the amino acid at position 126 is substituted with threonine.

7. The formulation according to claim 1, wherein the interleukin 2 analog includes any one of amino acid sequences of SEQ ID NOs: 3 to 106.

8. The formulation according to claim 1, wherein the interleukin 2 analog includes any one of amino acid sequences of SEQ ID NOs: 22, 42, 53, 87, 105 and 106.

9. The formulation according to claim 1, wherein the immunoglobulin Fc region is an IgG4 Fc region.

10. The formulation according to claim 1, wherein the immunoglobulin Fc region is aglycosylated.

11. The formulation according to claim 1, wherein the L includes an ethylene glycol repeating unit, and a formula weight of the ethylene glycol repeating unit moiety is in a range of 1 to 100 kDa.

12. The formulation according to claim 1, wherein in the long-acting conjugate, one molecule of X is covalently linked to one Fc region in the immunoglobulin Fc region in a dimeric form via the polyethylene glycol linker.

13. The formulation according to any one of claims 1 to 12, wherein the formulation is a liquid formulation.

14. The formulation according to claim 13, wherein the buffer contains a buffering substance selected from the group consisting of histidine and its salts, citric acid and its salts, acetic acid and its salts, phosphoric acid and its salts, and a combination thereof.

15. The formulation according to claim 14, wherein a concentration of the buffering substance is 5 to 100 mM to maintain a pH of the liquid formulation in a range of 6 to 8.

16. The formulation according to claim 13, which further comprises an antioxidant, a surfactant, sugar, an amino acid, a tonicity agent, or a combination thereof.

17. The formulation according to claim 16, wherein the antioxidant is methionine.

18. The formulation according to claim 17, which comprises methionine at 0.05 to 0.5 mg/mL.

19. The formulation according to claim 16, wherein the surfactant is selected from the group consisting of poloxamer 188, polysorbate 20, polysorbate 40, polysorbate 60, polysorbate 80, sodium lauryl sulfate, and a combination thereof.

20. The formulation according to claim 19, which comprises a surfactant at a concentration of 0.005% to 1.5% *(w*/*v).*

21. The formulation according to claim 19, which comprises poloxamer 188 at a concentration of 0.005% to 0.1% *(w*/*v).*

22. The formulation according to claim 16, wherein the sugar is glucose, fructose, galactose, lactose, maltose, sucrose or a combination thereof.

23. The formulation according to claim 22, which comprises sugar at a concentration of 10% to 20% *(w*/*v).*

24. The formulation according to claim 16, wherein the amino acid is selected from the group consisting of arginine, serine, alanine, glutamic acid, proline and a combination thereof.

25. The formulation according to claim 16, which comprises an amino acid at a concentration of 0.5% to 5% (w/v).

26. The formulation according to claim 16, which comprises arginine at 2% to 5% (w/v).

27. The formulation according to claim 16, wherein the tonicity agent is sodium chloride.

28. The formulation according to claim 27, which comprises 50 to 100 mM sodium chloride.

29. The formulation according to any one of claims 1 to 12, which is a lyophilized formulation comprising a mixture obtained by lyophilizing an aqueous solution containing an interleukin 2 analog long-acting conjugate and a buffer.

30. The formulation according to claim 29, wherein the buffer contains a buffering substance selected from the group consisting of histidine and its salts, citric acid and its salts, acetic acid and its salts, phosphoric acid and its salts, and a combination thereof.

31. The formulation according to claim 30, wherein the buffer contains 5 to 25 mM histidine.

32. The formulation according to claim 29, wherein the aqueous solution additionally contains a surfactant, sugar, an amino acid, an antioxidant, or a combination thereof.

33. The formulation according to claim 32, wherein the antioxidant is methionine.

34. The formulation according to claim 32, wherein the surfactant is selected from the group consisting of poloxamer 188, polysorbate 20, polysorbate 40, polysorbate 60, polysorbate 80, sodium lauryl sulfate, and a combination thereof.

35. The formulation according to claim 32, wherein the aqueous solution contains polysorbate 20 at a concentration of 0.01% to 0.1% *(w*/*v).*

36. The formulation according to claim 32, wherein the sugar is glucose, fructose, galactose, lactose, maltose, sucrose or a combination thereof.

37. The formulation according to claim 32, wherein the aqueous solution contains sugar at a concentration of 8% to 14% *(w*/*v).*

38. The formulation according to claim 32, wherein the amino acid is selected from the group consisting of arginine, serine, alanine, glutamic acid, proline and a combination thereof.

39. The formulation according to claim 38, wherein the aqueous solution contains arginine at 2% to 5% (w/v).

40. A method for preparing a lyophilized formulation, the method comprising lyophilizing an aqueous solution containing a long-acting conjugate including an interleukin 2 analog, a buffer, and an antioxidant

41. A method for reconstructing a lyophilized formulation, the method comprising adding a reconstructing solution to the formulation according to claim 29.

42. The method according to claim 41, wherein the reconstructing solution is distilled water.

43. The method according to claim 41, wherein a formulation reconstructed by the method comprises a long-acting conjugate including an interleukin 2 analog at 100 to 2000 nmol/mL.
